# EUROPÄISCHE PATENTSCHRIFT

(11) **EP 1 492 781 B1**
(45) Veröffentlichungstag und Bekanntmachung des Hinweises auf die Patenterteilung: **08.03.2006**
(21) Anmeldenummer: 02796585.4
(22) Anmeldetag: 09.12.2002
(51) Int. Cl.: C07D 295/08, A61K 31/495

(54) **N,N'-DISUBSTITUIERTE PIPERAZIN-VERBINDUNGEN UND IHRE VERWENDUNG ALS SCHMERZMITTEL**
N,N'-DISUBSTITUTED PIPERAZINE COMPOUNDS AND THE USE OF THE SAME AS ANALGESICS
COMPOSES DE PIPERAZINE N,N'-DISUBSTITUES ET LEUR UTILISATION COMME ANALGESIQUES

(30) Priorität: 14.12.2001 DE 10161644
(43) Veröffentlichungstag der Anmeldung: 05.01.2005
(73) Patentinhaber: Grünenthal GmbH, 52078 Aachen (DE)
(72) Erfinder: URAGG, Heinz, 52223 Stolberg (DE); MAUL, Corinna, 52066 Aachen (DE); BUSCHMANN, Helmut, E-08950 Esplugues de Llobregat (ES); SUNDERMANN, Bernd, 52066 Aachen (DE); HENNIES, Hagen-Heinrich, 52152 Simmerath (DE)
(74) Vertreter: Kutzenberger, Helga
(86) Internationale Anmeldenummer: PCT/EP2002/013913
(87) Internationale Veröffentlichungsnummer: WO 2003/051855

(56) Entgegenhaltungen:
- WO-A-02/30870
- NIKOLOVA M ET AL: "SYNTHESIS AND PHARMACOLOGICAL SCREENING OF A GROUP OF PIPERAZINE DERIVATIVES. ANALGESIV ACTIVITY" FARMACO, SOCIETA CHIMICA ITALIANA, PAVIA, IT, Bd. 48, Nr. 4, 1. April 1993 (1993-04-01), Seiten 459-472, XP000617338 ISSN: 0014-827X
- HASHIMOTO M ET AL: "METABOLISM OF A NEW ANALGESIC AGENT, DL-ERYTHRO-1-PHENYL-2-(0-CHLOROP HENYL)-2-4-(P-METHOXYBENZYL)-1- PIPERAZINYLETHANOL DIHYDROCHLORIDE, IN RATS" DRUG METABOLISM AND DISPOSITION, WILLIAMS AND WILKINS., BALTIMORE, MD, US, Bd. 7, Nr. 6, 1979, Seiten 435-441, XP000973712 ISSN: 0090-9556

## Beschreibung

Die vorliegende Erfindung betrifft N,N'-disubstituierte Piperazin-Verbindungen, Verfahren zu deren Herstellung, Arzneimittel enthaltend diese Verbindungen und die Verwendung von N,N'-disubstituierten Piperazin-Verbindungen zur Herstellung von Arzneimitteln.

Die Behandlung von Schmerz hat in der Medizin große Bedeutung. Es besteht ein weltweiter Bedarf an wirksamen Schmerztherapien. Der dringende Handlungsbedarf für eine patientengerechte und zielorientierte Behandlung chronischer und nicht chronischer Schmerzzustände, wobei hierunter die erfolgreiche und zufriedenstellende Schmerzbehandlung für den Patienten zu verstehen ist, dokumentiert sich in der großen Anzahl von wissenschaftlichen Arbeiten, die auf dem Gebiet der angewandten Analgetik bzw. der Grundlagenforschung zur Nociception in letzter Zeit erschienen sind. Ebenso ist die Behandlung von Depressionen in der Medizin von großer Bedeutung.

Aufgabe der vorliegenden Erfindung war es daher, neue Verbindungen zur Verfügung zu stellen, die als pharmazeutische Wirkstoffe in Arzneimitteln eingesetzt werden können und sich insbesondere zur Bekämpfung von Schmerz, zur Lokalanästesie, als Antiarrhythmikum, Antiemetikum und/oder Nootropikum (Neurotropikum), zur Behandlung und/oder Therapie von cardiovaskulären Erkrankungen, Harninkontinenz, Diarrhöe, Pruritus und/oder Alkohol- und/oder Drogen- und/oder Medikamentenabhängigkeit und/oder Entzündungen, zur Behandlung von Depressionen, zur Vigilanzsteigerung und/oder Libidosteigerung eignen.

Erfindungsgemäß wird diese Aufgabe durch Bereitstellung von N,N'-disubstituierten Piperazin-Verbindungen der nachstehenden allgemeinen Formel I gelöst, da diese Verbindungen insbesondere eine ausgeprägte analgetische Wirkung und eine ausgeprägte Wirkung gegen Depressionen aufweisen.

Nikolova et al, Il Formaco, 48(4), 1993, 459-472 sowie Hoshimoto et al, Drug Metabolism and Disposition, 7(6), 1979, 435-441 beschreiben N,N'-substituierte Piperazine mit analgetischer Wirkung

Gegenstand der Erfindung sind daher N,N'-disubstituierte Piperazin-Verbindungen der allgemeinen Formel I, 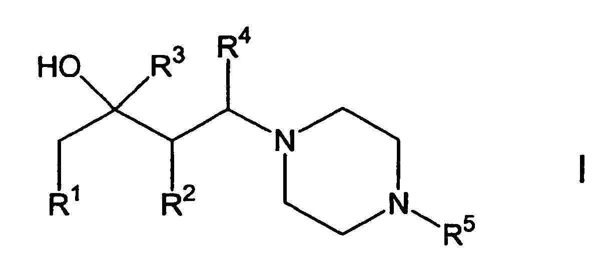
worin
R¹ und R², gleich oder verschieden, für jeweils einen linearen oder verzweigten, gesättigten oder ungesättigten aliphatischen Rest stehen oder zusammen eine (CH₂)ₙ-Kette bilden, wobei n für eine ganze Zahl steht,
R³ und R⁵, gleich oder verschieden, für jeweils einen linearen oder verzweigten, gesättigten oder ungesättigten aliphatischen Rest, einen gesättigten oder ungesättigten cycloaliphatischen Rest, einen Arylrest oder einen Heteroarylrest stehen, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann,
R⁴ für Wasserstoff oder einen gegebenenfalls einfach oder mehrfach substituierten Aryl- oder Heteroarylrest mit mindestens einem Heteroatom steht, wobei der Aryl- oder Heteroarylrest Teil eines polycyclischen Systems sein kann,
in Form ihrer Diastereomere, ihrer Enantiomere und Gemische davon - einschließlich ihrer Racemate - sowie in Form entsprechender Basen, Salze und Solvate.
Bevorzugt sind Verbindungen der allgemeinen Formel I, worin
R¹ und R², gleich oder verschieden, für jeweils einen linearen oder verzweigten, gesättigten oder ungesättigten aliphatischen C₁₋₆-Rest stehen oder zusammen eine (CH₂)ₙ-Kette bilden, wobei n für eine ganze Zahl von 2 - 9 steht,
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert sein und/oder über eine lineare oder verzweigte, gesättigte oder ungesättigte aliphatische C₁₋₅-Brücke gebunden sein kann,
R⁴ für Wasserstoff oder einen Phenylrest steht,
R⁵ für einen linearen oder verzweigten, gesättigten oder ungesättigten aliphatischen C₁₋₆-Rest oder einen gegebenenfalls substituierten Phenylrest steht.
Weiter bevorzugt sind Verbindungen der allgemeinen Formel I, worin
R¹ und R², gleich oder verschieden, für jeweils einen linearen oder verzweigten, gesättigten oder ungesättigten aliphatischen C₁₋₃-Rest stehen oder zusammen eine (CH₂)ₙ-Kette bilden, wobei n für eine ganze Zahl von 2 - 9 steht,
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert sein kann und/oder über eine lineare oder verzweigte, gesättigte oder ungesättigte aliphatische C₁₋₃-Brücke gebunden sein kann,
R⁴ für Wasserstoff oder einen Phenylrest steht,
R⁵ für einen linearen oder verzweigten, gesättigten oder ungesättigten aliphatischen C₁₋₃-Rest oder einen gegebenenfalls mit Halogen oder einer Alkoxygruppe substituierten Phenylrest steht.
Weiter bevorzugt sind Verbindungen der allgemeinen Formel I, worin
R¹ und R² jeweils für eine Methylgruppe stehen oder zusammen eine (CH₂)ₙ-Kette bilden, wobei n für 3, 4, 5 oder 9 steht,
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden sein kann oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert sein kann und/oder gegebenenfalls über eine lineare, gesättigte aliphatische C₁₋₃-Brücke oder eine Ethinyl-Brücke gebunden sein kann,
R⁴ für Wasserstoff oder einen Phenylrest steht,
R⁵ für eine Methylgruppe oder einen gegebenenfalls mit Chlor oder einer Methoxygruppe substituierten Phenylrest steht.

Besonders bevorzugt sind Verbindungen der allgemeinen Formel II, 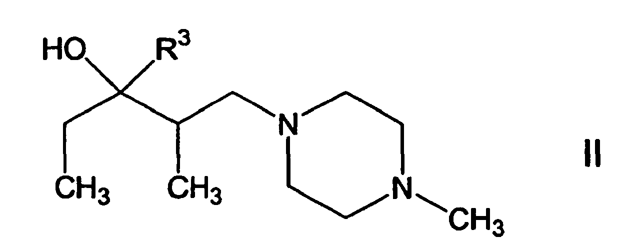
worin
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Besonders bevorzugt sind Verbindungen der allgemeinen Formel III, 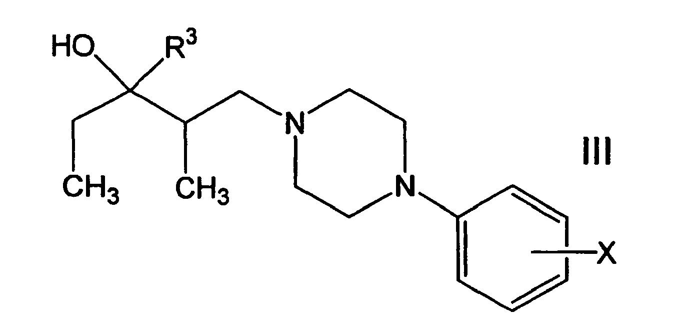
worin
X für Wasserstoff, Halogen oder eine Alkoxygruppe, vorzugsweise für Wasserstoff, Chlor oder eine Methoxygruppe, steht und
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Besonders bevorzugt sind Verbindungen der allgemeinen Formel IV, 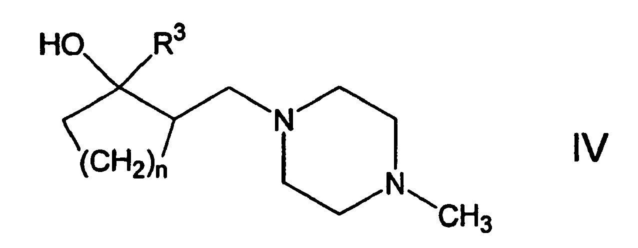
worin
n für eine ganze Zahl von 2-9 steht und
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Besonders bevorzugt sind Verbindungen der allgemeinen Formel V, 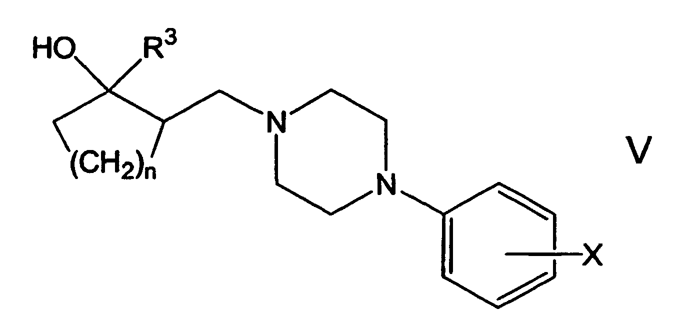
worin
n für eine ganze Zahl von 2 - 9 steht,
X für Wasserstoff, Halogen oder eine Alkoxygruppe, vorzugsweise für Wasserstoff, Chlor oder eine Methoxygruppe, steht und
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Besonders bevorzugt sind Verbindungen der allgemeinen Formel VI, 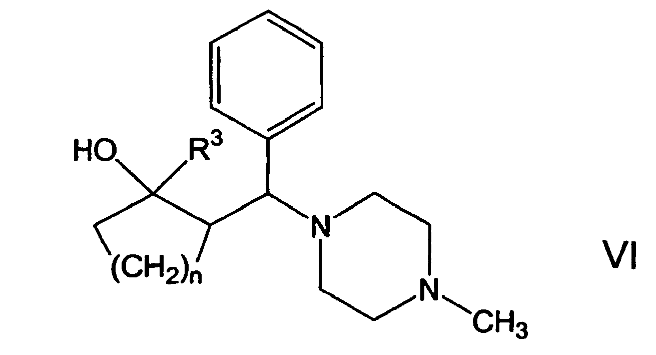
worin
n für eine ganze Zahl von 2 - 9 steht und
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.

Besonders bevorzugt sind Verbindungen der allgemeinen Formel VII, 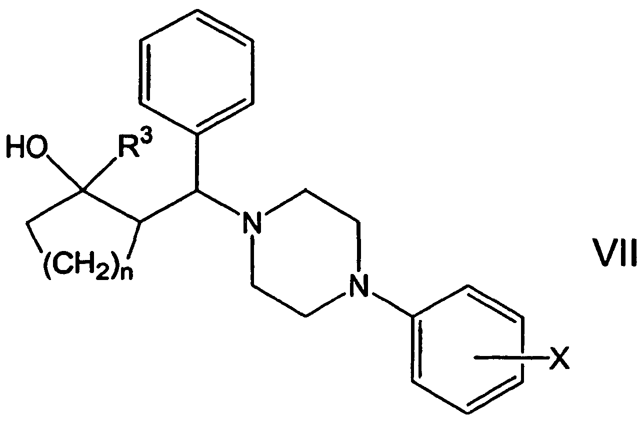
worin
n für eine ganze Zahl von 2 - 9 steht,
X für Wasserstoff, Halogen oder eine Alkoxygruppe, vorzugsweise für Wasserstoff, Chlor oder eine Methoxygruppe, steht und
R³ für einen linearen oder verzweigten, gesättigten oder ungesättigten aliphatischen Rest, einen gesättigten oder ungesättigten cycloaliphatischen Rest, einen Arylrest oder einen Heteroarylrest steht, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann.
Weiter bevorzugt sind Verbindungen der allgemeinen Formel II-VII, worin
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert sein und/oder über eine lineare oder verzweigte, gesättigte oder ungesättigte aliphatische C₁₋₅-Brücke gebunden sein kann
und die übrigen Symbole die vorstehend genannte Bedeutung haben.

Weiter bevorzugt sind Verbindungen der allgemeinen Formel II-VII, worin
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert sein kann und/oder über eine lineare oder verzweigte, gesättigte oder ungesättigte aliphatische C₁₋₃-Brücke gebunden sein kann
und die übrigen Symbole die vorstehend genannte Bedeutung haben.

Weiter bevorzugt sind Verbindungen der allgemeinen Formel II-VII, worin
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden sein kann oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert sein kann und/oder gegebenenfalls über eine lineare, gesättigte aliphatische C₁₋₃-Brücke oder eine Ethinyl-Brücke gebunden sein kann
und die übrigen Symbole die vorstehend genannte Bedeutung haben.

Unter einem Heteroarylrest wird ein gegebenenfalls ein- oder mehrfach substituierter, fünf oder sechsgliedriger aromatischer Rest mit mindestens einem, gegebenenfalls 2, 3, 4 oder 5 Heteroatomen, die gleich oder verschieden sein können, verstanden, der Teil eines polycylischen Systems sein kann. Bevorzugte Heteroatome sind Stickstoff, Sauerstoff und Schwefel. Besonders bevorzugt sind Heteroarylreste ausgewählt aus der Gruppe umfassend Pyrrolyl-, Indolyl-, Furyl- (Furanyl-), Benzofuranyl-, Thienyl- (Thiophenyl-), Benzothienyl-, Pyrazolyl-, Imidazolyl-, Thiazolyl-, Oxazolyl-, Isoxazoyl-, Pydridinyl-, Pyridazinyl-, Pyrimidinyl-, Pyrazinyl-, Pyranyl-, Indazolyl-, Purinyl-, Indolizinyl-, Chinolinyl-, Isochinolinyl-, Chinazolinyl-, Carbazolyl-, Phenazinyl- und Phenothiazinylrest. Die Bindung kann über jedes beliebige, bindungsfähige Ringatom erfolgen. Die gegebenenfalls vorhandenen Substituenten können gleich oder verschieden sein und an jedes beliebige, bindungsfähige Ringatom gebunden sein.

Unter einem Arylrest wird ein gegebenenfalls ein- oder mehrfach substituierter, aromatischer Rest verstanden, der Teil eines polycylischen Systems sein kann. Besonders bevorzugt ist ein Phenylrest. Die Bindung kann über jedes beliebige, bindungsfähige Ringatom erfolgen. Die gegebenenfalls vorhandenen Substituenten können gleich oder verschieden sein und an jedes beliebige, bindungsfähige Ringatom gebunden sein.

Ganz besonders bevorzugt sind Verbindungen ausgewählt aus der Gruppe umfassend
2-Methyl-1-(4-methyl-piperazin-1-yl)-3-phenyl-pentan-3-ol
3-(4-Chlor-phenyl)-2-methyl-1-(4-methyl-piperazin-1-yl)-pentan-3-ol
3-Benzyl-2-methyl-1-(4-methyl-piperazin-1-yl)-pentan-3-ol
3-(4-Fluor-3-methyl-phenyl)-2-methyl-1-(4-methyl-piperazin-1-yl)-pentan-3-ol
2-Methyl-1-(4-methyl-piperazin-1-yl)-3-o-tolyl-pentan-3-ol
3-Ethyl-4-methyl-5-(4-methyl-piperazin-1-yl)-pent-1-en-3-ol
3-(4-tert-Butyl-phenyl)-2-methyl-1-(4-methyl-piperazin-1-yl)-pentan-3-ol
3-Cyclopentyl-2-methyl-1-(4-methyl-piperazin-1-yl)-pentan-3-ol
2-Methyl-1-(4-methyl-piperazin-1-yl)-3-m-tolyl-pentan-3-ol
3-Cyclohexyl-2-methyl-1-(4-methyl-piperazin-1-yl)-pentan-3-ol
3-(4-Fluor-phenyl)-2-methyl-1-(4-methyl-piperazin-1-yl)-pentan-3-ol
3-Ethyl-2-methyl-1-(4-methyl-piperazin-1-yl)-5-phenyl-pentan-3-ol
3-Ethyl-4-methyl-5-(4-methyl-piperazin-1-yl)-1-phenyl-pent-1-yn-3-ol
2-Methyl-1-(4-methyl-piperazin-1-yl)-3-thiophen-2-yl-pentan-3-ol
3-(3-Methoxy-phenyl)-2-methyl-1-(4-methyl-piperazin-1-yl)-pentan-3-ol
3-Ethyl-2-methyl-1-(4-methyl-piperazin-1-yl)-6-phenyl-hexan-3-ol
2-Methyl-1-(4-methyl-piperazin-1-yl)-3-p-tolyl-pentan-3-ol
3-(4-Methoxy-phenyl)-2-methyl-1-(4-methyl-piperazin-1-yl)-pentan-3-ol
2-(4-Methyl-piperazin-1-ylmethyl)-1-phenyl-cyclohexanol
1-Benzyl-2-(4-methyl-piperazin-1-ylmethyl)-cyclohexanol
1-(4-Fluor-3-methyl-phenyl)-2-(4-methyl-piperazin-1-ylmethyl)-cyclohexanol
2-(4-Methyl-piperazin-1-ylmethyl)-1-o-tolyl-cyclohexanol
2-(4-Methyl-piperazin-1-ylmethyl)-1-vinyl-cyclohexanol
1-Cyclopentyl-2-(4-methyl-piperazin-1-ylmethyl)-cyclohexanol
2-(4-Methyl-piperazin-1-ylmethyl)-1-m-tolyl-cyclohexanol
2-(4-Methyl-piperazin-1-ylmethyl)-bicyclohexyl-1-ol
1-(4-Fluor-phenyl)-2-(4-methyl-piperazin-1-ylmethyl)-cyclohexanol
2-(4-Methyl-piperazin-1-ylmethyl)-1-phenethyl-cyclohexanol
2-(4-Methyl-piperazin-1-ylmethyl)-1-thiophen-2-yl-cyclohexanol
1-(2,4-Dichlor-phenyl)-2-(4-methyl-piperazin-1-ylmethyl)-cyclohexanol
1-(3-Methoxy-phenyl)-2-(4-methyl-piperazin-1-ylmethyl)-cyclohexanol
2-(4-Methyl-piperazin-1-ylmethyl)-1-(3-phenyl-propyl)-cyclohexanol
2-(4-Methyl-piperazin-1-ylmethyl)-1-p-tolyl-cyclohexanol
1-(4-Methoxy-phenyl)-2-(4-methyl-piperazin-1-ylmethyl)-cyclohexanol
1-Benzyl-2-(4-methyl-piperazin-1-ylmethyl)-cyclooctanol
2-(4-Methyl-piperazin-1-ylmethyl)-1-vinyl-cyclooctanol
1-(4-tert-Butyl-phenyl)-2-(4-methyl-piperazin-1-ylmethyl)-cyclooctanol
1-Cyclopentyl-2-(4-methyl-piperazin-1-ylmethyl)-cyclooctanol
2-(4-Methyl-piperazin-1-ylmethyl)-1-phenethyl-cyclooctanol
2-(4-Methyl-piperazin-1-ylmethyl)-1-phenylethynyl-cyclooctanol
2-(4-Methyl-piperazin-1-ylmethyl)-1-thiophen-2-yl-cyclooctanol
1-(3-Methoxy-phenyl)-2-(4-methyl-piperazin-1-ylmethyl)-cyclooctanol
2-(4-Methyl-piperazin-1-ylmethyl)-1-(3-phenyl-propyl)-cyclooctanol
2-(4-Methyl-piperazin-1-ylmethyl)-1-p-tolyl-cyclooctanol
1-(4-Methoxy-phenyl)-2-(4-methyl-piperazin-1-ylmethyl)-cyclooctanol
2-(4-Methyl-piperazin-1-ylmethyl)-1-phenyl-cycloheptanol
1-(4-Chlor-phenyl)-2-(4-methyl-piperazin-1-ylmethyl)-cycloheptanol
1-Benzyl-2-(4-methyl-piperazin-1-ylmethyl)-cycloheptanol
1-(4-Fluor-3-methyl-phenyl)-2-(4-methyl-piperazin-1-ylmethyl)-cycloheptanol
2-(4-Methyl-piperazin-1-ylmethyl)-1-o-tolyl-cycloheptanol
2-(4-Methyl-piperazin-1-ylmethyl)-1-vinyl-cycloheptanol
1-(4-tert-Butyl-phenyl)-2-(4-methyl-piperazin-1-ylmethyl)-cycloheptanol
1-Cyclopentyl-2-(4-methyl-piperazin-1-ylmethyl)-cycloheptanol
2-(4-Methyl-piperazin-1-ylmethyl)-1-m-tolyl-cycloheptanol
1-[4-(3-Chlor-phenyl)-piperazin-1-yl]-2-methyl-3-phenyl-pentan-3-ol
3-(4-Chlor-phenyl)-1-[4-(3-chlor-phenyl)-piperazin-1-yl]-2-methyl-pentan-3-ol
3-Benzyl-1-[4-(3-chlor-phenyl)-piperazin-1-yl]-2-methyl-pentan-3-ol
1-[4-(3-Chlor-phenyl)-piperazin-1-yl]-3-(4-fluor-3-methyl-phenyl)-2-methyl-pentan-3-ol
5-[4-(3-Chlor-phenyl)-piperazin-1-yl]-3-ethyl-4-methyl-pent-1-en-3-ol
3-(4-tert-Butyl-phenyl)-1-[4-(3-chlor-phenyl)-piperazin-1-yl]-2-methyl-pentan-3-ol
1-[4-(3-Chlor-phenyl)-piperazin-1-yl]-2-methyl-3-m-tolyl-pentan-3-ol
1-[4-(3-Chlor-phenyl)-piperazin-1-yl]-3-cyclohexyl-2-methyl-pentan-3-ol
1-[4-(3-Chlor-phenyl)-piperazin-1-yl]-3-(4-fluor-phenyl)-2-methyl-pentan-3-ol
1-[4-(3-Chlor-phenyl)-piperazin-1-yl]-3-ethyl-2-methyl-5-phenyl-pentan-3-ol
5-[4-(3-Chlor-phenyl)-piperazin-1-yl]-3-ethyl-4-methyl-1-phenyl-pent-1-yn-3-ol
1-[4-(3-Chlor-phenyl)-piperazin-1-yl]-2-methyl-3-thiophen-2-yl-pentan-3-ol
1-[4-(3-Chlor-phenyl)-piperazin-1-yl]-3-(3-methoxy-phenyl)-2-methyl-pentan-3-ol
1-[4-(3-Chlor-phenyl)-piperazin-1-yl]-3-ethyl-2-methyl-6-phenyl-hexan-3-ol
1-[4-(3-Chlor-phenyl)-piperazin-1-yl]-2-methyl-3-p-tolyl-pentan-3-ol
1-[4-(3-Chlor-phenyl)-piperazin-1-yl]-3-(4-methoxy-phenyl)-2-methyl-pentan-3-ol
1-[4-(3-Chlor-phenyl)-piperazin-1-yl]-3-(5-fluor-2-methoxy-phenyl)-2-methyl-pentan-3-ol
1-[4-(3-Chlor-phenyl)-piperazin-1-yl]-3-(3-fluor-phenyl)-2-methyl-pentan-3-ol
3-(3-Chlor-phenyl)-1-[4-(3-chlor-phenyl)-piperazin-1-yl]-2-methyl-pentan-3-ol
3-(2-Chlor-benzyl)-1-[4-(3-chlor-phenyl)-piperazin-1-yl]-2-methyl-pentan-3-ol
1-[4-(3-Chlor-phenyl)-piperazin-1-yl]-3-(4-fluor benzyl)-2-methyl-pentan-3-ol
1-[4-(3-Chlor-phenyl)-piperazin-1-yl]-3-(3-methoxy-benzyl)-2-methyl-pentan-3-ol
1-[4-(3-Chlor-phenyl)-piperazin-1-yl]-3-(3-fluor-benzyl)-2-methyl-pentan-3-ol
1-[4-(3-Chlor-phenyl)-piperazin-1-yl]-3-(2-methoxy-phenyl)-2-methyl-pentan-3-ol
1-[4-(3-Chlor-phenyl)-piperazin-1-yl]-2-methyl-3-(2-methyl-benzyl)-pentan-3-ol
1-[4-(3-Chlor-phenyl)-piperazin-1-yl]-2-methyl-3-(3-trifluormethyl-phenyl)-pentan-3-ol
1-[4-(3-Chlor-phenyl)-piperazin-1-yl]-2-methyl-3-(3-methyl-benzyl)-pentan-3-ol
3-(4-Chlor-benzyl)-1-[4-(3-chlor-phenyl)-piperazin-1-yl]-2-methyl-pentan-3-ol
3-(2-Chlor-6-fluor-benzyl)-1-[4-(3-chlor-phenyl)-piperazin-1-yl]-2-methyl-pentan-3-ol
1-[4-(3-Chlor-phenyl)-piperazin-1-yl]-3-(2,5-dimethyl-benzyl)-2-methyl-pentan-3-ol
3-(3-Chlor-benzyl)-1-[4-(3-chlor-phenyl)-piperazin-1-yl]-2-methyl-pentan-3-ol
1-[4-(3-Chlor-phenyl)-piperazin-1-yl]-3-(2,4-dichlor-benzyl)-2-methyl-pentan-3-ol
3-Cyclohexylmethyl-2-methyl-1-(4-methyl-piperazin-1-yl)-pentan-3-ol
3-(5-Fluor-2-methoxy-phenyl)-2-methyl-1-(4-methyl-piperazin-1-yl)-pentan-3-ol
3-(3-Fluor-phenyl)-2-methyl-1-(4-methyl-piperazin-1-yl)-pentan-3-ol
3-(3-Chlor-phenyl)-2-methyl-1-(4-methyl-piperazin-1-yl)-pentan-3-ol
3-(3,5-Dichlor-phenyl)-2-methyl-1-(4-methyl-piperazin-1-yl)-pentan-3-ol
3-(2-Chlor-benzyl)-2-methyl-1-(4-methyl-piperazin-1-yl)-pentan-3-ol
3-(4-Fluor-benzyl)-2-methyl-1-(4-methyl-piperazin-1-yl)-pentan-3-ol
3-(3-Methoxy-benzyl)-2-methyl-1-(4-methyl-piperazin-1-yl)-pentan-3-ol
3-(4-Chlor-3-trifluormethyl-phenyl)-2-methyl-1-(4-methyl-piperazin-1-yl)-pentan-3-ol
3-(3-Fluor-benzyl)-2-methyl-1-(4-methyl-piperazin-1-yl)-pentan-3-ol
3-(2-Methoxy-phenyl)-2-methyl-1-(4-methyl-piperazin-1-yl)-pentan-3-ol
2-Methyl-3-(2-methyl-benzyl)-1-(4-methyl-piperazin-1-yl)-pentan-3-ol
3-(3-Chlor-4-fluor-phenyl)-2-methyl-1-(4-methyl-piperazin-1-yl)-pentan-3-ol
2-Methyl-1-(4-methyl-piperazin-1-yl)-3-(3-trifluormethyl-phenyl)-pentan-3-ol
2-Methyl-3-(3-methyl-benzyl)-1-(4-methyl-piperazin-1-yl)-pentan-3-ol
3-(4-Chlor-benzyl)-2-methyl-1-(4-methyl-piperazin-1-yl)-pentan-3-ol
3-(2-Chlor-6-fluor-benzyl)-2-methyl-1-(4-methyl-piperazin-1-yl)-pentan-3-ol
3-(2,5-Dimethyl-benzyl)-2-methyl-1-(4-methyl-piperazin-1-yl)-pentan-3-ol
3-(3-Chlor-benzyl)-2-methyl-1-(4-methyl-piperazin-1-yl)-pentan-3-ol
3-(2,4-Dichlor-benzyl)-2-methyl-1-(4-methyl-piperazin-1-yl)-pentan-3-ol
1-Cyclohexylmethyl-2-(4-methyl-piperazin-1-ylmethyl)-cyclohexanol
1-(5-Fluor-2-methoxy-phenyl)-2-(4-methyl-piperazin-1-ylmethyl)-cyclohexanol
1-(3-Fluor-phenyl)-2-(4-methyl-piperazin-1-ylmethyl)-cyclohexanol
1-(3-Chlor-phenyl)-2-(4-methyl-piperazin-1-ylmethyl)-cyclohexanol
1-(3,5-Dichlor-phenyl)-2-(4-methyl-piperazin-1-ylmethyl)-cyclohexanol
1-(2-Chlor-benzyl)-2-(4-methyl-piperazin-1-ylmethyl)-cyclohexanol
1-(4-Fluor-benzyl)-2-(4-methyl-piperazin-1-ylmethyl)-cyclohexanol
1-(3-Methoxy-benzyl)-2-(4-methyl-piperazin-1-ylmethyl)-cyclohexanol
1-(4-Chlor-3-trifluormethyl-phenyl)-2-(4-methyl-piperazin-1-ylmethyl)-cyclohexanol
1-(3-Fluor-benzyl)-2-(4-methyl-piperazin-1-ylmethyl)-cyclohexanol
1-(2-Methoxy-phenyl)-2-(4-methyl-piperazin-1-ylmethyl)-cyclohexanol
1-(2-Methyl-benzyl)-2-(4-methyl-piperazin-1-ylmethyl)-cyclohexanol
1-(3-Chlor-4-fluor-phenyl)-2-(4-methyl-piperazin-1-ylmethyl)-cyclohexanol
2-(4-Methyl-piperazin-1-ylmethyl)-1-(3-trifluormethyl-phenyl)-cyclohexanol
1-(3-Methyl-benzyl)-2-(4-methyl-piperazin-1-ylmethyl)-cyclohexanol
1-(4-Chlor-benzyl)-2-(4-methyl-piperazin-1-ylmethyl)-cyclohexanol
1-(2,5-Dimethyl-benzyl)-2-(4-methyl-piperazin-1-ylmethyl)-cyclohexanol
1-(3-Chlor-benzyl)-2-(4-methyl-piperazin-1-ylmethyl)-cyclohexanol
1-(2,4-Dichlor-benzyl)-2-(4-methyl-piperazin-1-ylmethyl)-cyclohexanol
1-Cyclohexylmethyl-2-(4-methyl-piperazin-1-ylmethyl)-cyclooctanol
1-(5-Fluor-2-methoxy-phenyl)-2-(4-methyl-piperazin-1-ylmethyl)-cyclooctanol
1-(2-Chlor-benzyl)-2-(4-methyl-piperazin-1-ylmethyl)-cyclooctanol
1-(4-Fluor-benzyl)-2-(4-methyl-piperazin-1-ylmethyl)-cyclooctanol
1-(3-Fluor-benzyl)-2-(4-methyl-piperazin-1-ylmethyl)-cyclooctanol
1-(2-Methoxy-phenyl)-2-(4-methyl-piperazin-1-ylmethyl)-cyclooctanol
1-(3-Methyl-benzyl)-2-(4-methyl-piperazin-1-ylmethyl)-cyclooctanol
1-(4-Chlor-benzyl)-2-(4-methyl-piperazin-1-ylmethyl)-cyclooctanol
1-(2-Chlor-6-fluor-benzyl)-2-(4-methyl-piperazin-1-ylmethyl)-cyclooctanol
1-(2,5-Dimethyl-benzyl)-2-(4-methyl-piperazin-1-ylmethyl)-cyclooctanol
1-(3-Chlor-benzyl)-2-(4-methyl-piperazin-1-ylmethyl)-cyclooctanol
1-(2,4-Dichlor-benzyl)-2-(4-methyl-piperazin-1-ylmethyl)-cyclooctanol
1-Cyclohexylmethyl-2-(4-methyl-piperazin-1-ylmethyl)-cycloheptanol
1-(5-Fluor-2-methoxy-phenyl)-2-(4-methyl-piperazin-1-ylmethyl)-cycloheptanol
1-(3-Fluor-phenyl)-2-(4-methyl-piperazin-1-ylmethyl)-cycloheptanol
1-(3-Chlor-phenyl)-2-(4-methyl-piperazin-1-ylmethyl)-cycloheptanol
1-(3,5-Dichlor-phenyl)-2-(4-methyl-piperazin-1-ylmethyl)-cycloheptanol
1-(2-Chlor-benzyl)-2-(4-methyl-piperazin-1-ylmethyl)-cycloheptanol
1-(4-Fluor-benzyl)-2-(4-methyl-piperazin-1-ylmethyl)-cycloheptanol
1-(3-Methoxy-benzyl)-2-(4-methyl-piperazin-1-ylmethyl)-cycloheptanol
1-(4-Chlor-3-trifluormethyl-phenyl)-2-(4-methyl-piperazin-1-ylmethyl)-cycloheptanol
1-(3-Fluor-benzyl)-2-(4-methyl-piperazin-1-ylmethyl)-cycloheptanol
1-(2-Methoxy-phenyl)-2-(4-methyl-piperazin-1-ylmethyl)-cycloheptanol
1-(2-Methyl-benzyl)-2-(4-methyl-piperazin-1-ylmethyl)-cycloheptanol
1-(3-Chlor-4-fluor-phenyl)-2-(4-methyl-piperazin-1-ylmethyl)-cycloheptanol
2-(4-Methyl-piperazin-1-ylmethyl)-1-(3-trifluormethyl-phenyl)-cycloheptanol
1-(3-Methyl-benzyl)-2-(4-methyl-piperazin-1-ylmethyl)-cycloheptanol
1-(4-Chlor-benzyl)-2-(4-methyl-piperazin-1-ylmethyl)-cycloheptanol
1-(2-Chlor-6-fluor-benzyl)-2-(4-methyl-piperazin-1-ylmethyl)-cycloheptanol
1-(2,5-Dimethyl-benzyl)-2-(4-methyl-piperazin-1-ylmethyl)-cycloheptanol
1-(3-Chlor-benzyl)-2-(4-methyl-piperazin-1-ylmethyl)-cycloheptanol
1-(2,4-Dichlor-benzyl)-2-(4-methyl-piperazin-1-ylmethyl)-cycloheptanol
1-Benzyl-2-(4-methyl-piperazin-1-ylmethyl)-cyclododecanol
2-(4-Methyl-piperazin-1-ylmethyl)-1-vinyl-cyclododecanol
1-Cyclopentyl-2-(4-methyl-piperazin-1-ylmethyl)-cyclododecanol
2-(4-Methyl-piperazin-1-ylmethyl)-1-phenethyl-cyclododecanol
2-(4-Methyl-piperazin-1-ylmethyl)-1-phenylethynyl-cyclododecanol
2-(4-Methyl-piperazin-1-ylmethyl)-1-thiophen-2-yl-cyclododecanol
2-(4-Methyl-piperazin-1-ylmethyl)-1-(3-phenyl-propyl)-cyclododecanol
1-(5-Fluor-2-methoxy-phenyl)-2-(4-methyl-piperazin-1-ylmethyl)-cyclododecanol
1-(2-Chlor-benzyl)-2-(4-methyl-piperazin-1-ylmethyl)-cyclododecanol
1-(4-Fluor-benzyl)-2-(4-methyl-piperazin-1-ylmethyl)-cyclododecanol
1-(3-Methoxy-benzyl)-2-(4-methyl-piperazin-1-ylmethyl)-cyclododecanol
1-(3-Fluor-benzyl)-2-(4-methyl-piperazin-1-ylmethyl)-cyclododecanol
1-(2-Methoxy-phenyl)-2-(4-methyl-piperazin-1-ylmethyl)-cyclododecanol
1-(2-Methyl-benzyl)-2-(4-methyl-piperazin-1-ylmethyl)-cyclododecanol
1-(3-Methyl-benzyl)-2-(4-methyl-piperazin-1-ylmethyl)-cyclododecanol
1-(4-Chlor-benzyl)-2-(4-methyl-piperazin-1-ylmethyl)-cyclododecanol
1-(2,5-Dimethyl-benzyl)-2-(4-methyl-piperazin-1-ylmethyl)-cyclododecanol
1-(3-Chlor-benzyl)-2-(4-methyl-piperazin-1-ylmethyl)-cyclododecanol
1-(2,4-Dichlor-benzyl)-2-(4-methyl-piperazin-1-ylmethyl)-cyclododecanol
2-[4-(3-Chlor-phenyl)-piperazin-1-ylmethyl]-1-cyclohexylmethyl-cyclohexanol
2-[4-(3-Chlor-phenyl)-piperazin-1-ylmethyl]-1-(5-fluor-2-methoxy-phenyl)-cyclohexanol
2-[4-(3-Chlor-phenyl)-piperazin-1-ylmethyl]-1-(3-fluor-phenyl)-cyclohexanol
1-(3-Chlor-phenyl)-2-[4-(3-chlor-phenyl)-piperazin-1-ylmethyl]-cyclohexanol
2-[4-(3-Chlor-phenyl)-piperazin-1-ylmethyl]-1-(3,5-dichlor-phenyl)-cyclohexanol
1-(2-Chlor-benzyl)-2-[4-(3-chlor-phenyl)-piperazin-1-ylmethyl]-cyclohexanol
2-[4-(3-Chlor-phenyl)-piperazin-1-ylmethyl]-1-( 4-fluor-benzyl)-cyclohexanol
2-[4-(3-Chlor-phenyl)-piperazin-1-ylmethyl]-1-(4-chlor-3-trifluormethyl-phenyl)-cyclohexanol
2-[4-(3-Chlor-phenyl)-piperazin-1-ylmethyl]-1-(3-fluor-benzyl)-cyclohexanol
2-[4-(3-Chlor-phenyl)-piperazin-1-ylmethyl]-1-(2-methoxy-phenyl)-cyclohexanol
1-(3-Chlor-4-fluor-phenyl)-2-[4-(3-chlor-phenyl)-piperazin-1-ylmethyl]-cyclohexanol
2-[4-(3-Chlor-phenyl)-piperazin-1-ylmethyl]-1-(3-trifluormethyl-phenyl)-cyclohexanol
2-[4-(3-Chlor-phenyl)-piperazin-1-ylmethyl]-1-(3-methyl-benzyl)-cyclohexanol
1-(4-Chlor-benzyl)-2-[4-(3-chlor-phenyl)-piperazin-1-ylmethyl]-cyclohexanol
1-(2-Chlor-6-fluor-benzyl)-2-[4-(3-chlor-phenyl)-piperazin-1-ylmethyl]-cyclohexanol
2-[4-(3-Chlor-phenyl)-piperazin-1-ylmethyl]-1-(2,5-dimethyl-benzyl)-cyclohexanol
1-(3-Chlor-benzyl)-2-[4-(3-chlor-phenyl)-piperazin-1-ylmethyl]-cyclohexanol
2-[4-(2-Chlor-phenyl)-piperazin-1-ylmethyl]-1-phenyl-cyclohexanol
1-(4-Chlor-phenyl)-2-[4-(2-chlor-phenyl)-piperazin-1-ylmethyl]-cyclohexanol
1-Benzyl-2-[4-(2-chlor-phenyl)-piperazin-1-ylmethyl]-cyclohexanol
2-[4-(2-Chlor-phenyl)-piperazin-1-ylmethyl]-1-(4-fluor-3-methyl-phenyl)-cyclohexanol
2-[4-(2-Chlor-phenyl)-piperazin-1-ylmethyl]-1-o-tolyl-cyclohexanol
2-[4-(2-Chlor-phenyl)-piperazin-1-ylmethyl]-1-vinyl-cyclohexanol
1-(4-tert-Butyl-phenyl)-2-[4-(2-chlor-phenyl)-piperazin-1-ylmethyl]-cyclohexanol
2-[4-(2-Chlor-phenyl)-piperazin-1-ylmethyl]-1-cyclopentyl-cyclohexanol
2-[4-(2-Chlor-phenyl)-piperazin-1-ylmethyl]-1-m-tolyl-cyclohexanol
2-[4-(2-Chlor-phenyl)-piperazin-1-ylmethyl]-bicyclohexyl-1-ol
2-[4-(2-Chlor-phenyl)-piperazin-1-ylmethyl]-1-(4-fluor-phenyl)-cyclohexanol
2-[4-(2-Chlor-phenyl)-piperazin-1-ylmethyl]-1-phenethyl-cyclohexanol
2-[4-(2-Chlor-phenyl)-piperazin-1-ylmethyl]-1-phenylethynyl-cyclohexanol
2-[4-(2-Chlor-phenyl)-piperazin-1-ylmethyl]-1-thiophen-2-yl-cyclohexanol
2-[4-(2-Chlor-phenyl)-piperazin-1-ylmethyl]-1-(3-methoxy-phenyl)-cyclohexanol
2-[4-(2-Chlor-phenyl)-piperazin-1-ylmethyl]-1-(3-phenyl-propyl)-cyclohexanol
2-[4-(2-Chlor-phenyl)-piperazin-1-ylmethyl]-1-p-tolyl-cyclohexanol
2-[4-(2-Chlor-phenyl)-piperazin-1-ylmethyl]-1-(4-methoxy-phenyl)-cyclohexanol
2-[4-(2-Chlor-phenyl)-piperazin-1-ylmethyl]-1-cyclohexylmethyl-cyclohexanol
2-[4-(2-Chlor-phenyl)-piperazin-1-ylmethyl]-1-(5-fluor-2-methoxy-phenyl)-cyclohexanol
1-(3-Chlor-phenyl)-2-[4-(2-chlor-phenyl)-piperazin-1-ylmethyl]-cyclohexanol
2-[4-(2-Chlor-phenyl)-piperazin-1-ylmethyl]-1-(3,5-dichlor-phenyl)-cyclohexanol
1-(2-Chlor-benzyl)-2-[4-(2-chlor-phenyl)-piperazin-1-ylmethyl]-cyclohexanol
2-[4-(2-Chlor-phenyl)-piperazin-1-ylmethyl]-1-(4-fluor-benzyl)-cyclohexanol
2-[4-(2-Chlor-phenyl)-piperazin-1-ylmethyl]-1-(3-methoxy-benzyl)-cyclohexanol
2-[4-(2-Chlor-phenyl)-piperazin-1-ylmethyl]-1-(4-chlor-3-trifluormethyl-phenyl)-cyclohexanol
2-[4-(2-Chlor-phenyl)-piperazin-1-ylmethyl]-1-(3-fluor-benzyl)-cyclohexanol
2-[4-(2-Chlor-phenyl)-piperazin-1-ylmethyl]-1-(2-methoxy-phenyl)-cyclohexanol
2-[4-(2-Chlor-phenyl)-piperazin-1-ylmethyl]-1-(2-methyl-benzyl)-cyclohexanol
1-(3-Chlor-4-fluor-phenyl)-2-[4-(2-chlor-phenyl)-piperazin-1-ylmethyl]-cyclohexanol
2-[4-(2-Chlor-phenyl)-piperazin-1-ylmethyl]-1-(3-trifluormethyl-phenyl)-cyclohexanol
2-[4-(2-Chlor-phenyl)-piperazin-1-ylmethyl]-1-(3-methyl-benzyl)-cyclohexanol
1-(4-Chlor-benzyl)-2-[4-(2-chlor-phenyl)-piperazin-1-ylmethyl]-cyclohexanol
1-(2-Chlor-6-fluor-benzyl)-2-[4-(2-chlor-phenyl)-piperazin-1-ylmethyl]-cyclohexanol
2-[4-(2-Chlor-phenyl)-piperazin-1-ylmethyl]-1-(2,5-dimethyl-benzyl)-cyclohexanol
1-(3-Chlor-benzyl)-2-[4-(2-chlor-phenyl)-piperazin-1-ylmethyl]-cyclohexanol
2-[4-(2-Chlor-phenyl)-piperazin-1-ylmethyl]-1-(2,4-dichlor-benzyl)-cyclohexanol
2-[4-(2-Methoxy-phenyl)-piperazin-1-ylmethyl]-1-phenyl-cyclohexanol
1-(4-Chlor-phenyl)-2-[4-(2-methoxy-phenyl)-piperazin-1-ylmethyl]-cyclohexanol
1-Benzyl-2-[4-(2-methoxy-phenyl)-piperazin-1-ylmethyl]-cyclohexanol
1-(4-Fluor-3-methyl-phenyl)-2-[4-(2-methoxy-phenyl)-piperazin-1-ylmethyl]-cyclohexanol
2-[4-(2-Methoxy-phenyl)-piperazin-1-ylmethyl]-1-o-tolyl-cyclohexanol
2-[4-(2-Methoxy-phenyl)-piperazin-1-ylmethyl]-1-vinyl-cyclohexanol
1-(4-tert-Butyl-phenyl)-2-[4-(2-methoxy-phenyl)-piperazin-1-ylmethyl]-cyclohexanol
1-Cyclopentyl-2-[4-(2-methoxy-phenyl)-piperazin-1-ylmethyl]-cyclohexanol
2-[4-(2-Methoxy-phenyl)-piperazin-1-ylmethyl]-1-m-tolyl-cyclohexanol
2-[4-(2-Methoxy-phenyl)-piperazin-1-ylmethyl]-bicyclohexyl-1-ol
1-(4-Fluor-phenyl)-2-[4-(2-methoxy-phenyl)-piperazin-1-ylmethyl]-cyclohexanol
2-[4-(2-Methoxy-phenyl)-piperazin-1-ylmethyl]-1-phenethyl-cyclohexanol
2-[4-(2-Methoxy-phenyl)-piperazin-1-ylmethyl]-1-phenylethynyl-cyclohexanol
2-[4-(2-Methoxy-phenyl)-piperazin-1-ylmethyl]-1-thiophen-2-yl-cyclohexanol
1-(3-Methoxy-phenyl)-2-[4-(2-methoxy-phenyl)-piperazin-1-ylmethyl]-cyclohexanol
2-[4-(2-Methoxy-phenyl)-piperazin-1-ylmethyl]-1-(3-phenyl-propyl)-cyclohexanol
1-(2,3-Dichlor-phenyl)-2-[4-(2-methoxy-phenyl)-piperazin-1-ylmethyl]-cyclohexanol
2-[4-(2-Methoxy-phenyl)-piperazin-1-ylmethyl]-1-p-tolyl-cyclohexanol
1-(4-Methoxy-phenyl)-2-[4-(2-methoxy-phenyl)-piperazin-1-ylmethyl]-cyclohexanol
1-Cyclohexylmethyl-2-[4-(2-methoxy-phenyl)-piperazin-1-ylmethyl]-cyclohexanol
1-(5-Fluor-2-methoxy-phenyl)-2-[4-(2-methoxy-phenyl)-piperazin-1-ylmethyl]-cyclohexanol
1-(3-Fluor-phenyl)-2-[4-(2-methoxy-phenyl)-piperazin-1-ylmethyl]-cyclohexanol
1-(3-Chlor-phenyl)-2-[4-(2-methoxy-phenyl)-piperazin-1-ylmethyl]-cyclohexanol
1-(3,5-Dichlor-phenyl)-2-[4-(2-methoxy-phenyl)-piperazin-1-ylmethyl]-cyclohexanol
1-(2-Chlor-benzyl)-2-[4-(2-methoxy-phenyl)-piperazin-1-ylmethyl]-cyclohexanol
1-(4-Fluor-benzyl)-2-[4-(2-methoxy-phenyl)-piperazin-1-ylmethyl]-cyclohexanol
1-(3-Methoxy-benzyl)-2-[4-(2-methoxy-phenyl)-piperazin-1-ylmethyl]-cyclohexanol
1-(4-Chlor-3-trifluormethyl-phenyl)-2-[4-(2-methoxy-phenyl)-piperazin-1-ylmethyl]-cyclohexanol
1-(3-Fluor-benzyl)-2-[4-(2-methoxy-phenyl)-piperazin-1-ylmethyl]-cyclohexanol
1-(2-Methoxy-phenyl)-2-[4-(2-methoxy-phenyl)-piperazin-1-ylmethyl]-cyclohexanol
2-[4-(2-Methoxy-phenyl)-piperazin-1-ylmethyl]-1-(2-methyl-benzyl)-cyclohexanol
1-(3-Chlor-4-fluor-phenyl)-2-[4-(2-methoxy-phenyl)-piperazin-1-ylmethyl]-cyclohexanol
2-[4-(2-Methoxy-phenyl)-piperazin-1-ylmethyl]-1-(3-trifluormethyl-phenyl)-cyclohexanol
2-[4-(2-Methoxy-phenyl)-piperazin-1-ylmethyl]-1-(3-methyl-benzyl)-cyclohexanol
1-(4-Chlor-benzyl)-2-[4-(2-methoxy-phenyl)-piperazin-1-ylmethyl]-cyclohexanol
1-(2-Chlor-6-fluor-benzyl)-2-[4-(2-methoxy-phenyl)-piperazin-1-ylmethyl]-cyclohexanol
1-(2,5-Dimethyl-benzyl)-2-[4-(2-methoxy-phenyl)-piperazin-1-ylmethyl]-cyclohexanol
1-(3-Chlor-benzyl)-2-[4-(2-methoxy-phenyl)-piperazin-1-ylmethyl]-cyclohexanol
1-(2,4-Dichlor-benzyl)-2-[4-(2-methoxy-phenyl)-piperazin-1-ylmethyl]-cyclohexanol
1-Benzyl-2-[4-(2-methoxy-phenyl)-piperazin-1-ylmethyl]-cyclododecanol
2-[4-(2-Methoxy-phenyl)-piperazin-1-ylmethyl]-1-vinyl-cyclododecanol
1-Phenethyl-2-[phenyl-(4-phenyl-piperazin-1-yl)-methyl]-cyclohexanol
1-Benzyl-2-[(4-methyl-piperazin-1-yl)-phenyl-methyl]-cyclohexanol
1-Benzyl-2-[phenyl-(4-phenyl-piperazin-1-yl)-methyl]-cyclohexanol
2-[(4-Methyl-piperazin-1-yl)-phenyl-methyl]-1-phenethyl-cyclohexanol
2-[4-(2-Methoxy-phenyl)-piperazin-1-ylmethyl]-1-phenethyl-cyclododecanol
in Form ihrer Diastereomeren, ihrer Enantiomeren und Gemischen davon - einschließlich der Racemate - sowie in Form der entsprechenden Basen, der entsprechenden Salze und der entsprechenden Solvate.

Ein weiterer Gegenstand der Erfindung ist ein Verfahren zur Herstellung von N,N, disubstituierten Piperazin-Verbindungen der allgemeinen Formel I, dadurch gekennzeichnet, daß man
A₁) ein Keton der Formel (1), worin R¹ und R² die vorstehend genannte Bedeutung haben, 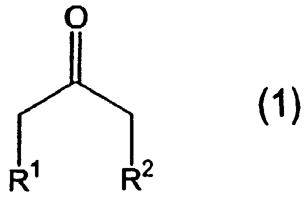 mit Paraformaldehyd und einen Piperazin der Formel (2), worin R⁵ die vorstehend genannte Bedeutung hat, 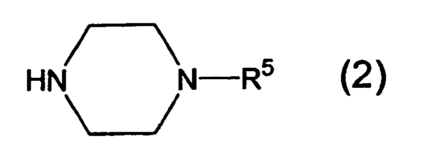 gemäß einer Mannich-Reaktion in einem geeigneten Lösungsmittel, vorzugsweise in Ethanol unter Zusatz von Salzsäure oder in Essigsäure, unter Erhitzen umsetzt, anschließend das Reaktionsgemisch aufarbeitet, das Produkt der Formel (3) isoliert und gegebenenfalls reinigt oder 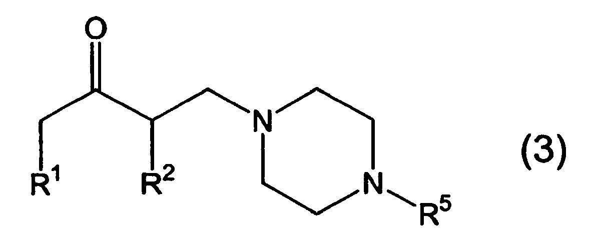
A₂) ein Enamin der Formel (1a), worin R¹ und R² die vorstehend genannte Bedeutung haben und R für einen aliphatischen C₁₋₆-Rest, einen Morpholinyl-, Piperidyl- oder Pyrrolidinylrest steht, wobei die beiden Reste R gleich oder verschieden sein können, 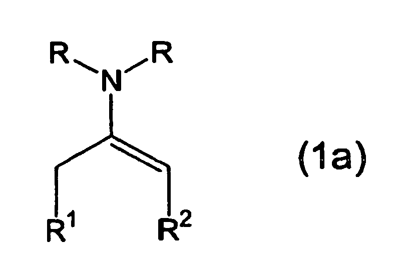 mit einem Aldehyd der Formel (4), worin R⁴ die vorstehend genannte Bedeutung mit Ausnahme von Wasserstoff hat, 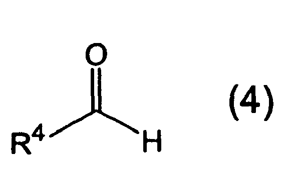 und einen Piperazin der Formel (2), worin R⁵ die vorstehend genannte Bedeutung hat, gegebenenfalls in Form seines Hydrochlorids 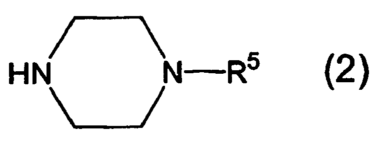 gemäß einer Mannich-Reaktion in Gegenwart von Triethylamin, Chlortrimethylsilan und Natriumiodid in einem geeigneten Lösungsmittel, vorzugsweise in Acetonitril, umsetzt, anschließend das Reaktionsgemisch aufarbeitet, das Produkt der Formel (3a) isoliert und gegebenenfalls reinigt oder 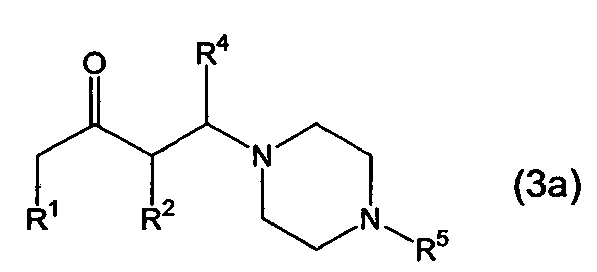
A₃) ein Enamin der Formel (1a), worin R¹ und R² die vorstehend genannte Bedeutung haben und R für einen aliphatischen C₁₋₆-Rest, einen Morpholinyl-, Piperidyl- oder Pyrrolidinylrest steht, wobei die beiden Reste R gleich oder verschieden sein können, 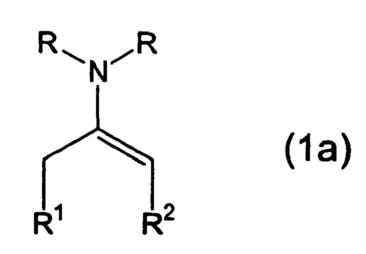 mit einem Iminiumsalz der Formel (5), worin R⁴ die vorstehend genannte Bedeutung mit Ausnahme von Wasserstoff und R⁵ die vorstehend genannte Bedeutung haben und Y⁻ für ein Chlorid-, Bromid-, lodid- oder AlCl₄-lon steht, 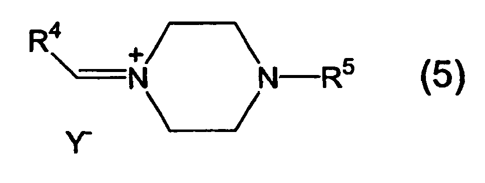
   gemäß einer Mannich-Reaktion in einem geeigneten Lösungsmittel, vorzugsweise in Acetonitril, umsetzt, anschließend das Reaktionsgemisch aufarbeitet, das Produkt der Formel (3a) isoliert und gegebenenfalls reinigt und 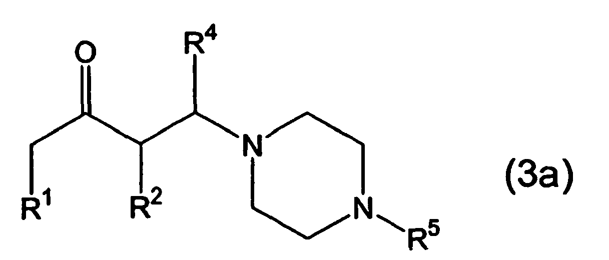
B) eine Verbindung der Formel (3) oder (3a) mit einer Grignard-Verbindung oder einer lithiumorganischen Verbindung der Formeln R³MgCl, R³MgBr, R³MgI, MgR³₂ oder LiR³, worin R³ die vorstehend genannte Bedeutung hat, in einem geeigneten Lösungsmittel, vorzugsweise Diethylether oder Tetrahydrofuran, umsetzt, anschließend das Reaktionsgemisch aufarbeitet, die Verbindung der allgemeinen Formel I isoliert und gegebenenfalls reinigt.

Die zum Einsatz kommenden Ausgangsverbindungen sind am Markt erhältlich oder können nach üblichen dem Fachmann bekannten Methoden gewonnen werden.

Die Umsetzungen sind dem Fachmann aus der Literatur bekannt.

Die zum Einsatz kommenden Lösungsmittel und Umsetzungsbedindungen für die jeweilige Verfahrensstufe entsprechen den für diese Reaktionstypen üblichen Lösungsmitteln und Umsetzungsbedingungen.

Die freien Basen der jeweiligen erfindungsgemäßen Verbindungen der allgemeinen Formel I können durch Umsetzung mit einer anorganischen oder organischen Säure, vorzugsweise mit Salzsäure, Bromwasserstoffsäure, Schwefelsäure, Phosphorsäure, Methansulfonsäure, p-Toluolsulfonsäure, Kohlensäure, Ameisensäure, Essigsäure, Oxalsäure, Bernsteinsäure, Weinsäure, Mandelsäure, Fumarsäure, Milchsäure, Citronensäure, Glutaminsäure oder Asparaginsäure, in die entsprechenden physiologisch verträglichen Salze überführt werden. Bei den gebildeten Salzen handelt es sich unter anderem um Hydrochloride, Hydrobromide, Phosphate, Carbonate, Hydrogencarbonate, Formiate, Acetate, Oxalate, Succinate, Tartrate, Fumarate, Citrate und Glutaminate.

Die freien Basen der jeweiligen erfindungsgemäßen Verbindungen der allgemeinen Formel I können durch Versetzen der in einem geeigneten organischen Lösungsmittel, wie z.B. Butan-2-on (Methylethylketon), gelösten erfindungsgemäßen Verbindungen der allgemeinen Formel I als freie Basen mit Trimethylsilylchlorid (TMSCI) in die entsprechenden Hydrochloride überführt werden. Entsprechend können sie auch in die Hydrobromide überführt werden.

Durch Kristallisation aus wäßriger Lösung können die Hydrate gebildet werden.

Sofern die erfindungsgemäßen Verbindungen der allgemeinen Formel I nach dem erfindungsgemäßen Herstellungsverfahren in Form ihrer Racemate oder anderer Mischungen ihrer verschiedenen Enantiomeren und/oder Diastereomeren erhalten werden, können diese nach üblichen, dem Fachmann bekannten Verfahren getrennt und ggf. isoliert werden. Beispielhaft seien chromatographische Trennverfahren, insbesondere Flüssigkeitschromatographie-Verfahren unter Normaldruck oder unter erhöhtem Druck, bevorzugt MPLC- und HPLC-Verfahren, sowie Verfahren der fraktionierten Kristallisation genannt. Dabei können insbesondere einzelne Enantiomeren, z.B. mittels HPLC an chiraler Phase oder mittels Kristallisation mit chiralen Säuren, etwa (+)-Weinsäure, (-)-Weinsäure oder (+)-10-Camphersulfonsäure, gebildete diastereomere Salze voneinander getrennt werden.

Die erfindungsgemäßen Verbindungen der allgemeinen Formel 1 sind toxikologisch unbedenklich und eignen sich daher als pharmazeutische Wirkstoffe in Arzneimitteln.

Ein weiterer Gegenstand der vorliegenden Erfindung sind daher Arzneimittel, die wenigstens eine erfindungsgemäße Verbindung der allgemeinen Formel I, vorzugsweise der allgemeinen Formel II, III, IV, V, VI oder VII, als Wirkstoff sowie gegebenenfalls physiologisch verträgliche Hilfsstoffe enthalten.

Sofern die erfindungsgemäßen Verbindungen der allgemeinen Formel I oder deren entsprechende physiologisch verträglichen Salze chiral sind, können sie in Form ihrer Enantiomeren, ihrer Diastereomeren oder in Form eines Gemisches aus wenigstens zwei der vorstehend genannten Stereoisomeren - einschließlich ihrer Racemate - in dem erfindungsgemäßen Arzneimittel vorliegen.

Vorzugsweise eignen sich die erfindungsgemäßen Arzneimittel zur Bekämpfung von Schmerz, zur Lokalanästesie, als Antiarrhythmikum, Antiemetikum und/oder Nootropikum (Neurotropikum), zur Behandlung von cardiovaskulären Erkrankungen, Haminkontinenz, Diarrhöe, Pruritus und/oder Alkohol- und/oder Drogen- und/oder Medikamentenabhängigkeit und/oder Entzündungen, zur Behandlung von Depressionen, zur Vigilanzsteigerung und/oder Libidosteigerung.

Die Verwendung wenigstens einer Verbindung der allgemeinen Formel I, vorzugsweise der allgemeinen Formel II, III, IV, V, VI oder VII, zur Herstellung eines Arzneimittels zur Bekämpfung von Schmerz, zur Lokalanästesie, zur Behandlung von Arrhythmien, Emesis, cardiovaskulären Erkrankungen, Harninkontinenz, Diarrhöe, Pruritus und/oder Alkohol- und/oder Drogen- und/oder Medikamentenabhängigkeit und/oder Entzündungen, zur Behandlung von Depressionen, zur Antriebssteigerung, Vigilanzsteigerung und/oder Libidosteigerung ist ein weiterer Gegenstand der vorliegenden Erfindung.

Die erfindungsgemäßen Arzneimittel können als flüssige, halbfeste oder feste Arzneiformen, beispielsweise in Form von Injektionslösungen, Tropfen, Säften, Sirupen, Sprays, Suspensionen, Tabletten, Patches, Kapseln, Pflastem, Zäpfchen, Salben, Cremes, Lotionen, Gelen, Emulsionen, Aerosolen oder in multipartikulärer Form, beispielsweise in Form von Pellets oder Granulaten, formuliert und als solche auch verabreicht werden.

Neben wenigstens einer erfindungsgemäßen Verbindung der allgemeinen Formel I, vorzugsweise der allgemeinen Formel II, III, IV, V, VI oder VII, enthalten die erfindungsgemäßen Arzneimittel üblicherweise weitere physiologisch verträgliche pharmazeutische Hilfsstoffe, die bevorzugt ausgewählt sind aus der Gruppe bestehend aus Trägermaterialien, Füllstoffen, Lösungsmitteln, Verdünnungsmitteln, oberflächenaktiven Stoffen. Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays. Erfindungsgemäße Verbindungen der allgemeinen Formel I, vorzugsweise der allgemeinen Formel II, III, IV, V, VI oder VII, in einem Depot in gelöster Form oder in einem Pflaster, gegebenenfalls unter Zusatz von die Hautpenetration fördemden Mitteln, sind geeignete perkutane Applikationszubereitungen.

Die Herstellung der erfindungsgemäßen Arzneimittel erfolgt mit Hilfe von üblichen, dem Fachmann bekannten Mitteln, Vorrichtungen, Methoden und Verfahren, wie sie beispielsweise in A.R. Gennaro (Hrsg.), Remington's Pharmaceutical Sciences, 17. Edition, Mack Publishing Company, Easton, Pa. (1985), insbesondere in Teil 8, Kapitel 76 bis 93, beschrieben sind. Die entsprechende Literaturbeschreibung wird hiermit als Referenz eingeführt und gilt als Teil der Offenbarung.

Die an den Patienten zu verabreichende Menge der jeweiligen erfindungsgemäßen Verbindung der allgemeinen Formel I, vorzugsweise der allgemeinen Formel II, III, IV, V, VI oder VII, kann variieren und ist beispielsweise abhängig vom Gewicht oder Alter des Patienten sowie von der Applikationsart, der Indikation und dem Schweregrad der Erkrankung. Üblicherweise werden 0.005 bis 500 mg/kg, vorzugsweise 0.05 bis 5 mg/kg Körpergewicht des Patienten wenigstens einer erfindungsgemäßen Verbindung der allgemeinen Formel I, vorzugsweise der allgemeinen Formel II, III, IV, V, VI oder VII, appliziert.

### Beispiele

Die folgenden Beispiele zeigen die Darstellung der erfindungsgemäßen Verbindungen und die mit den erfindungsgemäßen Verbindungen durchgeführten Wirksamkeitsuntersuchungen.

Die eingesetzten Chemikalien und Lösungsmittel wurden kommerziell bei den herkömmlichen Anbietem erworben (Acros, Avocado, Aldrich, Fluka, Lancaster, Maybridge, Merck, Sigma, TCI etc.) oder synthetisiert.

### Darstellung

### Mannichreaktion I

Ein Äquivalent des Piperazin-Hydrochlorids, 1-1,5 Äquivalente des Ketons und 1-1,2 Äquivalente Paraformaldehyd in 8-10 Äquivalenten Essigsäure wurden unter Rühren 15-20 Minuten auf 95-98°C erhitzt. Anschließend wurde die Essigsäure im Vakuum schonend abdestilliert und das Reaktionsgemisch mit Aceton oder nacheinander mit Aceton und Diisopropylether versetzt. Die Mannichverbindung fiel als Hydrochlorid in kristalliner Form aus.
Die Mannichreaktion konnte in einigen Fällen vorteilhaft in Ethanol, versetzt mit wenigen Tropfen konzenzierter Salzsäure, unter Rückfluß über mehrere Stunden (5 bis 7 Stunden) durchgeführt werden.
Die weitere Reinigung der Mannichverbindung erfolgte, falls erforderlich, durch Kieselgel-Chromatographie mit Ethylacetat als Eluenten.

### Mannichreaktion 11

Zu einer Natriumiodid-Lösung in Acetonitril (2,2 Äquivalente) wurde unter Eiskühlung die Piperazin-Verbindung (1Äquivalent) gegeben. Triethylamin (1Äquivalent), und Chlortrimethylsilan (2,2 Äquivalente) wurden zugetropft. Die Suspension wurde eine Stunde bei Raumtemperatur gerührt. Unter Eiskühlung wurde der Aldehyd (1 Äquivalent) zugegeben und eine Stunde bei Raumtemperatur gerührt.

Der Ansatz wurde unter Eiskühlung mit verdünnter Salzsäure versetzt und 15 Minuten gerührt. Die Lösung wurde 3x mit Ether gewaschen. Mit verdünnter Ammoniak-Lösung wurde ein basischer pH-Wert eingestellt und mit Ether extrahiert. Nach dem Trocknen über Magnesiumsulfat wurde eingeengt.

### Grignardreaktion

In einem ausgeheizten und unter Inertgas auf -10°C abgekühlten Reaktionsgefäß wurde die in THF gelöste Mannichverbindung (400µl, 0,5 M) vorgelegt. Unter Rühren wurden daraufhin 2 Äquivalente des vorbereiteten Grignard- oder lithiumorganischen Reagenzes in THF oder Diethylether (800 µl, 0,5 M) zugegeben. Das Reaktionsgemisch wurde bei Raumtemperatur gerührt. Nach drei Stunden wurde erneut auf -10°C gekühlt und mit Ammoniumchlorid-Lösung hydrolisiert.
Das Reaktionsgemisch wurde zweimal mit Ethylacetat extrahiert und bei 40°C im Vakuum eingeengt.
Zur Charakterisierung des Produktes wurde ein ESI-MS aufgenommen.

### Biochemische Untersuchungen

Es wurde gefunden, daß die erfindungsgemäßen Verbindungen eine deutliche Affinität zur Bindungsstelle 2 des Natriumkanals (BTX-Bindung) zeigen und die synaptosomale Noradrenalin-Wiederaufnahme (NA-Uptake-Hemmung) hemmen. Dadurch sind diese Verbindungen der allgemeinen Formel I, vorzugsweise der allgemeinen Formel II, III, IV, V, VI oder VII, auch zur zur Bekämpfung von Schmerz, zur Lokalanästesie, als Antiarrhythmikum, Antiemetikum und/oder Nootropikum (Neurotropikum), zur Behandlung von cardiovaskulären Erkrankungen, Haminkontinenz, Diarrhöe, Pruritus und/oder Alkohol- und/oder Drogen- und/oder Medikamentenabhängigkeit und/oder Entzündungen, zur Behandlung von Depressionen, zur Vigilanzsteigerung und/oder Libidosteigerung geeignet.

### Untersuchungen zur Noradrenalin-Wiederaufnahmehemmung (NA-Uptake-Hemmung)

Um diese in vitro Studien durchführen zu können, wurden Synaptosomen aus Rattenhimarealen frisch isoliert. Es fand jeweils eine sogenannte "P₂"-Fraktion Verwendung, die nach der Vorschrift von Gray und Whittaker (E.G. Gray und V.P. Whittaker (1962) J. Anat. 76, 79-88) präpariert wurde. Für den NA-Uptake wurden diese vesikulären Partikel aus dem Hypothalamus männlicher Rattengehirne isoliert.

Eine detaillierte Methodenbeschreibung kann der Litaratur entnommen werden (M.Ch. Frink, H.-H. Hennies, W. Englberger, M. Haurand und B. Wilffert (1996) Arzneim.-Forsch./Drug Res. 46 (III), 11, 1029-1036).

Folgende Kenndaten wurden für den NA-Transporter ermittelt:
NA-Uptake : Km = 0,32 ± 0,11 µM

### Bindungsuntersuchungen am Natriumkanal Bindungsstelle 2 (BTX-Bindung)

Die Bindungsstelle 2 des Natriumkanals ist die sogenannte Batrachotoxin-(BTX) Bindungsstelle. Als Ligand wurde [³H]-Batrachotoxinin A20 α-Benzoat (10 nM im Ansatz) eingesetzt. Diese Ionenkanal-Partikel (Synaptosomen) wurden aus dem Ratten Cerebrocortex nach Gray und Whittaker (E.G. Gray und V.P. Whittaker (1962) J. Anat. 76, 79-88) angereichert. Als unspezifische Bindung ist die Radioaktivität definiert, die in Gegenwart von Veratridin (0,3 mM im Ansatz) gemessen wird.

Die Assaybedingungen sind nach der Veröffentlichung von Pauwels, Leysen und Laduron (P.J. Pauwels, J.E. Leysen und P.M. Laduron (1986) Eur. J. Pharmacol. 124, 291-298) durchgeführt worden. Inkubation bei 37°C für 120 min.

Der K_{D}-Wert für diese Bindungsstelle liegt bei 24,63 ± 1,56 nM.

Die Ergebnisse der biochemischen Untersuchungen sind in den folgenden Tabellen zusammengefaßt.

### NA-Uptake

| | NA-Uptake Hemmung bei 10 µM [%]-Hemmung |
|---|---|
| 3-Ethyl-2-methyl-1-(4-methyl-piperazin-1-yl)-5-phenyl-pentan-3-ol | 88 |
| 1-(4-Fluor-3-methyl-phenyl)-2-(4-methyl-piperazin-1-ylmethyl)-cyclohexanol | 65 |
| 2-(4-Methyl-piperazin-1-ylmethyl)-1-m-tolyl-cyclohexanol | 44 |
| 1-(4-Fluor-phenyl)-2-(4-methyl-piperazin-1-ylmethyl)-cyclohexanol | 51 |
| 2-(4-Methyl-piperazin-1-ylmethyl)-1-phenethyl-cyclohexanol | 71 |
| 2-(4-Methyl-piperazin-1-ylmethyl)-1-p-tolyl-cyclohexanol | 75 |
| 2-(4-Methyl-piperazin-1-ylmethyl)-1-phenethyl-cyclooctanol | 61 |
| 2-(4-Methyl-piperazin-1-ylmethyl)-1-phenyl-cycloheptanol | 50 |
| 1-(4-Chlor-phenyl)-2-(4-methyl-piperazin-1-ylmethyl)-cycloheptanol | 70 |
| 1-(4-Fluor-3-methyl-phenyl)-2-(4-methyl-piperazin-1-ylmethyl)-cycloheptanol | 66 |
| 1-[4-(3-Chlor-phenyl)-piperazin-1-yl]-2-methyl-3-phenyl-pentan-3-ol | 61 |
| 3-(4-Chlor-phenyl)-1-[4-(3-chlor-phenyl)-piperazin-1-yl]-2-methyl-pentan-3-ol | 54 |
| 3-Benzyl-1-[4-(3-chlor-phenyl)-piperazin-1-yl]-2-methyl-pentan-3-ol | 55 |
| 1-[4-(3-Chlor-phenyl)-piperazin-1-yl]-3-(4-fluor-3-methyl-phenyl)-2-methyl-pentan-3-ol | 39 |
| 5-[4-(3-Chlor-phenyl)-piperazin-1-yl]-3-ethyl-4-methyl-pent-1-en-3-ol | 53 |
| 3-(4-tert-Butyl-phenyl)-1-[4-(3-chlor-phenyl)-piperazin-1-yl]-2-methyl-pentan-3-ol | 62 |
| 1-[4-(3-Chlor-phenyl)-piperazin-1-yl]-2-methyl-3-m-tolyl-pentan-3-ol | 42 |
| 1-[4-(3-Chlor-phenyl)-piperazin-1-yl]-3-cyclohexyl-2-methyl-pentan-3-ol | 73 |
| 1-[4-(3-Chlor-phenyl)-piperazin-1-yl]-3-(4-fluor-phenyl)-2-methyl-pentan-3-ol | 53 |
| 1-[4-(3-Chlor-phenyl)-piperazin-1-yl]-3-ethyl-2-methyl-5-phenyl-pentan-3-ol | 52 |
| 5-[4-(3-Chlor-phenyl)-piperazin-1-yl]-3-ethyl-4-methyl-1-phenyl-pent-1-yn-3-ol | 46 |
| 1-[4-(3-Chlor-phenyl)-piperazin-1-yl]-2-methyl-3-thiophen-2-yl-pentan-3-ol | 45 |
| 1-[4-(3-Chlor-phenyl)-piperazin-1-yl]-3-ethyl-2-methyl-6-phenyl-hexan-3-ol | 55 |
| 1-[4-(3-Chlor-phenyl)-piperazin-1-yl]-2-methyl-3-p-tolyl-pentan-3-ol | 41 |
| 1-[4-(3-Chlor-phenyl)-piperazin-1-yl]-3-(4-methoxy-phenyl)-2-methyl-pentan-3-ol | 45 |
| 1-[4-(3-Chlor-phenyl)-piperazin-1-yl]-3-(3-fluor-phenyl)-2-methyl-pentan-3-ol | 53 |
| 3-(3-Chlor-phenyl)-1-[4-(3-chlor-phenyl)-piperazin-1-yl]-2-methyl-pentan-3-ol | 53 |
| 1-[4-(3-Chlor-phenyl)-piperazin-1-yl]-3-(3-fluor-benzyl)-2-methyl-pentan-3-ol | 50 |
| 1-[4-(3-Chlor-phenyl)-piperazin-1-yl]-2-methyl-3-(3-trifluormethyl-phenyl)-pentan-3-ol | 65 |
| 1-[4-(3-Chlor-phenyl)-piperazin-1-yl]-2-methyl-3-(3-methyl-benzyl)-pentan-3-ol | 55 |
| 3-(4-Chlor-benzyl)-1-[4-(3-chlor-phenyl)-piperazin-1-yl]-2-methyl-pentan-3-ol | 39 |

### BTX-Bindung

| | BTX-Bindung bei 10 µM [%]-Hemmung |
|---|---|
| 2-Methyl-1-(4-methyl-piperazin-1-yl)-3-phenyl-pentan-3-ol | 58 |
| 3-(4-Chlor-phenyl)-2-methyl-1-(4-methyl-piperazin-1-yl)-pentan-3-ol | 59 |
| 3-Benzyl-2-methyl-1-(4-methyl-piperazin-1-yl)-pentan-3-ol | 42 |
| 3-(4-Fluor-3-methyl-phenyl)-2-methyl-1-(4-methyl-piperazin-1-yl)-pentan-3-ol | 56 |
| 2-Methyl-1-(4-methyl-piperazin-1-yl)-3-o-tolyl-pentan-3-ol | 43 |
| 3-Ethyl-4-methyl-5-(4-methyl-piperazin-1-yl)-pent-1-en-3-ol | 67 |
| 3-(4-tert-Butyl-phenyl)-2-methyl-1-(4-methyl-piperazin-1-yl)-pentan-3-ol | 68 |
| 3-Cyclohexyl-2-methyl-1-(4-methyl-piperazin-1-yl)-pentan-3-ol | 59 |
| 3-Ethyl-2-methyl-1-(4-methyl-piperazin-1-yl)-5-phenyl-pentan-3-ol | 74 |
| 2-Methyl-1-(4-methyl-piperazin-1-yl)-3-thiophen-2-yl-pentan-3-ol | 47 |
| 3-Ethyl-2-methyl-1-(4-methyl-piperazin-1-yl)-6-phenyl-hexan-3-ol | 74 |
| 2-Methyl-1-(4-methyl-piperazin-1-yl)-3-p-tolyl-pentan-3-ol | 48 |
| 1-Benzyl-2-(4-methyl-piperazin-1-ylmethyl)-cyclohexanol | 71 |
| 1-(4-Fluor-3-methyl-phenyl)-2-(4-methyl-piperazin-1-ylmethyl)-cyclohexanol | 60 |
| 2-(4-Methyl-piperazin-1-ylmethyl)-1-o-tolyl-cyclohexanol | 50 |
| 1-Cyclopentyl-2-(4-methyl-piperazin-1-ylmethyl)-cyclohexanol | 49 |
| 2-(4-Methyl-piperazin-1-ylmethyl)-1-m-tolyl-cyclohexanol | 58 |
| 2-(4-Methyl-piperazin-1-ylmethyl)-bicyclohexyl-1-ol | 63 |
| 2-(4-Methyl-piperazin-1-ylmethyl)-1-phenethyl-cyclohexanol | 65 |
| 1-(2,4-Dichlor-phenyl)-2-(4-methyl-piperazin-1-ylmethyl)-cyclohexanol | 55 |
| 2-(4-Methyl-piperazin-1-ylmethyl)-1-(3-phenyl-propyl)-cyclohexanol | 80 |
| 2-(4-Methyl-piperazin-1-ylmethyl)-1-p-tolyl-cyclohexanol | 53 |
| 1-Benzyl-2-(4-methyl-piperazin-1-ylmethyl)-cyclooctanol | 75 |
| 1-(4-tert-Butyl-phenyl)-2-(4-methyl-piperazin-1-ylmethyl)-cyclooctanol | 64 |
| 1-Cyclopentyl-2-(4-methyl-piperazin-1-ylmethyl)-cyclooctanol | 48 |
| 2-(4-Methyl-piperazin-1-ylmethyl)-1-phenethyl-cyclooctanol | 78 |
| 2-(4-Methyl-piperazin-1-ylmethyl)-1-thiophen-2-yl-cyclooctanol | 64 |
| 2-(4-Methyl-piperazin-1-ylmethyl)-1-(3-phenyl-propyl)-cyclooctanol | 87 |
| 1-(4-Methoxy-phenyl)-2-(4-methyl-piperazin-1-ylmethyl)-cyclooctanol | 42 |
| 2-(4-Methyl-piperazin-1-ylmethyl)-1-phenyl-cycloheptanol | 63 |
| 1-(4-Chlor-phenyl)-2-(4-methyl-piperazin-1-yimethyl)-cycloheptanol | 69 |
| 1-Benzyl-2-(4-methyl-piperazin-1-ylmethyl)-cycloheptanol | 86 |
| 1-(4-Fluor-3-methyl-phenyl)-2-(4-methyl-piperazin-1-ylmethyl)-cycloheptanol | 69 |
| 1-(4-tert-Butyl-phenyl)-2-(4-methyl-piperazin-1-ylmethyl)-cycloheptanol | 80 |
| 1-Cyclopentyl-2-(4-methyl-piperazin-1-ylmethyl)-cycloheptanol | 55 |
| 2-(4-Methyl-piperazin-1-ylmethyl)-1-m-tolyl-cycloheptanol | 44 |

## Patentansprüche

1. N,N'-disubstituierte Piperazin-Verbindungen der allgemeinen Formel I, 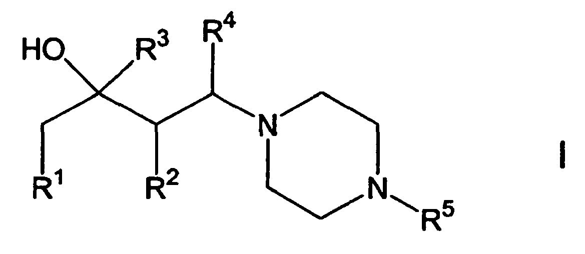
worin
R¹ und R², gleich oder verschieden, für jeweils einen linearen oder verzweigten, gesättigten oder ungesättigten aliphatischen Rest stehen oder zusammen eine (CH₂)ₙ-Kette bilden, wobei n für eine ganze Zahl steht,
R³ und R⁵, gleich oder verschieden, für jeweils einen linearen oder verzweigten, gesättigten oder ungesättigten aliphatischen Rest, einen gesättigten oder ungesättigten cycloaliphatischen Rest, einen Arylrest oder einen Heteroarylrest, wobei das jeweilige Ringsystem gegebenenfalls einfach oder mehrfach substituiert sein und/oder über eine lineare oder verzweigte, gesättigte oder ungesättigte aliphatische Brücke gebunden sein kann und/oder der Aryl- oder Heteroarylrest Teil eines polycyclischen Systems sein kann,
R⁴ für Wasserstoff oder einen gegebenenfalls einfach oder mehrfach, gleich oder verschieden, substituierten Aryl- oder Heteroarylrest steht, wobei der Aryl- oder Heteroarylrest Teil eines polycyclischen Systems sein kann,
in Form ihrer Diastereomere, ihrer Enantiomere und Gemische davon - einschließlich ihrer Racemate - sowie in Form entsprechender Basen, Salze und Solvate.

2. Verbindungen gemäß Anspruch 1, **dadurch gekennzeichnet, daß**
R¹ und R², gleich oder verschieden, für jeweils einen linearen oder verzweigten, gesättigten oder ungesättigten aliphatischen C₁₋₆-Rest stehen oder zusammen eine (CH₂)ₙ-Kette bilden, wobei n für eine ganze Zahl von 2 - 9 steht,
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert sein und/oder über eine lineare oder verzweigte, gesättigte oder ungesättigte aliphatische C₁₋₅-Brücke gebunden sein kann,
R⁴ für Wasserstoff oder einen Phenylrest steht,
R⁵ für einen linearen oder verzweigten, gesättigten oder ungesättigten aliphatischen C₁₋₆-Rest oder einen gegebenenfalls substituierten Phenylrest steht.

3. Verbindungen gemäß Anspruch 1, **dadurch gekennzeichnet, daß**
R¹ und R², gleich oder verschieden, für jeweils einen linearen oder verzweigten, gesättigten oder ungesättigten aliphatischen C₁₋₃-Rest stehen oder zusammen eine (CH₂)ₙ-Kette bilden, wobei n für eine ganze Zahl von 2 - 9 steht,
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert sein kann und/oder über eine lineare oder verzweigte, gesättigte oder ungesättigte aliphatische C₁₋₃-Brücke gebunden sein kann,
R⁴ für Wasserstoff oder einen Phenylrest steht,
R⁵ für einen linearen oder verzweigten, gesättigten oder ungesättigten aliphatischen C₁₋₃-Rest oder einen gegebenenfalls mit Halogen oder einer Alkoxygruppe substituierten Phenylrest steht.

4. Verbindungen gemäß Anspruch 1, **dadurch gekennzeichnet, daß**
R¹ und R² jeweils für eine Methylgruppe stehen oder zusammen eine (CH₂)ₙ₋Kette bilden, wobei n für 3, 4, 5 oder 9 steht,
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden sein kann oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert sein kann und/oder gegebenenfalls über eine lineare, gesättigte aliphatische C₁₋₃-Brücke oder eine Ethinyl-Brücke gebunden sein kann,
R⁴ für Wasserstoff oder einen Phenylrest steht,
R⁵ für eine Methylgruppe oder einen gegebenenfalls mit Chlor oder einer Methoxygruppe substituierten Phenylrest steht.

5. Verbindungen gemäß Anspruch 1 entsprechend der allgemeinen Formel II

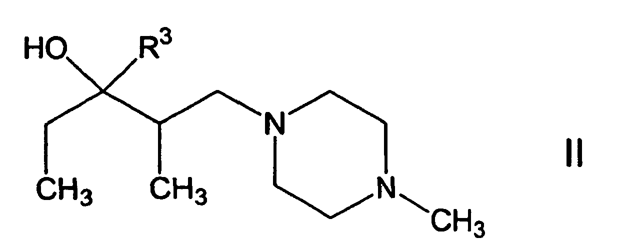

6. Verbindungen gemäß Anspruch 1 entsprechend der allgemeinen Formel III, 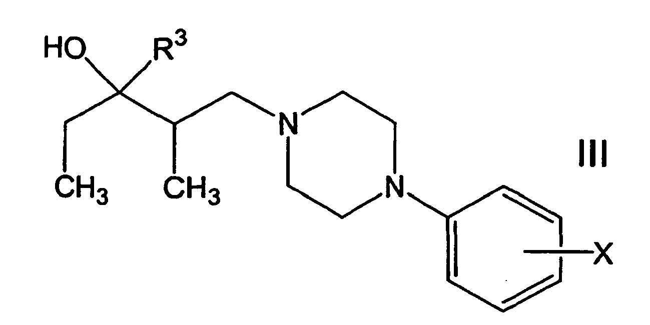
worin
X für Wasserstoff, Halogen oder eine Alkoxygruppe, vorzugsweise für Wasserstoff, Chlor oder eine Methoxygruppe, steht.

7. Verbindungen gemäß Anspruch 1 entsprechend der allgemeinen Formel IV, 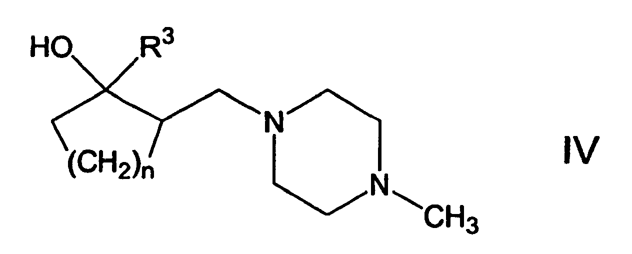
worin
n für eine ganze Zahl von 2-9 steht.

8. Verbindungen gemäß Anspruch 1 entsprechend der allgemeinen Formel V, 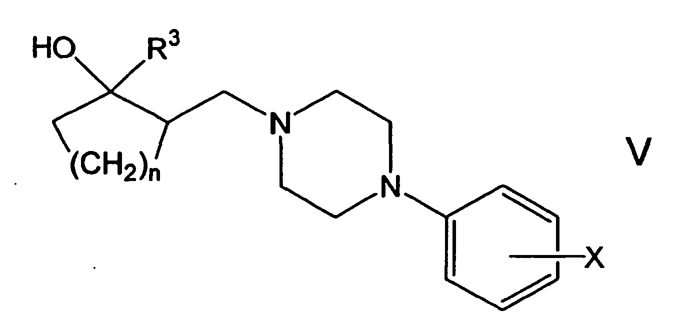
worin
n für eine ganze Zahl von 2 - 9 steht und
X für Wasserstoff, Halogen oder eine Alkoxygruppe, vorzugsweise für Wasserstoff, Chlor oder eine Methoxygruppe, steht.

9. Verbindungen gemäß Anspruch 1 entsprechend der allgemeinen Formel VI, 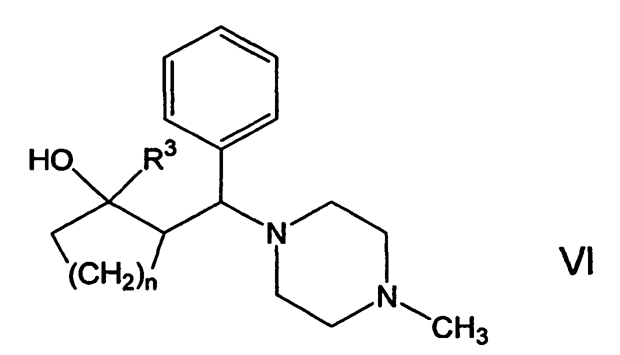
worin
n für eine ganze Zahl von 2 - 9 steht.

10. Verbindungen gemäß Anspruch 1 entsprechend der allgemeinen Formel VII, 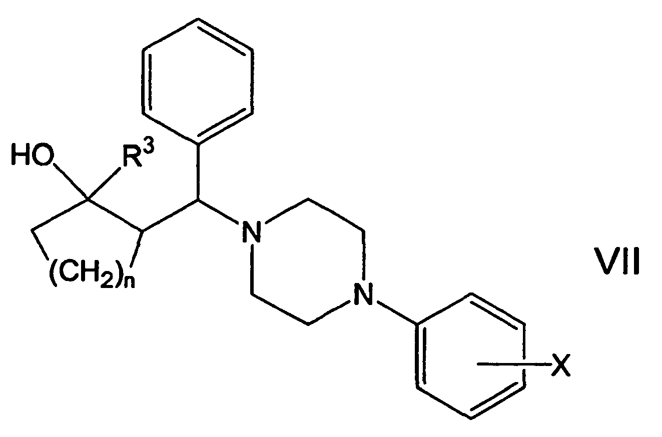
worin
n für eine ganze Zahl von 2 - 9 steht und
X für Wasserstoff, Halogen oder eine Alkoxygruppe, vorzugsweise für Wasserstoff, Chlor oder eine Methoxygruppe, steht.

11. Verbindungen gemäß einem der Ansprüchen 5-10, **dadurch gekennzeichnet, daß**
R³ für einen linearen oder verzweigten, gesättigten oder ungesättigten aliphatischen C₁₋₆-Rest, einen gesättigten oder ungesättigten cycloaliphatischen C₃₋₇-Rest, einen Phenylrest oder einen fünf- oder sechsgliedrigen Heteroarylrest steht, wobei das jeweilige Ringsystem gegebenenfalls einfach oder mehrfach substituiert sein und/oder über eine lineare oder verzweigte, gesättigte oder ungesättigte aliphatische C₁₋₅-Brücke gebunden sein kann.

12. Verbindungen gemäß einem der Ansprüche 5-10, **dadurch gekennzeichnet, daß**
R³ für einen linearen oder verzweigten, gesättigten oder ungesättigten aliphatischen C₁₋₃-Rest, einen gesättigten oder ungesättigten cycloaliphatischen C₅₋₆-Rest, einen Phenylrest oder einen fünf- oder sechsgliedrigen Heteroarylrest steht, wobei das jeweilige Ringsystem gegebenenfalls einfach oder mehrfach mit Halogen, einer Alkylgruppe, einer Alkoxygruppe und/oder einer trihalogenierten Alkylgruppe substituiert sein kann und/oder über eine lineare oder verzweigte, gesättigte oder ungesättigte aliphatische C₁₋₃-Brücke gebunden sein kann.

13. Verbindungen gemäß einem der Ansprüche 5-10, **dadurch gekennzeichnet, daß**
R³ für einen Vinylrest, einen Cyclopentylrest, einen Cyclohexylrest, einen Thiophenylrest oder einen Phenylrest steht, wobei der Cyclohexylrest gegebenenfalls über eine Methylen-Brücke gebunden sein kann oder der Phenylrest gegebenenfalls einfach oder mehrfach mit Fluor, Chlor, einer Methylgruppe, einer Isopropylgruppe, einer Methoxygruppe und/oder einer Trifluormethylgruppe substituiert sein kann und/oder gegebenenfalls über eine lineare, gesättigte aliphatische C₁₋₃-Brücke oder eine Ethinyl-Brücke gebunden sein kann.

14. Eine N,N'-disubstituierte Piperazin-Verbindung gemäß Anspruch 1 ausgewählt aus der Gruppe umfassend
2-Methyl-1-(4-methyl-piperazin-1-yl)-3-phenyl-pentan-3-ol
3-(4-Chlor-phenyl)-2-methyl-1-(4-methyl-piperazin-1-yl)-pentan-3-ol
3-Benzyl-2-methyl-1-(4-methyl-piperazin-1-yl)-pentan-3-ol
3-(4-Fluor-3-methyl-phenyl)-2-methyl-1-(4-methyl-piperazin-1-yl)-pentan-3-ol
2-Methyl-1-(4-methyl-piperazin-1-yl)-3-o-tolyl-pentan-3-ol
3-Ethyl-4-methyl-5-(4-methyl-piperazin-1-yl)-pent-1-en-3-ol
3-(4-tert-Butyl-phenyl)-2-methyl-1-(4-methyl-piperazin-1-yl)-pentan-3-ol
3-Cyclopentyl-2-methyl-1-(4-methyl-piperazin-1-yl)-pentan-3-ol
2-Methyl-1-(4-methyl-piperazin-1-yl)-3-m-tolyl-pentan-3-ol
3-Cyclohexyl-2-methyl-1-(4-methyl-piperazin-1-yl)-pentan-3-ol
3-(4-Fluor-phenyl)-2-methyl-1-(4-methyl-piperazin-1-yl)-pentan-3-ol
3-Ethyl-2-methyl-1-(4-methyl-piperazin-1-yl)-5-phenyl-pentan-3-ol
3-Ethyl-4-methyl-5-(4-methyl-piperazin-1-yl)-1-phenyl-pent-1-yn-3-ol
2-Methyl-1-(4-methyl-piperazin-1-yl)-3-thiophen-2-yl-pentan-3-ol
3-(3-Methoxy-phenyl)-2-methyl-1-(4-methyl-piperazin-1-yl)-pentan-3-ol
3-Ethyl-2-methyl-1-(4-methyl-piperazin-1-yl)-6-phenyl-hexan-3-ol
2-Methyl-1-(4-methyl-piperazin-1-yl)-3-p-tolyl-pentan-3-ol
3-(4-Methoxy-phenyl)-2-methyl-1-(4-methyl-piperazin-1-yl)-pentan-3-ol
2-(4-Methyl-piperazin-1-ylmethyl)-1-phenyl-cyclohexanol
1-Benzyl-2-(4-methyl-piperazin-1-ylmethyl)-cyclohexanol
1-(4-Fluor-3-methyl-phenyl)-2-(4-methyl-piperazin-1-ylmethyl)-cyclohexanol
2-(4-Methyl-piperazin-1-ylmethyl)-1-o-tolyl-cyclohexanol
2-(4-Methyl-piperazin-1-ylmethyl)-1-vinyl-cyclohexanol
1-Cyclopentyl-2-(4-methyl-piperazin-1-ylmethyl)-cyclohexanol
2-(4-Methyl-piperazin-1-ylmethyl)-1-m-tolyl-cyclohexanol
2-(4-Methyl-piperazin-1-ylmethyl)-bicyclohexyl-1-ol
1-(4-Fluor-phenyl)-2-(4-methyl-piperazin-1-ylmethyl)-cyclohexanol
2-(4-Methyl-piperazin-1-ylmethyl)-1-phenethyl-cyclohexanol
2-(4-Methyl-piperazin-1-ylmethyl)-1-thiophen-2-yl-cyclohexanol
1-(2,4-Dichlor-phenyl)-2-(4-methyl-piperazin-1-ylmethyl)-cyclohexanol
1-(3-Methoxy-phenyl)-2-(4-methyl-piperazin-1-ylmethyl)-cyclohexanol
2-(4-Methyl-piperazin-1-ylmethyl)-1-(3-phenyl-propyl)-cyclohexanol
2-(4-Methyl-piperazin-1-ylmethyl)-1-p-tolyl-cyclohexanol
1-(4-Methoxy-phenyl)-2-(4-methyl-piperazin-1-ylmethyl)-cyclohexanol
1-Benzyl-2-(4-methyl-piperazin-1-ylmethyl)-cyclooctanol
2-(4-Methyl-piperazin-1-ylmethyl)-1-vinyl-cyclooctanol
1-(4-tert-Butyl-phenyl)-2-(4-methyl-piperazin-1-ylmethyl)-cyclooctanol
1-Cyclopentyl-2-(4-methyl-piperazin-1-ylmethyl)-cyclooctanol
2-(4-Methyl-piperazin-1-ylmethyl)-1-phenethyl-cyclooctanol
2-(4-Methyl-piperazin-1-ylmethyl)-1-phenylethynyl-cyclooctanol
2-(4-Methyl-piperazin-1-ylmethyl)-1-thiophen-2-yl-cyclooctanol
1-(3-Methoxy-phenyl)-2-(4-methyl-piperazin-1-ylmethyl)-cyclooctanol
2-(4-Methyl-piperazin-1-ylmethyl)-1-(3-phenyl-propyl)-cyclooctanol
2-(4-Methyl-piperazin-1-ylmethyl)-1-p-tolyl-cyclooctanol
1-(4-Methoxy-phenyl)-2-(4-methyl-piperazin-1-ylmethyl)-cyclooctanol
2-(4-Methyl-piperazin-1-ylmethyl)-1-phenyl-cycloheptanol
1-(4-Chlor-phenyl)-2-(4-methyl-piperazin-1-ylmethyl)-cycloheptanol
1-Benzyl-2-(4-methyl-piperazin-1-ylmethyl)-cycloheptanol
1-(4-Fluor-3-methyl-phenyl)-2-(4-methyl-piperazin-1-ylmethyl)-cycloheptanol
2-(4-Methyl-piperazin-1-ylmethyl)-1-o-tolyl-cycloheptanol
2-(4-Methyl-piperazin-1-ylmethyl)-1-vinyl-cycloheptanol
1-(4-tert-Butyl-phenyl)-2-(4-methyl-piperazin-1-ylmethyl)-cycloheptanol
1-Cyclopentyl-2-(4-methyl-piperazin-1-ylmethyl)-cycloheptanol
2-(4-Methyl-piperazin-1-ylmethyl)-1-m-tolyl-cycloheptanol
1-[4-(3-Chlor-phenyl)-piperazin-1-yl]-2-methyl-3-phenyl-pentan-3-ol
3-(4-Chlor-phenyl)-1-[4-(3-chlor-phenyl)-piperazin-1-yl]-2-methyl-pentan-3-ol
3-Benzyl-1-[4-(3-chlor-phenyl)-piperazin-1-yl]-2-methyl-pentan-3-ol
1-[4-(3-Chlor-phenyl)-piperazin-1-yl]-3-(4-fluor-3-methyl-phenyl)-2-methyl-pentan-3-ol
5-[4-(3-Chlor-phenyl)-piperazin-1-yl]-3-ethyl-4-methyl-pent-1-en-3-ol
3-(4-tert-Butyl-phenyl)-1-[4-(3-chlor-phenyl)-piperazin-1-yl]-2-methyl-pentan-3-ol
1-[4-(3-Chlor-phenyl)-piperazin-1-yl]-2-methyl-3-m-tolyl-pentan-3-ol
1-[4-(3-Chlor-phenyl)-piperazin-1-yl]-3-cyclohexyl-2-methyl-pentan-3-ol
1-[4-(3-Chlor-phenyl)-piperazin-1-yl]-3-(4-fluor-phenyl)-2-methyl-pentan-3-ol
1-[4-(3-Chlor-phenyl)-piperazin-1-yl]-3-ethyl-2-methyl-5-phenyl-pentan-3-ol
5-[4-(3-Chlor-phenyl)-piperazin-1-yl]-3-ethyl-4-methyl-1-phenyl-pent-1-yn-3-ol
1-[4-(3-Chlor-phenyl)-piperazin-1-yl]-2-methyl-3-thiophen-2-yl-pentan-3-ol
1-[4-(3-Chlor-phenyl)-piperazin-1-yl]-3-(3-methoxy-phenyl)-2-methyl-pentan-3-ol
1-[4-(3-Chlor-phenyl)-piperazin-1-yl]-3-ethyl-2-methyl-6-phenyl-hexan-3-ol
1-[4-(3-Chlor-phenyl)-piperazin-1-yl]-2-methyl-3-p-tolyl-pentan-3-ol
1-[4-(3-Chlor-phenyl)-piperazin-1-yl]-3-(4-methoxy-phenyl)-2-methyl-pentan-3-ol
1-[4-(3-Chlor-phenyl)-piperazin-1-yl]-3-(5-fluor-2-methoxy-phenyl)-2-methyl-pentan 3-ol
1-[4-(3-Chlor-phenyl)-piperazin-1-yl]-3-(3-fluor-phenyl)-2-methyl-pentan-3-ol
3-(3-Chlor-phenyl)-1-[4-(3-chlor-phenyl)-piperazin-1-yl]-2-methyl-pentan-3-ol
3-(2-Chlor-benzyl)-1-[4-(3-chlor-phenyl)-piperazin-1-yl]-2-methyl-pentan-3-ol
1-[4-(3-Chlor-phenyl)-piperazin-1-yl]-3-(4-fluor-benzyl)-2-methyl-pentan-3-ol
1-[4-(3-Chlor-phenyl)-piperazin-1-yl]-3-(3-methoxy-benzyl)-2-methyl-pentan-3-ol
1-[4-(3-Chlor-phenyl)-piperazin-1-yl]-3-(3-fluor-benzyl)-2-methyl-pentan-3-ol
1-[4-(3-Chlor-phenyl)-piperazin-1-yl]-3-(2-methoxy-phenyl)-2-methyl-pentan-3-ol
1-[4-(3-Chlor-phenyl)-piperazin-1-yl]-2-methyl-3-(2-methyl-benzyl)-pentan-3-ol
1-[4-(3-Chlor-phenyl)-piperazin-1-yl]-2-methyl-3-(3-trifluormethyl-phenyl)-pentan-3-ol
1-[4-(3-Chlor-phenyl)-piperazin-1-yl]-2-methyl-3-(3-methyl-benzyl)-pentan-3-ol
3-(4-Chlor-benzyl)-1-[4-(3-chlor-phenyl)-piperazin-1-yl]-2-methyl-pentan-3-ol
3-(2-Chlor-6-fluor-benzyl)-1-[4-(3-chlor-phenyl)-piperazin-1-yl]-2-methyl-pentan-3-ol
1-[4-(3-Chlor-phenyl)-piperazin-1-yl]-3-(2,5-dimethyl-benzyl)-2-methyl-pentan-3-ol
3-(3-Chlor-benzyl)-1-[4-(3-chlor-phenyl)-piperazin-1-yl]-2-methyl-pentan-3-ol
1-[4-(3-Chlor-phenyl)-piperazin-1-yl]-3-(2,4-dichlor-benzyl)-2-methyl-pentan-3-ol
3-Cyclohexylmethyl-2-methyl-1-(4-methyl-piperazin-1-yl)-pentan-3-ol
3-(5-Fluor-2-methoxy-phenyl)-2-methyl-1-(4-methyl-piperazin-1-yl)-pentan-3-ol
3-(3-Fluor-phenyl)-2-methyl-1-(4-methyl-piperazin-1-yl)-pentan-3-ol
3-(3-Chlor-phenyl)-2-methyl-1-(4-methyl-piperazin-1-yl)-pentan-3-ol
3-(3,5-Dichlor-phenyl)-2-methyl-1-(4-methyl-piperazin-1-yl)-pentan-3-ol
3-(2-Chlor-benzyl)-2-methyl-1-(4-methyl-piperazin-1-yl)-pentan-3-ol
3-(4-Fluor-benzyl)-2-methyl-1-(4-methyl-piperazin-1-yl)-pentan-3-ol
3-(3-Methoxy-benzyl)-2-methyl-1-(4-methyl-piperazin-1-yl)-pentan-3-ol
3-(4-Chlor-3-trifluormethyl-phenyl)-2-methyl-1-(4-methyl-piperazin-1-yl)-pentan-3-ol
3-(3-Fluor-benzyl)-2-methyl-1-(4-methyl-piperazin-1-yl)-pentan-3-ol
3-(2-Methoxy-phenyl)-2-methyl-1-(4-methyl-piperazin-1-yl)-pentan-3-ol
2-Methyl-3-(2-methyl-benzyl)-1-(4-methyl-piperazin-1-yl)-pentan-3-ol
3-(3-Chlor-4-fluor-phenyl)-2-methyl-1-(4-methyl-piperazin-1-yl)-pentan-3-ol
2-Methyl-1-(4-methyl-piperazin-1-yl)-3-(3-trifluormethyl-phenyl)-pentan-3-ol
2-Methyl-3-(3-methyl-benzyl)-1-(4-methyl-piperazin-1-yl)-pentan-3-ol
3-(4-Chlor-benzyl)-2-methyl-1-(4-methyl-piperazin-1-yl)-pentan-3-ol
3-(2-Chlor-6-fluor-benzyl)-2-methyl-1-(4-methyl-piperazin-1-yl)-pentan-3-ol
3-(2,5-Dimethyl-benzyl)-2-methyl-1-(4-methyl-piperazin-1-yl)-pentan-3-ol
3-(3-Chlor-benzyl)-2-methyl-1-(4-methyl-piperazin-1-yl)-pentan-3-ol
3-(2,4-Dichlor-benzyl)-2-methyl-1-(4-methyl-piperazin-1-yl)-pentan-3-ol
1-Cyclohexylmethyl-2-(4-methyl-piperazin-1-ylmethyl)-cyclohexanol
1-(5-Fluor-2-methoxy-phenyl)-2-(4-methyl-piperazin-1-ylmethyl)-cyclohexanol
1-(3-Fluor-phenyl)-2-(4-methyl-piperazin-1-ylmethyl)-cyclohexanol
1-(3-Chlor-phenyl)-2-(4-methyl-piperazin-1-ylmethyl)-cyclohexanol
1-(3,5-Dichlor-phenyl)-2-(4-methyl-piperazin-1-ylmethyl)-cyclohexanol
1-(2-Chlor-benzyl)-2-(4-methyl-piperazin-1-ylmethyl)-cyclohexanol
1-(4-Fluor-benzyl)-2-(4-methyl-piperazin-1-ylmethyl)-cyclohexanol
1-(3-Methoxy-benzyl)-2-(4-methyl-piperazin-1-ylmethyl)-cyclohexanol
1-(4-Chlor-3-trifluormethyl-phenyl)-2-(4-methyl-piperazin-1-ylmethyl)-cyclohexanol
1-(3-Fluor-benzyl)-2-(4-methyl-piperazin-1-ylmethyl)-cyclohexanol
1-(2-Methoxy-phenyl)-2-(4-methyl-piperazin-1-ylmethyl)-cyclohexanol
1-(2-Methyl-benzyl)-2-(4-methyl-piperazin-1-ylmethyl)-cyclohexanol
1-(3-Chlor-4-fluor-phenyl)-2-(4-methyl-piperazin-1-ylmethyl)-cyclohexanol
2-(4-Methyl-piperazin-1-ylmethyl)-1-(3-trifluormethyl-phenyl)-cyclohexanol
1-(3-Methyl-benzyl)-2-(4-methyl-piperazin-1-ylmethyl)-cyclohexanol
1-(4-Chlor-benzyl)-2-(4-methyl-piperazin-1-ylmethyl)-cyclohexanol
1-(2,5-Dimethyl-benzyl)-2-(4-methyl-piperazin-1-ylmethyl)-cyclohexanol
1-(3-Chlor-benzyl)-2-(4-methyl-piperazin-1-ylmethyl)-cyclohexanol
1-(2,4-Dichlor-benzyl)-2-(4-methyl-piperazin-1-ylmethyl)-cyclohexanol
1-Cyclohexylmethyl-2-(4-methyl-piperazin-1-ylmethyl)-cyclooctanol
1-(5-Fluor-2-methoxy-phenyl)-2-(4-methyl-piperazin-1-ylmethyl)-cyclooctanol
1-(2-Chlor-benzyl)-2-(4-methyl-piperazin-1-ylmethyl)-cyclooctanol
1-(4-Fluor-benzyl)-2-(4-methyl-piperazin-1-ylmethyl)-cyclooctanol
1-(3-Fluor-benzyl)-2-(4-methyl-piperazin-1-ylmethyl)-cyclooctanol
1-(2-Methoxy-phenyl)-2-(4-methyl-piperazin-1-ylmethyl)-cyclooctanol
1-(3-Methyl-benzyl)-2-(4-methyl-piperazin-1-ylmethyl)-cyclooctanol
1-(4-Chlor-benzyl)-2-(4-methyl-piperazin-1-ylmethyl)-cyclooctanol
1-(2-Chlor-6-fluor-benzyl)-2-(4-methyl-piperazin-1-ylmethyl)-cyclooctanol
1-(2,5-Dimethyl-benzyl)-2-(4-methyl-piperazin-1-ylmethyl)-cyclooctanol
1-(3-Chlor-benzyl)-2-(4-methyl-piperazin-1-ylmethyl)-cyclooctanol
1-(2,4-Dichlor-benzyl)-2-(4-methyl-piperazin-1-ylmethyl)-cyclooctanol
1-Cyclohexylmethyl-2-(4-methyl-piperazin-1-ylmethyl)-cycloheptanol
1-(5-Fluor-2-methoxy-phenyl)-2-(4-methyl-piperazin-1-ylmethyl)-cycloheptanol
1-(3-Fluor-phenyl)-2-(4-methyl-piperazin-1-ylmethyl)-cycloheptanol
1-(3-Chlor-phenyl)-2-(4-methyl-piperazin-1-ylmethyl)-cycloheptanol
1-(3,5-Dichlor-phenyl)-2-(4-methyl-piperazin-1-ylmethyl)-cycloheptanol
1-(2-Chlor-benzyl)-2-(4-methyl-piperazin-1-ylmethyl)-cycloheptanol
1-(4-Fluor-benzyl)-2-(4-methyl-piperazin-1-ylmethyl)-cycloheptanol
1-(3-Methoxy-benzyl)-2-(4-methyl-piperazin-1-ylmethyl)-cycloheptanol
1-(4-Chlor-3-trifluormethyl-phenyl)-2-(4-methyl-piperazin-1-ylmethyl)-cycloheptanol
1-(3-Fluor-benzyl)-2-(4-methyl-piperazin-1-ylmethyl)-cycloheptanol
1-(2-Methoxy-phenyl)-2-(4-methyl-piperazin-1-ylmethyl)-cycloheptanol
1-(2-Methyl-benzyl)-2-(4-methyl-piperazin-1-ylmethyl)-cycloheptanol
1-(3-Chlor-4-fluor-phenyl)-2-(4-methyl-piperazin-1-ylmethyl)-cycloheptanol
2-(4-Methyl-piperazin-1-ylmethyl)-1-(3-trifluormethyl-phenyl)-cycloheptanol
1-(3-Methyl-benzyl)-2-(4-methyl-piperazin-1-ylmethyl)-cycloheptanol
1-(4-Chlor-benzyl)-2-(4-methyl-piperazin-1-ylmethyl)-cycloheptanol
1-(2-Chlor-6-fluor-benzyl)-2-(4-methyl-piperazin-1-ylmethyl)-cycloheptanol
1-(2,5-Dimethyl-benzyl)-2-(4-methyl-piperazin-1-ylmethyl)-cycloheptanol
1-(3-Chlor-benzyl)-2-(4-methyl-piperazin-1-ylmethyl)-cycloheptanol
1-(2,4-Dichlor-benzyl)-2-(4-methyl-piperazin-1-ylmethyl)-cycloheptanol
1-Benzyl-2-(4-methyl-piperazin-1-ylmethyl)-cyclododecanol
2-(4-Methyl-piperazin-1-ylmethyl)-1-vinyl-cyclododecanol
1-Cyclopentyl-2-(4-methyl-piperazin-1-ylmethyl)-cyclododecanol
2-(4-Methyl-piperazin-1-ylmethyl)-1-phenethyl-cyclododecanol
2-(4-Methyl-piperazin-1-ylmethyl)-1-phenylethynyl-cyclododecanol
2-(4-Methyl-piperazin-1-ylmethyl)-1-thiophen-2-yl-cyclododecanol
2-(4-Methyl-piperazin-1-ylmethyl)-1-(3-phenyl-propyl)-cyclododecanol
1-(5-Fluor-2-methoxy-phenyl)-2-(4-methyl-piperazin-1-ylmethyl)-cyclododecanol
1-(2-Chlor-benzyl)-2-(4-methyl-piperazin-1-ylmethyl)-cyclododecanol
1-(4-Fluor-benzyl)-2-(4-methyl-piperazin-1-ylmethyl)-cyclododecanol
1-(3-Methoxy-benzyl)-2-(4-methyl-piperazin-1-ylmethyl)-cyclododecanol
1-(3-Fluor-benzyl)-2-(4-methyl-piperazin-1-ylmethyl)-cyclododecanol
1-(2-Methoxy-phenyl)-2-(4-methyl-piperazin-1-ylmethyl)-cyclododecanol
1-(2-Methyl-benzyl)-2-(4-methyl-piperazin-1-ylmethyl)-cyclododecanol
1-(3-Methyl-benzyl)-2-(4-methyl-piperazin-1-ylmethyl)-cyclododecanol
1-(4-Chlor-benzyl)-2-(4-methyl-piperazin-1-ylmethyl)-cyclododecanol
1-(2,5-Dimethyl-benzyl)-2-(4-methyl-piperazin-1-ylmethyl)-cyclododecanol
1-(3-Chlor-benzyl)-2-(4-methyl-piperazin-1-ylmethyl)-cyclododecanol
1-(2,4-Dichlor-benzyl)-2-(4-methyl-piperazin-1-ylmethyl)-cyclododecanol
2-[4-(3-Chlor-phenyl)-piperazin-1-ylmethyl]-1-cyclohexylmethyl-cyclohexanol
2-[4-(3-Chlor-phenyl)-piperazin-1-ylmethyl]-1-(5-fluor-2-methoxy-phenyl)-cyclohexanol
2-[4-(3-Chlor-phenyl)-piperazin-1-ylmethyl]-1-(3-fluor-phenyl)-cyclohexanol
1-(3-Chlor-phenyl)-2-[4-(3-chlor-phenyl)-piperazin-1-ylmethyl]-cyclohexanol
2-[4-(3-Chlor-phenyl)-piperazin-1-ylmethyl]-1-(3,5-dichlor-phenyl)-cyclohexanol
1-(2-Chlor-benzyl)-2-[4-(3-chlor-phenyl)-piperazin-1-ylmethyl]-cyclohexanol
2-[4-(3-Chlor-phenyl)-piperazin-1-ylmethyl]-1-(4-fluor-benzyl)-cyclohexanol
2-[4-(3-Chlor-phenyl)-piperazin-1-ylmethyl]-1-(4-chlor-3-trifluormethyl-phenyl)-cyclohexanol
2-[4-(3-Chlor-phenyl)-piperazin-1-ylmethyl]-1-(3-fluor-benzyl)-cyclohexanol
2-[4-(3-Chlor-phenyl)-piperazin-1-ylmethyl]-1-(2-methoxy-phenyl)-cyclohexanol
1-(3-Chlor-4-fluor-phenyl)-2-[4-(3-chlor-phenyl)-piperazin-1-ylmethyl]-cyclohexanol
2-[4-(3-Chlor-phenyl)-piperazin-1-ylmethyl]-1-(3-trifluormethyl-phenyl)-cyclohexanol
2-[4-(3-Chlor-phenyl)-piperazin-1-ylmethyl]-1-(3-methyl-benzyl)-cyclohexanol
1-(4-Chlor-benzyl)-2-[4-(3-chlor-phenyl)-piperazin-1-ylmethyl]-cyclohexanol
1-(2-Chlor-6-fluor-benzyl)-2-[4-(3-chlor-phenyl)-piperazin-1-ylmethyl]-cyclohexanol
2-[4-(3-Chlor-phenyl)-piperazin-1-ylmethyl]-1-(2,5-dimethyl-benzyl)-cyclohexanol
1-(3-Chlor-benzyl)-2-[4-(3-chlor-phenyl)-piperazin-1-ylmethyl]-cyclohexanol
2-[4-(2-Chlor-phenyl)-piperazin-1-ylmethyl]-1-phenyl-cyclohexanol
1-(4-Chlor-phenyl)-2-[4-(2-chlor-phenyl)-piperazin-1-ylmethyl]-cyclohexanol
1-Benzyl-2-[4-(2-chlor-phenyl)-piperazin-1-ylmethyl]-cyclohexanol
2-[4-(2-Chlor-phenyl)-piperazin-1-ylmethyl]-1-(4-fluor-3-methyl-phenyl)-cyclohexanol
2-[4-(2-Chlor-phenyl)-piperazin-1-ylmethyl]-1-o-tolyl-cyclohexanol
2-[4-(2-Chlor-phenyl)-piperazin-1-ylmethyl]-1-vinyl-cyclohexanol
1-(4-tert-Butyl-phenyl)-2-[4-(2-chlor-phenyl)-piperazin-1-ylmethyl]-cyclohexanol
2-[4-(2-Chlor-phenyl)-piperazin-1-ylmethyl]-1-cyclopentyl-cyclohexanol
2-[4-(2-Chlor-phenyl)-piperazin-1-ylmethyl]-1-m-tolyl-cyclohexanol
2-[4-(2-Chlor-phenyl)-piperazin-1-ylmethyl]-bicyclohexyl-1-ol
2-[4-(2-Chlor-phenyl)-piperazin-1-ylmethyl]-1-(4-fluor-phenyl)-cyclohexanol
2-[4-(2-Chlor-phenyl)-piperazin-1-ylmethyl]-1-phenethyl-cyclohexanol
2-[4-(2-Chlor-phenyl)-piperazin-1-ylmethyl]-1-phenylethynyl-cyclohexanol
2-[4-(2-Chlor-phenyl)-piperazin-1-ylmethyl]-1-thiophen-2-yl-cyclohexanol
2-[4-(2-Chlor-phenyl)-piperazin-1-ylmethyl]-1-(3-methoxy-phenyl)-cyclohexanol
2-[4-(2-Chlor-phenyl)·piperazin-1-ylmethyt]-1-(3-phenyt-propyt)-cyclohexanol
2-[4-(2-Chlor-phenyl)-piperazin-1-ylmethyl]-1-p-tolyl-cyclohexanol
2-[4-(2-Chlor-phenyl)-piperazin-1-ylmethyl]-1-(4-methoxy-phenyl)-cyclohexanol
2-[4-(2-Chlor-phenyl)-piperazin-1-ylmethyl]-1-cydohexylnlethyl-cyclohexanol
2-[4-(2-Chlor-phenyl)-piperazin-1-ylmethyl]-1-(5-fluor-2-methoxy-phenyl)-cyclohexanol
1-(3-Chlor-phenyl)-2-[4-(2-chlor-phenyl)-piperazin-1-ylmethyl]-cyclohexanol
2-[4-(2-Chlor-phenyl)-piperazin-1-ylmethyl]-1-(3,5-dichlor-phenyl)-cyclohexanol
1-(2-Chlor-benzyl)-2-[4-(2-chlor-phenyl)-piperazin-1-ylmethyl]-cyclohexanol
2-[4-(2-Chlor-phenyl)-piperazin-1-ylmethyl]-1-(4-fluor-benzyl)-cyclohexanol
2-[4-(2-Chlor-phenyl)-piperazin-1-ylmethyl]-1-(3-methoxy-benzyl)-cyclohexanol
2-[4-(2-Chlor-phenyl)-piperazin-1-ylmethyl]-1-(4-chlor-3-trifluormethyl-phenyl)-cyclohexanol
2-[4-(2-Chlor-phenyl)-piperazin-1-ylmethyl]-1-(3-fluor-benzyl)-cyclohexanol
2-[4-(2-Chlor-phenyl)-piperazin-1-ylmethyl]-1-(2-methoxy-phenyl)-cyclohexanol
2-[4-(2-Chlor-phenyl)-piperazin-1-ylmethyl]-1-(2-methyl-benzyl)-cyclohexanol
1-(3-Chlor-4-fluor-phenyl)-2-[4-(2-chlor-phenyl)-piperazin-1-ylmethyl]-cyclohexanol
2-[4-(2-Chlor-phenyl)-piperazin-1-ylmethyl]-1-(3-trifluormethyl-phenyl)-cyclohexanol
2-[4-(2-Chlor-phenyl)-piperazin-1-ylmethyl]-1-(3-methyl-benzyl)-cyclohexanol
1-(4-Chlor-benzyl)-2-[4-(2-chlor-phenyl)-piperazin-1-ylmethyl]-cyclohexanol
1-(2-Chlor-6-fluor-benzyl)-2-[4-(2-chlor-phenyl)-piperazin-1-ylmethyl]-cyclohexanol
2-[4-(2-Chlor-phenyl)-piperazin-1-ylmethyl]-1-(2,5-dimethyl-benzyl)-cyclohexanol
1-(3-Chlor-benzyl)-2-[4-(2-chlor-phenyl)-piperazin-1-ylmethyl]-cyclohexanol
2-[4-(2-Chlor-phenyl)-piperazin-1-ylmethyl]-1-(2,4-dichlor-benzyl)-cyclohexanol
2-[4-(2-Methoxy-phenyl)-piperazin-1-ylmethyl]-1-phenyl-cyclohexanol
1-(4-Chlor-phenyl)-2-[4-(2-methoxy-phenyl)-piperazin-1-ylmethyl]-cyclohexanol
1-Benzyl-2-[4-(2-methoxy-phenyl)-piperazin-1-ylmethyl]-cyclohexanol
1-(4-Fluor-3-methyl-phenyl)-2-[4-(2-methoxy-phenyl)-piperazin-1-ylmethyl]-cyclohexanol
2-[4-(2-Methoxy-phenyl)-piperazin-1-ylmethyl]-1-o-tolyl-cyclohexanol
2-[4-(2-Methoxy-phenyl)-piperazin-1-ylmethyl]-1-vinyl-cyclohexanol 1-(4-tert-Butyl-phenyl)-2-[4-(2-methoxy-phenyl)-piperazin-1-ylmethyl]-cyclohexanol
1-Cyclopentyi-2-[4-(2-methoxy-phenyl)-piperazin-1-ylmethyl]-cyclohexanol
2-[4-(2-Methoxy-phenyl)-piperazin-1-ylmethyl]-1-m-tolyl-cydohexanol
2-[4-(2-Methoxy-phenyl)-piperazin-1-ylmethyl]-bicyclohexyl-1-ol
1-(4-Fluor-phenyl)-2-[4-(2-methoxy-phenyl)-piperazin-1-ylmethyl]-cyclohexanol
2-[4-(2-Methoxy-phenyl)-piperazin-1-ylmethyl]-1-phenethyl-cyclohexanol
2-[4-(2-Methoxy-phenyl)-piperazin-1-ylmethyl]-1-phenylethynyl-cyclohexanol
2-[4-(2-Methoxy-phenyl)-piperazin-1-ylmethyl]-1-thiophen-2-yl-cyclohexanol
1-(3-Methoxy-phenyl)-2-[4-(2-methoxy-phenyl)-piperazin-1-ylmethyl]-cyclohexanol
2-[4-(2-Methoxy-phenyl)-piperazin-1-ylmethyl]-1-(3-phenyl-propyl)-cyclohexanol
1-(2,3-Dichlor-phenyl)-2-[4-(2-methoxy-phenyl)-piperazin-1-ylmethyl]-cyclohexanol
2-[4-(2-Methoxy-phenyl)-piperazin-1-ylmethyl]-1-p-tolyl-cyclohexanol
1-(4-Methoxy-phenyl)-2-[4-(2-methoxy-phenyl)-piperazin-1-ylmethyl]-cyclohexanol
1-Cyclohexylmethyl-2-[4-(2-methoxy-phenyl)-piperazin-1-ylmethyl]-cyclohexanol
1-(5-Fluor-2-methoxy-phenyl)-2-[4-(2-methoxy-phenyl)-piperazin-1-ylmethyl] cyclohexanol
1-(3-Fluor-phenyl)-2-[4-(2-methoxy-phenyl)-piperazin-1-ylmethyl]-cyclohexanol
1-(3-Chlor-phenyl)-2-[4-(2-methoxy-phenyl)-piperazin-1-ylmethyl]-cyclohexanol
1-(3,5-Dichlor-phenyl)-2-[4-(2-methoxy-phenyl)-piperazin-1-ylmethyl]-cyclohexanol
1-(2-Chlor-benzyl)-2-[4-(2-methoxy-phenyl)-piperazin-1-ylmethyl]-cyclohexanol
1-(4-Fluor-benzyl)-2-[4-(2-methoxy-phenyl)-piperazin-1-ylmethyl]-cyclohexanol
1-(3-Methoxy-benzyl)-2-[4-(2-methoxy-phenyl)-piperazin-1-ylmethyl]-cyclohexanol
1-(4-Chlor-3-trifluormethyl-phenyl)-2-[4-(2-methoxy-phenyl)-piperazin-1-ylmethyl]-cyclohexanol
1-(3-Fluor-benzyl)-2-[4-(2-methoxy-phenyl)-piperazin-1-ylmethyl]-cyclohexanol
1-(2-Methoxy-phenyl)-2-[4-(2-methoxy-phenyl)-piperazin-1-ylmethyl]-cyclohexanol
2-[4-(2-Methoxy-phenyl)-piperazin-1-ylmethyl]-1-(2-methyl-benzyl)-cyclohexanol
1-(3-Chlor-4-fluor-phenyl)-2-[4-(2-methoxy-phenyl)-piperazin-1-ylmethyl]-cyclohexanol
2-[4-(2-Methoxy-phenyl)-piperazin-1-ylmethyl]-1-(3-trifluormethyl-phenyl)-cyclohexanol
2-[4-(2-Methoxy-phenyl)-piperazin-1-ylmethyl]-1-(3-methyl-benzyl)-cyclohexanol
1-(4-Chlor-benzyl)-2-[4-(2-methoxy-phenyl)-piperazin-1-ylmethyl]-cyclohexanol
1-(2-Chlor-6-fluor-benzyl)-2-[4-(2-methoxy-phenyl)-piperazin-1-ylmethyl]-cyclohexanol
1-(2,5-Dimethyl-benzyl)-2-[4-(2-methoxy-phenyl)-piperazin-1-ylmethyl]-cyclohexanol,
1-(3-Chlor-benzyl)-2-[4-(2-methoxy-phenyl)-piperazin-1-ylmethyl]-cyclohexanol
1-(2,4-Dichlor-benzyl)-2-[4-(2-methoxy-phenyl)-piperazin-1-ylmethyl]-cyclohexanol
1-Benzyl-2-[4-(2-methoxy-phenyl)-piperazin-1-ylmethyl]-cyclododecanol
2-[4-(2-Methoxy-phenyl)-piperazin-1-ylmethyl]-1-vinyl-cyclododecanol
1-Phenethyl-2-[phenyl-(4-phenyl-piperazin-1-yl)-methyl]-cyclohexanol
1-Benzyl-2-[(4-methyl-piperazin-1-yl)-phenyl-methyl]-cyclohexanol
1-Benzyl-2-[phenyl-(4-phenyl-piperazin-1-yl)-methyl]-cyclohexanol
2-[(4-Methyl-piperazin-1-yl)-phenyl-methyl]-1-phenethyl-cyclohexanol
2-[4-(2-Methoxy-phenyl)-piperazin-1-ylmethyl]-1-phenethyl-cyclododecanol
in Form des Diastereomeren, des Enantiomeren und eines Gemisches davon - einschließlich des Racemats - sowie in Form der entsprechenden Base, des entsprechenden Salzes und des entsprechenden Solvats.

15. Verfahren zur Herstellung von N,N'-disubstituierten Piperazin-Verbindungen gemäß einem der Ansprüche 1-14, **dadurch gekennzeichnet, daß** man
A₁) ein Keton der Formel (1), worin R¹ und R² die in Anspruch 1 genannte Bedeutung haben, 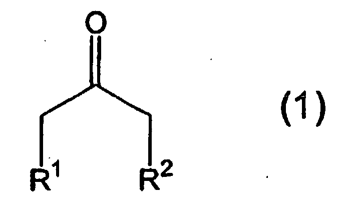 mit Paraformaldehyd und einen Piperazin der Formel (2), worin R⁵ die in Anspruch 1 genannte Bedeutung hat, 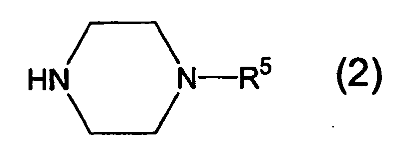 gemäß einer Mannich-Reaktion in einem geeigneten Lösungsmittel, vorzugsweise in Ethanol unter Zusatz von Salzsäure oder in Essigsäure, unter Erhitzen umsetzt, anschließend das Reaktionsgemisch aufarbeitet, das Produkt der Formel (3) isoliert und gegebenenfalls reinigt oder 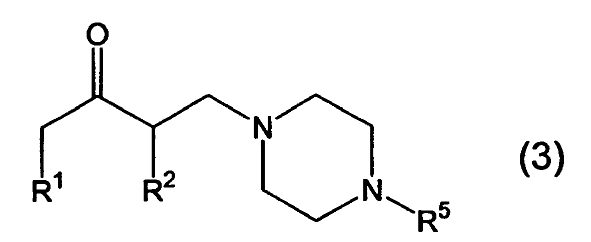
A₂) ein Enamin der Formel (1a), worin R¹ und R² die in Anspruch 1 genannte Bedeutung haben und R für einen aliphatischen C₁₋₆-Rest, einen Morpholinyl-, Piperidyl- oder Pyrrolidinylrest steht, wobei die beiden Reste R gleich oder verschieden sein können, 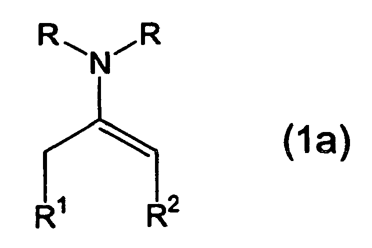 mit einem Aldehyd der Formel (4), worin R⁴ die in Anspruch 1 genannte Bedeutung mit Ausnahme von Wasserstoff hat, 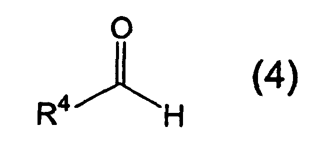 und einen Piperazin der Formel (2), worin R⁵ die in Anspruch 1 genannte Bedeutung hat, gegebenenfalls in Form seines Hydrochlorids 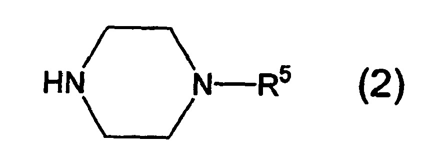 gemäß einer Mannich-Reaktion in Gegenwart von Triethylamin, Chlortrimethylsilan und Natriumiodid in einem geeigneten Lösungsmittel, vorzugsweise in Acetonitril, umsetzt, anschließend das Reaktionsgemisch aufarbeitet, das Produkt der Formel (3a) isoliert und gegebenenfalls reinigt oder 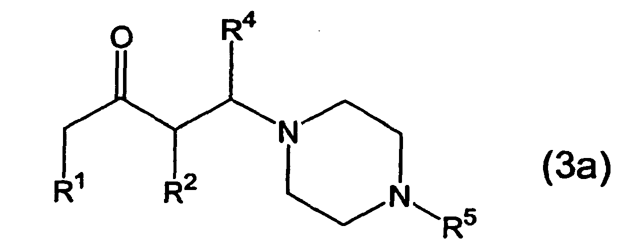
A₃) ein Enamin der Formel (1a), worin R¹ und R² die in Anspruch 1 genannte Bedeutung haben und R für einen aliphatischen C₁₋₆-Rest, einen Morpholinyl-, Piperidyl- oder Pyrrolidinylrest steht, wobei die beiden Reste R gleich oder verschieden sein können, 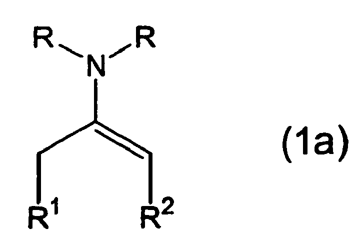 mit einem Iminiumsalz der Formel (5), worin R⁴ die in Anspruch 1 genannte Bedeutung mit Ausnahme von Wasserstoff und R⁵ die in Anspruch 1 genannte Bedeutung haben und Y⁻ für ein Chlorid-, Bromid-, lodid- oder AlCl₄⁻-Ion steht, 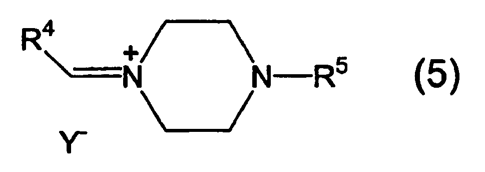 gemäß einer Mannich-Reaktion in einem geeigneten Lösungsmittel, vorzugsweise in Acetonitril, umsetzt, anschließend das Reaktionsgemisch aufarbeitet, das Produkt der Formel (3a) isoliert und gegebenenfalls reinigt und 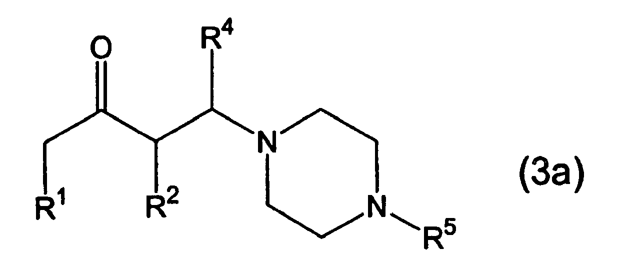
B) eine Verbindung der Formel (3) oder (3a) mit einer Grignard-Verbindung oder einer lithiumorganischen Verbindung der Formeln R³MgCl, R³MgBr, R³MgI, MgR³₂ oder LiR³, worin R³ die in Anspruch 1 genannte Bedeutung hat, in einem geeigneten Lösungsmittel, vorzugsweise Diethylether oder Tetrahydrofuran, umsetzt, anschließend das Reaktionsgemisch aufarbeitet, die Verbindung der allgemeinen Formel I isoliert und gegebenenfalls reinigt.

16. Arzneimittel enthaltend wenigstens eine Verbindung gemäß einem der Ansprüche 1-14 als Wirkstoff und gegebenenfalls physiologisch verträgliche Hilfsstoffe.

17. Arzneimittel gemäß Anspruch 16 zur Bekämpfung von Schmerz.

18. Arzneimittel gemäß Anspruch 16 zur Lokalanästesie.

19. Arzneimittel gemäß Anspruch 16 als Antiarrhythmikum.

20. Arzneimittel gemäß Anspruch 16 als Antiemetikum.

21. Arzneimittel gemäß Anspruch 16 als Nootropikum (Neurotropikum).

22. Arzneimittel gemäß Anspruch 16 zur Behandlung von cardiovaskulären Erkrankungen.

23. Arzneimittel gemäß Anspruch 16 zur Behandlung von Haminkontinenz.

24. Arzneimittel gemäß Anspruch 16 zur Behandlung von Diarrhöe.

25. Arzneimittel gemäß Anspruch 16 zur Behandlung von Pruritus.

26. Arzneimittel gemäß Anspruch 16 zur Behandlung von Alkoholabhängigkeit.

27. Arzneimittel gemäß Anspruch 16 zur Behandlung von Drogenabhängigkeit.

28. Arzneimittel gemäß Anspruch 16 zur Behandlung von Medikamentenabhängigkeit.

29. Arzneimittel gemäß Anspruch 16 zur Behandlung von Entzündungen.

30. Arzneimittel gemäß Anspruch 16 zur Behandlung von Depressionen.

31. Arzneimittel gemäß Anspruch 16 zur Vigilanzsteigerung.

32. Arzneimittel gemäß Anspruch 16 zur Libidosteigerung.

33. Verwendung wenigstens einer Verbindung gemäß einem der Ansprüche 1-14 zur Herstellung eines Arzneimittels zur Bekämpfung von Schmerz.

34. Verwendung wenigstens einer Verbindung gemäß einem der Ansprüche 1-14 zur Herstellung eines Arzneimittels zur Behandlung von Depressionen.

35. Verwendung wenigstens einer Verbindung gemäß einem der Ansprüche 1-14 zur Herstellung eines Arzneimittels zur Lokalanästesie und/oder zur Behandlung von Arrhythmien, Emesis, cardiovaskulären Erkrankungen, Haminkontinenz, Diarrhöe, Pruritus, Entzündungen, Alkohol- und/oder Drogen- und/oder Medikamentenabhängigkeit, zur Antriebssteigerung, Vigilanzsteigerung und/oder Libidosteigerung.

## Claims

1. N,N'-disubstituted piperazine compounds of the general formula I, 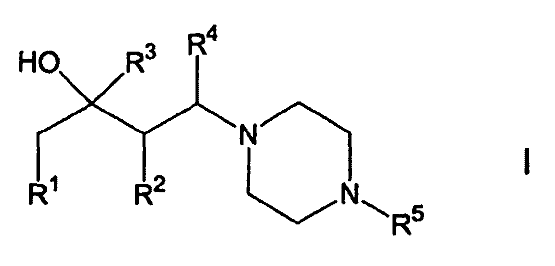
in which
R¹ and R², identical or different, in each case denote a linear or branched, saturated or unsaturated aliphatic residue, or together form a (CH₂)ₙ chain, wherein n denotes an integer,
R³ and R⁵, identical or different, in each case denote a linear or branched, saturated or unsaturated aliphatic residue, a saturated or unsaturated cycloaliphatic residue, an aryl residue or a heteroaryl residue, wherein the respective ring system may be optionally mono- or polysubstituted and/or attached via a linear or branched, saturated or unsaturated aliphatic bridge and/or the aryl or heteroaryl residue may be part of a polycyclic system,
R⁴ denotes hydrogen or an optionally identically or differently mono- or polysubstituted aryl or heteroaryl residue, wherein the aryl or heteroaryl residue may be part of a polycyclic system,
in the form of the diastereomers thereof, the enantiomers thereof and mixtures thereof - including the racemates thereof - and in the form of corresponding bases, salts and solvates.

2. Compounds according to claim 1, **characterised in that**
R¹ and R², identical or different, in each case denote a linear or branched, saturated or unsaturated aliphatic C₁₋₆ residue, or together form a (CH₂)ₙ chain, wherein n denotes an integer from 2-9,
R³ denotes a linear or branched, saturated or unsaturated aliphatic C₁₋₆ residue, a saturated or unsaturated cycloaliphatic C₃₋₇ residue, a phenyl residue or a five- or six-membered heteroaryl residue, wherein the respective ring system may optionally be mono- or polysubstituted and/or attached via a linear or branched, saturated or unsaturated aliphatic C₁₋₅ bridge,
R⁴ denotes hydrogen or a phenyl residue,
R⁵ denotes a linear or branched, saturated or unsaturated aliphatic C₁₋₆ residue or an optionally substituted phenyl residue.

3. Compounds according to claim 1, **characterised in that**
R¹ and R², identical or different, in each case denote a linear or branched, saturated or unsaturated aliphatic C₁₋₃ residue, or together form a (CH₂)ₙ chain, wherein n denotes an integer from 2-9,
R³ denotes a linear or branched, saturated or unsaturated aliphatic C₁₋₃ residue, a saturated or unsaturated cycloaliphatic C₅₋₆ residue, a phenyl residue or a five- or six-membered heteroaryl residue, wherein the respective ring system may optionally be mono- or polysubstituted with halogen, an alkyl group, an alkoxy group and/or a trihalogenated alkyl group and/or may be attached via a linear or branched, saturated or unsaturated aliphatic C₁₋₃ bridge,
R⁴ denotes hydrogen or a phenyl residue,
R⁵ denotes a linear or branched, saturated or unsaturated aliphatic C₁₋₃ residue or a phenyl residue optionally substituted with halogen or an alkoxy group.

4. Compounds according to claim 1, **characterised in that**
R¹ and R² in each case denote a methyl group, or together form a (CH₂)ₙ chain, wherein n denotes 3, 4, 5 or 9,
R³ denotes a vinyl residue, a cyclopentyl residue, a cyclohexyl residue, a thiophenyl residue or a phenyl residue, wherein the cyclohexyl residue may optionally be attached via a methylene bridge or the phenyl residue may optionally be mono- or polysubstituted with fluorine, chlorine, a methyl group, an isopropyl group, a methoxy group and/or a trifluoromethyl group and/or may optionally be attached via a linear saturated aliphatic C₁₋₃ bridge or an ethynyl bridge,
R⁴ denotes hydrogen or a phenyl residue,
R⁵ denotes a methyl group or a phenyl residue optionally substituted with chlorine or a methoxy group.

5. Compounds according to claim 1 corresponding to the general formula II

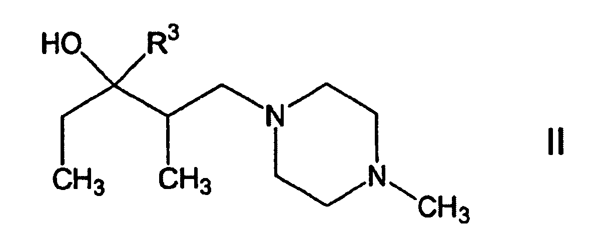

6. Compounds according to claim 1 corresponding to the general formula III, 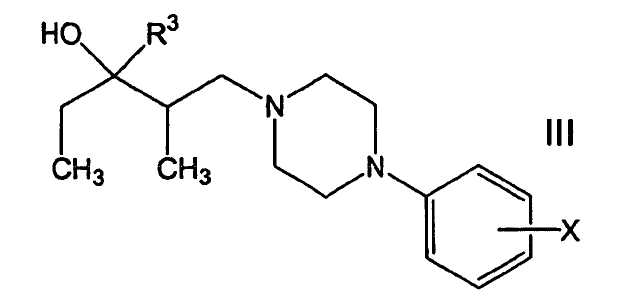
in which
X denotes hydrogen, halogen or an alkoxy group, preferably hydrogen, chlorine or a methoxy group.

7. Compounds according to claim 1 corresponding to the general formula IV, 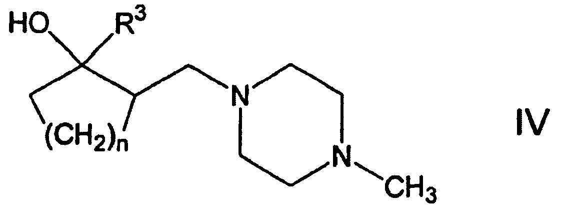
in which
n denotes an integer from 2-9.

8. Compounds according to claim 1 corresponding to the general formula V, 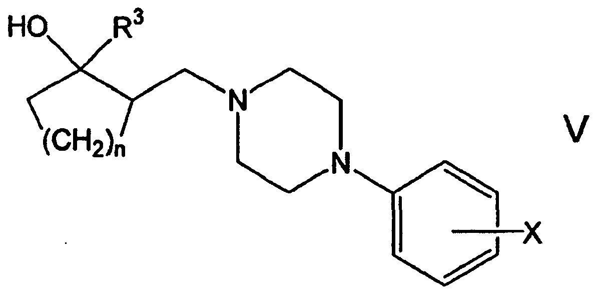
in which
n denotes an integer from 2-9 and
X denotes hydrogen, halogen or an alkoxy group, preferably hydrogen, chlorine or a methoxy group.

9. Compounds according to claim 1 corresponding to the general formula VI, 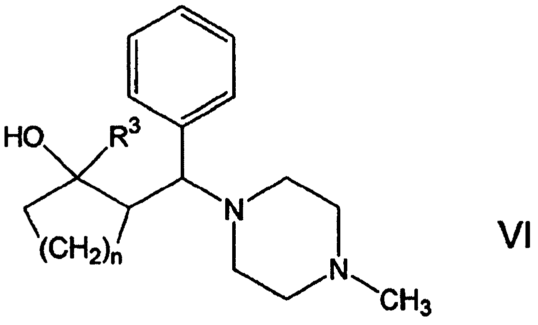
in which
n denotes an integer from 2-9.

10. Compounds according to claim 1 corresponding to the general formula VII, 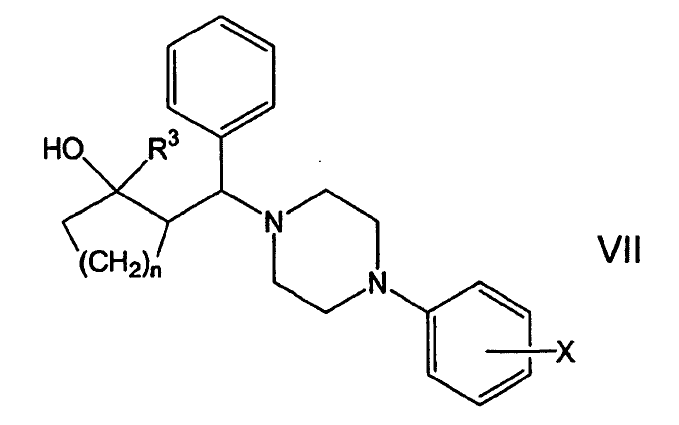
in which
n denotes an integer from 2-9 and
X denotes hydrogen, halogen or an alkoxy group, preferably hydrogen, chlorine or a methoxy group.

11. Compounds according to any one of claims 5-10,
**characterised in that**
R³ denotes a linear or branched, saturated or unsaturated aliphatic C₁₋₆ residue, a saturated or unsaturated cycloaliphatic C₃₋₇ residue, a phenyl residue or a five- or six-membered heteroaryl residue, wherein the respective ring system may optionally be mono- or polysubstituted and/or attached via a linear or branched, saturated or unsaturated aliphatic C₁₋₅ bridge.

12. Compounds according to any one of claims 5-10,
**characterised in that**
R³ denotes a linear or branched, saturated or unsaturated aliphatic C₁₋₃ residue, a saturated or unsaturated cycloaliphatic C₅₋₆ residue, a phenyl residue or a five- or six-membered heteroaryl residue, wherein the respective ring system may optionally be mono- or polysubstituted with halogen, an alkyl group, an alkoxy group and/or a trihalogenated alkyl group and/or may be attached via a linear or branched, saturated or unsaturated aliphatic C₁₋₃ bridge.

13. Compounds according to any one of claims 5-10,
**characterised in that**
R³ denotes a vinyl residue, a cyclopentyl residue, a cyclohexyl residue, a thiophenyl residue or a phenyl residue, wherein the cyclohexyl residue may optionally be attached via a methylene bridge or the phenyl residue may optionally be mono- or polysubstituted with fluorine, chlorine, a methyl group, an isopropyl group, a methoxy group and/or a trifluoromethyl group and/or may optionally be attached via a linear saturated aliphatic C₁₋₃ bridge or an ethynyl bridge.

14. An N,N'-disubstituted piperazine compound according to claim 1 selected from the group comprising
2-Methyl-1-(4-methyl-piperazin-1-yl)-3-phenyl-pentan-3-ol
3-(4-Chloro-phenyl)-2-methyl-1-(4-methyl-piperazin-1-yl)-pentan-3-ol
3-Benzyl-2-methyl-1-(4-methyl-piperazin-1-yl)-pentan-3-ol
3-(4-Fluoro-3-methyl-phenyl)-2-methyl-1-(4-methyl-piperazin-1-yl)-pentan-3-ol
2-Methyl-1-(4-methyl-piperazin-1-yl)-3-o-tolyl-pentan-3-ol
3-Ethyl-4-methyl-5-(4-methyl-piperazin-1-yl)-pent-1-en-3-ol
3-(4-tert-Butyl-phenyl)-2-methyl-1-(4-methyl-piperazin-1-yl)-pentan-3-ol
3-Cyclopentyl-2-methyl-1-(4-methyl-piperazin-1-yl)-pentan-3-ol
2-Methyl-1-(4-methyl-piperazin-1-yl)-3-m-tolyl-pentan-3-ol
3-Cyclohexyl-2-methyl-1-(4-methyl-piperazin-1-yl)-pentan-3-ol
3-(4-Fluoro-phenyl)-2-methyl-1-(4-methyl-piperazin-1-yl)-pentan-3-ol
3-Ethyl-2-methyl-1-(4-methyl-piperazin-1-yl)-5-phenylpentan-3-ol
3-Ethyl-4-methyl-5-(4-methyl-piperazin-1-yl)-1-phenyl-pent-1-yn-3-ol
2-Methyl-1-(4-methyl-piperazin-1-yl)-3-thiophen-2-yl-pentan-3-ol
3-(3-Methoxy-phenyl)-2-methyl-1-(4-methyl-piperazin-1-yl)-pentan-3-ol
3-Ethyl-2-methyl-1-(4-methyl-piperazin-1-yl)-6-phenyl-hexan-3-ol
2-Methyl-1-(4-methyl-piperazin-1-yl)-3-p-tolyl-pentan-3-ol
3-(4-Methoxy-phenyl)-2-methyl-1-(4-methyl-piperazin-1-yl)-pentan-3-ol
2-(4-Methyl-piperazin-1-ylmethyl)-1-phenyl-cyclohexanol
1-Benzyl-2-(4-methyl-piperazin-1-ylmethyl)-cyclohexanol
1-(4-Fluoro-3-methyl-phenyl)-2-(4-methyl-piperazin-1-ylmethyl)-cyclohexanol
2-(4-Methyl-piperazin-1-ylmethyl)-1-o-tolylcyclohexanol
2-(4-Methyl-piperazin-1-ylmethyl)-1-vinyl-cyclohexanol
1-Cyclopentyl-2-(4-methyl-piperazin-1-ylmethyl)-cyclohexanol
2-(4-Methyl-piperazin-1-ylmethyl)-1-m-tolylcyclohexanol
2-(4-Methyl-piperazin-1-ylmethyl)-bicyclohexyl-1-ol
1-(4-Fluoro-phenyl)-2-(4-methyl-piperazin-1-ylmethyl)-cyclohexanol
2-(4-Methyl-piperazin-1-ylmethyl)-1-phenethylcyclohexanol
2-(4-Methyl-piperazin-1-ylmethyl)-1-thiophen-2-yl-cyclohexanol
1-(2,4-Dichloro-phenyl)-2-(4-methyl-piperazin-1-ylmethyl)-cyclohexanol
1-(3-Methoxy-phenyl)-2-(4-methyl-piperazin-1-ylmethyl)-cyclohexanol
2-(4-Methyl-piperazin-1-ylmethyl)-1-(3-phenyl-propyl)-cyclohexanol
2-(4-Methyl-piperazin-1-ylmethyl)-1-p-tolyl-cyclohexanol
1-(4-Methoxy-phenyl)-2-(4-methyl-piperazin-1-ylmethyl)-cyclohexanol
1-Benzyl-2-(4-methyl-piperazin-1-ylmethyl)-cyclooctanol
2-(4-Methyl-piperazin-1-ylmethyl)-1-vinyl-cyclooctanol
1-(4-tert-Butyl-phenyl)-2-(4-methyl-piperazin-1-ylmethyl)-cyclooctanol
1-Cyclopentyl-2-(4-methyl-piperazin-1-ylmethyl)-cyclooctanol
2-(4-Methyl-piperazin-1-ylmethyl)-1-phenethyl-cyclooctanol
2-(4-Methyl-piperazin-1-ylmethyl)-1-phenylethynyl-cyclooctanol
2-(4-Methyl-piperazin-1-ylmethyl)-1-thiophen-2-yl-cyclooctanol
1-(3-Methoxy-phenyl)-2-(4-methyl-piperazin-1-ylmethyl)-cyclooctanol
2-(4-Methyl-piperazin-1-ylmethyl)-1-(3-phenyl-propyl)-cyclooctanol
2-(4-Methyl-piperazin-1-ylmethyl)-1-p-tolyl-cyclooctanol
1-(4-Methoxy-phenyl)-2-(4-methyl-piperazin-1-ylmethyl)-cyclooctanol
2-(4-Methyl-piperazin-1-ylmethyl)-1-phenyl-cycloheptanol
1-(4-Chloro-phenyl)-2-(4-methyl-piperazin-1-ylmethyl)-cycloheptanol
1-Benzyl-2-(4-methyl-piperazin-1-ylmethyl)-cycloheptanol
1-(4-Fluoro-3-methyl-phenyl)-2-(4-methyl-piperazin-1-ylmethyl)-cycloheptanol
2-(9-Methyl-piperazin-1-ylmethyl)-1-o-tolyl-cycloheptanol
2-(4-Methyl-piperazin-1-ylmethyl)-1-vinyl-cycloheptanol
1-(4-tert-Butyl-phenyl)-2-(4-methyl-piperazin-1-ylmethyl)-cycloheptanol
1-Cyclopentyl-2-(4-methyl-piperazin-1-ylmethyl)-cycloheptanol
2-(4-Methyl-piperazin-1-ylmethyl)-1-m-tolyl-cycloheptanol
1-[4-(3-Chloro-phenyl)-piperazin-1-yl]-2-methyl-3-phenyl-pentan-3-ol
3-(4-Chloro-phenyl)-1-[4-(3-chloro-phenyl)-piperazin-1-yl]-2-methyl-pentan-3-ol
3-Benzyl-1-[4-(3-chloro-phenyl)-piperazin-1-yl]-2-methyl-pentan-3-ol
1-[4-(3-Chloro-phenyl)-piperazin-1-yl]-3-(4-fluoro-3-methyl-phenyl)-2-methyl-pentan-3-ol
5-[4-(3-Chloro-phenyl)-piperazin-1-yl]-3-ethyl-4-methyl-pent-1-en-3-ol
3-(4-tert-Butyl-phenyl)-1-[4-(3-chloro-phenyl)-piperazin-1-yl]-2-methyl-pentan-3-ol
1-[4-(3-Chloro-phenyl)-piperazin-1-yl]-2-methyl-3-m-tolyl-pentan-3-ol
1-[4-(3-Chloro-phenyl)-piperazin-1-yl]-3-cyclohexyl-2-methyl-pentan-3-ol
1-[4-(3-Chloro-phenyl)-piperazin-1-yl]-3-(4-fluorophenyl)-2-methyl-pentan-3-ol
1-[4-(3-Chloro-phenyl)-piperazin-1-yl]-3-ethyl-2-methyl-5-phenyl-pentan-3-ol
5-[4-(3-Chloro-phenyl)-piperazin-1-yl]-3-ethyl-4-methyl-1-phenyl-pent-1-yn-3-ol
1-[4-(3-Chloro-phenyl)-piperazin-1-yl]-2-methyl-3-thiophen-2-yl-pentan-3-ol
1-[4-(3-Chloro-phenyl)-piperazin-1-yl]-3-(3-methoxyphenyl)-2-methyl-pentan-3-ol
1-[4-(3-Chloro-phenyl)-piperazin-1-yl]-3-ethyl-2-methyl-6-phenyl-hexan-3-ol
1-[4-(3-Chloro-phenyl)-piperazin-1-yl]-2-methyl-3-p-tolyl-pentan-3-ol
1-[4-(3-Chloro-phenyl)-piperazin-1-yl]-3-(4-methoxyphenyl)-2-methyl-pentan-3-ol
1-[4-(3-Chloro-phenyl)-piperazin-1-yl]-3-(5-fluoro-2-methoxy-phenyl)-2-methyl-pentan-3-ol
1-[4-(3-Chloro-phenyl)-piperazin-1-yl]-3-(3-fluorophenyl)-2-methyl-pentan-3-ol
3-(3-Chloro-phenyl)-1-[4-(3-chloro-phenyl)-piperazin-1-yl]-2-methyl-pentan-3-ol
3-(2-Chloro-benzyl)-1-[4-(3-chloro-phenyl)-piperazin-1-yl]-2-methyl-pentan-3-ol
1-[4-(3-Chloro-phenyl)-piperazin-1-yl]-3-(4-fluorobenzyl)-2-methyl-pentan-3-ol
1-[4-(3-Chloro-phenyl)-piperazin-1-yl]-3-(3-methoxybenzyl)-2-methyl-pentan-3-ol
1-[4-(3-Chloro-phenyl)-piperazin-1-yl]-3-(3-fluorobenzyl)-2-methyl-pentan-3-ol
1-[4-(3-Chloro-phenyl)-piperazin-1-yl]-3-(2-methoxyphenyl)-2-methyl-pentan-3-ol
1-[4-(3-Chloro-phenyl)-piperazin-1-yl]-2-methyl-3-(2-methyl-benzyl)-pentan-3-ol
1-[4-(3-Chloro-phenyl)-piperazin-1-yl]-2-methyl-3-(3-trifluoromethyl-phenyl)-pentan-3-ol
1-[4-(3-Chloro-phenyl)-piperazin-1-yl]-2-methyl-3-(3-methyl-benzyl)-pentan-3-ol
3-(4-Chloro-benzyl)-1-[4-(3-chloro-phenyl)-piperazin-1-yl]-2-methyl-pentan-3-ol
3-(2-Chloro-6-fluoro-benzyl)-1-[4-(3-chloro-phenyl)-piperazin-1-yl]-2-methyl-pentan-3-ol
1-[4-(3-Chloro-phenyl)-piperazin-1-yl]-3-(2,5-dimethyl-benzyl)-2-methyl-pentan-3-ol
3-(3-Chloro-benzyl)-1-[4-(3-chloro-phenyl)-piperazin-1-yl]-2-methyl-pentan-3-ol
1-[4-(3-Chloro-phenyl)-piperazin-1-yl]-3-(2,4-dichloro-benzyl)-2-methyl-pentan-3-ol
3-Cyclohexylmethyl-2-methyl-1-(4-methyl-piperazin-1-yl)-pentan-3-ol
3-(5-Fluoro-2-methoxy-phenyl)-2-methyl-1-(4-methyl-piperazin-1-yl)-pentan-3-ol
3-(3-Fluoro-phenyl)-2-methyl-1-(4-methyl-piperazin-1-yl)-pentan-3-ol
3-(3-Chloro-phenyl)-2-methyl-1-(4-methyl-piperazin-1-yl)-pentan-3-ol
3-(3,5-Dichloro-phenyl)-2-methyl-1-(4-methyl-piperazin-1-yl)-pentan-3-ol
3-(2-Chloro-benzyl)-2-methyl-1-(4-methyl-piperazin-1-yl)-pentan-3-ol
3-(4-Fluoro-benzyl)-2-methyl-1-(4-methyl-piperazin-1-yl)-pentan-3-ol
3-(3-Methoxy-benzyl)-2-methyl-1-(4-methyl-piperazin-1-yl)-pentan-3-ol
3-(4-Chloro-3-trifluoromethyl-phenyl)-2-methyl-1-(4-methyl-piperazin-1-yl)-pentan-3-ol
3-(3-Fluoro-benzyl)-2-methyl-l-(4-methyl-piperazin-l-yl)-pentan-3-ol
3-(2-Methoxy-phenyl)-2-methyl-1-(4-methyl-piperazin-1-yl)-pentan-3-ol
2-Methyl-3-(2-methyl-benzyl)-1-(4-methyl-piperazin-1-yl)-pentan-3-ol
3-(3-Chloro-4-fluoro-phenyl)-2-methyl-1-(4-methyl-piperazin-1-yl)-pentan-3-ol
2-Methyl-1-(4-methyl-piperazin-1-yl)-3-(3-trifluoromethyl-phenyl)-pentan-3-ol
2-Methyl-3-(3-methyl-benzyl)-1-(4-methyl-piperazin-1-yl)-pentan-3-ol
3-(4-Chloro-benzyl)-2-methyl-1-(4-methyl-piperazin-1-yl)-pentan-3-ol
3-(2-Chloro-6-fluoro-benzyl)-2-methyl-1-(4-methyl-piperazin-1-yl)-pentan-3-ol
3-(2,5-Dimethyl-benzyl)-2-methyl-1-(4-methyl-piperazin-1-yl)-pentan-3-ol
3-(3-Chloro-benzyl)-2-methyl-1-(4-methyl-piperazin-1-yl)-pentan-3-ol
3-(2,4-Dichloro-benzyl)-2-methyl-1-(4-methyl-piperazin-1-yl)-pentan-3-ol
1-Cyclohexylmethyl-2-(4-methyl-piperazin-1-ylmethyl)-cyclohexanol
1-(5-Fluoro-2-methoxy-phenyl)-2-(4-methyl-piperazin-1-ylmethyl)-cyclohexanol
1-(3-Fluoro-phenyl)-2-(4-methyl-piperazin-1-ylmethyl)-cyclohexanol
1-(3-Chloro-phenyl)-2-(4-methyl-piperazin-1-ylmethyl)-cyclohexanol
1-(3,5-Dichloro-phenyl)-2-(4-methyl-piperazin-1-ylmethyl)-cyclohexanol
1-(2-Chloro-benzyl)-2-(4-methyl-piperazin-1-ylmethyl)-cyclohexanol
1-(4-Fluoro-benzyl)-2-(4-methyl-piperazin-1-ylmethyl)-cyclohexanol
1-(3-Methoxy-benzyl)-2-(4-methyl-piperazin-1-ylmethyl)-cyclohexanol
1-(4-Chloro-3-trifluoromethyl-phenyl)-2-(4-methyl-piperazin-1-ylmethyl)-cyclohexanol
1-(3-Fluoro-benzyl)-2-(4-methyl-piperazin-1-ylmethyl)-cyclohexanol
1-(2-Methoxy-phenyl)-2-(4-methyl-piperazin-1-ylmethyl)-cyclohexanol
1-(2-Methyl-benzyl)-2-(4-methyl-piperazin-1-ylmethyl)-cyclohexanol
1-(3-Chloro-4-fluoro-phenyl)-2-(4-methyl-piperazin-1-ylmethyl)-cyclohexanol
2-(4-Methyl-piperazin-1-ylmethyl)-1-(3-trifluoromethyl-phenyl)-cyclohexanol
1-(3-Methyl-benzyl)-2-(4-methyl-piperazin-1-ylmethyl)-cyclohexanol
1-(4-Chloro-benzyl)-2-(4-methyl-piperazin-1-ylmethyl)-cyclohexanol
1-(2,5-Dimethyl-benzyl)-2-(4-methyl-piperazin-1-ylmethyl)-cyclohexanol
1-(3-Chloro-benzyl)-2-(4-methyl-piperazin-1-ylmethyl)-cyclohexanol
1-(2,4-Dichloro-benzyl)-2-(4-methyl-piperazin-1-ylmethyl)-cyclohexanol
1-Cyclohexylmethyl-2-(4-methyl-piperazin-1-ylmethyl)-cyclooctanol
1-(5-Fluoro-2-methoxy-phenyl)-2-(4-methyl-piperazin-1-ylmethyl)-cyclooctanol
1-(2-Chloro-benzyl)-2-(4-methyl-piperazin-1-ylmethyl)-cyclooctanol
1-(4-Fluoro-benzyl)-2-(4-methyl-piperazin-1-ylmethyl)-cyclooctanol
1-(3-Fluoro-benzyl)-2-(4-methyl-piperazin-1-ylmethyl)-cyclooctanol
1-(2-Methoxy-phenyl)-2-(4-methyl-piperazin-1-ylmethyl)-cyclooctanol
1-(3-Methyl-benzyl)-2-(4-methyl-piperazin-1-ylmethyl)-cyclooctanol
1-(4-Chloro-benzyl)-2-(4-methyl-piperazin-1-ylmethyl)-cyclooctanol
1-(2-Chloro-6-fluoro-benzyl)-2-(4-methyl-piperazin-1-ylmethyl)-cyclooctanol
1-(2,5-Dimethyl-benzyl)-2-(4-methyl-piperazin-1-ylmethyl)-cyclooctanol
1-(3-Chloro-benzyl)-2-(4-methyl-piperazin-1-ylmethyl)-cyclooctanol
1-(2,4-Dichloro-benzyl)-2-(4-methyl-piperazin-1-ylmethyl)-cyclooctanol
1-Cyclohexylmethyl-2-(4-methyl-piperazin-1-ylmethyl)-cycloheptanol
1-(5-Fluoro-2-methoxy-phenyl)-2-(4-methyl-piperazin-1-ylmethyl)-cycloheptanol
1-(3-Fluoro-phenyl)-2-(4-methyl-piperazin-1-ylmethyl)-cycloheptanol
1-(3-Chloro-phenyl)-2-(4-methyl-piperazin-1-ylmethyl)-cycloheptanol
1-(3,5-Dichloro-phenyl)-2-(4-methyl-piperazin-1-ylmethyl)-cycloheptanol
1-(2-Chloro-benzyl)-2-(4-methyl-piperazin-1-ylmethyl)-cycloheptanol
1-(4-Fluoro-benzyl)-2-(4-methyl-piperazin-1-ylmethyl)-cycloheptanol
1-(3-Methoxy-benzyl)-2-(4-methyl-piperazin-1-ylmethyl)-cycloheptanol
1-(4-Chloro-3-trifluoromethyl-phenyl)-2-(4-methyl-piperazin-1-ylmethyl)-cycloheptanol
1-(3-Fluoro-benzyl)-2-(4-methyl-piperazin-1-ylmethyl)-cycloheptanol
1-(2-Methoxy-phenyl)-2-(4-methyl-piperazin-1-ylmethyl)-cycloheptanol
1-(2-Methyl-benzyl)-2-(4-methyl-piperazin-1-ylmethyl)-cycloheptanol
1-(3-Chloro-4-fluoro-phenyl)-2-(4-methyl-piperazin-1-ylmethyl)-cycloheptanol
2-(4-Methyl-piperazin-1-ylmethyl)-1-(3-trifluoromethyl-phenyl)-cycloheptanol
1-(3-Methyl-benzyl)-2-(4-methyl-piperazin-1-ylmethyl)-cycloheptanol
1-(4-Chloro-benzyl)-2-(4-methyl-piperazin-1-ylmethyl)-cycloheptanol
1-(2-Chloro-6-fluoro-benzyl)-2-(4-methyl-piperazin-1-ylmethyl)-cycloheptanol
1-(2,5-Dimethyl-benzyl)-2-(4-methyl-piperazin-1-ylmethyl)-cycloheptanol
1-(3-Chloro-benzyl)-2-(4-methyl-piperazin-1-ylmethyl)-cycloheptanol
1-(2,4-Dichloro-benzyl)-2-(4-methyl-piperazin-1-ylmethyl)-cycloheptanol
1-Benzyl-2-(4-methyl-piperazin-1-ylmethyl)-cyclododecanol
2-(4-Methyl-piperazin-1-ylmethyl)-1-vinyl-cyclododecanol
1-Cyclopentyl-2-(4-methyl-piperazin-1-ylmethyl)-cyclododecanol
2-(4-Methyl-piperazin-1-ylmethyl)-1-phenethyl-cyclododecanol
2-(4-Methyl-piperazin-1-ylmethyl)-1-phenylethynyl-cyclododecanol
2-(4-Methyl-piperazin-1-ylmethyl)-1-thiophen-2-yl-cyclododecanol
2-(4-Methyl-piperazin-1-ylmethyl)-1-(3-phenyl-propyl)-cyclododecanol
1-(5-Fluoro-2-methoxy-phenyl)-2-(4-methyl-piperazin-1-ylmethyl)-cyclododecanol
1-(2-Chloro-benzyl)-2-(4-methyl-piperazin-1-ylmethyl)-cyclododecanol
1-(4-Fluoro-benzyl)-2-(4-methyl-piperazin-1-ylmethyl)-cyclododecanol
1-(3-Methoxy-benzyl)-2-(4-methyl-piperazin-1-ylmethyl)-cyclododecanol
1-(3-Fluoro-benzyl)-2-(4-methyl-piperazin-1-ylmethyl)-cyclododecanol
1-(2-Methoxy-phenyl)-2-(4-methyl-piperazin-1-ylmethyl)-cyclododecanol
1-(2-Methyl-benzyl)-2-(4-methyl-piperazin-1-ylmethyl)-cyclododecanol
1-(3-Methyl-benzyl)-2-(4-methyl-piperazin-1-ylmethyl)-cyclododecanol
1-(4-Chloro-benzyl)-2-(4-methyl-piperazin-1-ylmethyl)-cyclododecanol
1-(2,5-Dimethyl-benzyl)-2-(4-methyl-piperazin-1-ylmethyl)-cyclododecanol
1-(3-Chloro-benzyl)-2-(4-methyl-piperazin-1-ylmethyl)-cyclododecanol
1-(2,4-Dichloro-benzyl)-2-(4-methyl-piperazin-1-ylmethyl)-cyclododecanol
2-[4-(3-Chloro-phenyl)-piperazin-1-ylmethyl]-1-cyclohexylmethyl-cyclohexanol
2-[4-(3-Chloro-phenyl)-piperazin-1-ylmethyl]-1-(5-fluoro-2-methoxy-phenyl)-cyclohexanol
2-[4-(3-Chloro-phenyl)-piperazin-1-ylmethyl]-1-(3-fluoro-phenyl)-cyclohexanol
1-(3-Chloro-phenyl)-2-[4-(3-chloro-phenyl)-piperazin-1-ylmethyl]-cyclohexanol
2-[4-(3-Chloro-phenyl)-piperazin-1-ylmethyl]-1-(3,5-dichloro-phenyl)-cyclohexanol
1-(2-Chloro-benzyl)-2-[4-(3-chloro-phenyl)-piperazin-1-ylmethyl]-cyclohexanol
2-[4-(3-Chloro-phenyl)-piperazin-1-ylmethyl]-1-(4-fluoro-benzyl)-cyclohexanol
2-[4-(3-Chloro-phenyl)-piperazin-1-ylmethyl]-1-(4-chloro-3-trifluoromethyl-phenyl)-cyclohexanol
2-[4-(3-Chloro-phenyl)-piperazin-1-ylmethyl]-1-(3-fluoro-benzyl)-cyclohexanol
2-[4-(3-Chloro-phenyl)-piperazin-1-ylmethyl]-1-(2-methoxy-phenyl)-cyclohexanol
1-(3-Chloro-4-fluoro-phenyl)-2-[4-(3-chloro-phenyl)-piperazin-1-ylmethyl]-cyclohexanol
2-[4-(3-Chloro-phenyl)-piperazin-1-ylmethyl]-1-(3-trifluoromethyl-phenyl)-cyclohexanol
2-[4-(3-Chloro-phenyl)-piperazin-1-ylmethyl]-1-(3-methyl-benzyl)-cyclohexanol
1-(4-Chloro-benzyl)-2-[4-(3-chloro-phenyl)-piperazin-1-ylmethyl]-cyclohexanol
1-(2-Chloro-6-fluoro-benzyl)-2-[4-(3-chloro-phenyl)-piperazin-1-ylmethyl]-cyclohexanol
2-[4-(3-Chloro-phenyl)-piperazin-1-ylmethyl]-1-(2,5-dimethyl-benzyl)-cyclohexanol
1-(3-Chloro-benzyl)-2-[4-(3-chloro-phenyl)-piperazin-1-ylmethyl]-cyclohexanol
2-[4-(2-Chloro-phenyl)-piperazin-1-ylmethyl]-1-phenyl-cyclohexanol
1-(4-Chloro-phenyl)-2-[4-(2-chloro-phenyl)-piperazin-1-ylmethyl]-cyclohexanol
1-Benzyl-2-[4-(2-chloro-phenyl)-piperazin-1-ylmethyl]-cyclohexanol
2-[4-(2-Chloro-phenyl)-piperazin-1-ylmethyl]-1-(4-fluoro-3-methyl-phenyl)-cyclohexanol
2-[4-(2-Chloro-phenyl)-piperazin-1-ylmethyl]-1-o-tolyl-cyclohexanol
2-[4-(2-Chloro-phenyl)-piperazin-1-ylmethyl]-1-vinyl-cyclohexanol
1-(4-tert-Butyl-phenyl)-2-[4-(2-chloro-phenyl)-piperazin-1-ylmethyl]-cyclohexanol
2-[4-(2-Chloro-phenyl)-piperazin-1-ylmethyl]-1-cyclopentyl-cyclohexanol
2-[4-(2-Chloro-phenyl)-piperazin-1-ylmethyl]-1-m-tolyl-cyclohexanol
2-[4-(2-Chloro-phenyl)-piperazin-1-ylmethyl]-bicyclohexyl-1-ol
2-[4-(2-Chloro-phenyl)-piperazin-1-ylmethyl]-1-(4-fluoro-phenyl)-cyclohexanol
2-[4-(2-Chloro-phenyl)-piperazin-1-ylmethyl]-1-phenethyl-cyclohexanol
2-[4-(2-Chloro-phenyl)-piperazin-1-ylmethyl]-1-phenylethynyl-cyclohexanol
2-[4-(2-Chloro-phenyl)-piperazin-1-ylmethyl]-1-thiophen-2-yl-cyclohexanol
2-[4-(2-Chloro-phenyl)-piperazin-1-ylmethyl]-1-(3-methoxy-phenyl)-cyclohexanol
2-[4-(2-Chloro-phenyl)-piperazin-1-ylmethyl]-1-(3-phenyl-propyl)-cyclohexanol
2-[4-(2-Chloro-phenyl)-piperazin-1-ylmethyl]-1-p-tolyl-cyclohexanol
2-[4-(2-Chloro-phenyl)-piperazin-1-ylmethyl]-1-(4-methoxy-phenyl)-cyclohexanol
2-[4-(2-Chloro-phenyl)-piperazin-1-ylmethyl]-1-cyclohexylmethyl-cyclohexanol
2-[4-(2-Chloro-phenyl)-piperazin-1-ylmethyl]-1-(5-fluoro-2-methoxy-phenyl)-cyclohexanol
1-(3-Chloro-phenyl)-2-[4-(2-chloro-phenyl)-piperazin-1-ylmethyl]-cyclohexanol
2-[4-(2-Chloro-phenyl)-piperazin-1-ylmethyl]-1-(3,5-dichloro-phenyl)-cyclohexanol
1-(2-Chloro-benzyl)-2-[4-(2-chloro-phenyl)-piperazin-1-ylmethyl]-cyclohexanol
2-[4-(2-Chloro-phenyl)-piperazin-1-ylmethyl]-1-(4-fluoro-benzyl)-cyclohexanol
2-[4-(2-Chloro-phenyl)-piperazin-1-ylmethyl]-1-(3-methoxy-benzyl)-cyclohexanol
2-[4-(2-Chloro-phenyl)-piperazin-1-ylmethyl]-1-(4-chloro-3-trifluoromethyl-phenyl)-cyclohexanol
2-[4-(2-Chloro-phenyl)-piperazin-1-ylmethyl]-1-(3-fluoro-benzyl)-cyclohexanol
2-[4-(2-Chloro-phenyl)-piperazin-1-ylmethyl]-1-(2-methoxy-phenyl)-cyclohexanol
2-[4-(2-Chloro-phenyl)-piperazin-1-ylmethyl]-1-(2-methyl-benzyl)-cyclohexanol
1-(3-Chloro-4-fluoro-phenyl)-2-[4-(2-chloro-phenyl)-piperazin-1-ylmethyl]-cyclohexanol
2-[4-(2-Chloro-phenyl)-piperazin-1-ylmethyl]-1-(3-trifluoromethyl-phenyl)-cyclohexanol
2-[4-(2-Chloro-phenyl)-piperazin-1-ylmethyl]-1-(3-methyl-benzyl)-cyclohexanol
1-(4-Chloro-benzyl)-2-[4-(2-chloro-phenyl)-piperazin-1-ylmethyl]-cyclohexanol
1-(2-Chloro-6-fluoro-benzyl)-2-[4-(2-chloro-phenyl)-piperazin-1-ylmethyl]-cyclohexanol
2-[4-(2-Chloro-phenyl)-piperazin-1-ylmethyl]-1-(2,5-dimethyl-benzyl)-cyclohexanol
1-(3-Chloro-benzyl)-2-[4-(2-chloro-phenyl)-piperazin-1-ylmethyl]-cyclohexanol
2-[4-(2-Chloro-phenyl)-piperazin-1-ylmethyl]-1-(2,4-dichloro-benzyl)-cyclohexanol
2-[4-(2-Methoxy-phenyl)-piperazin-1-ylmethyl]-1-phenyl-cyclohexanol
1-(4-Chloro-phenyl)-2-[4-(2-methoxy-phenyl)-piperazin-1-ylmethyl]-cyclohexanol
1-Benzyl-2-[4-(2-methoxy-phenyl)-piperazin-1-ylmethyl]-cyclohexanol
1-(4-Fluoro-3-methyl-phenyl)-2-[4-(2-methoxy-phenyl)-piperazin-1-ylmethyl]-cyclohexanol
2-[4-(2-Methoxy-phenyl)-piperazin-1-ylmethyl]-1-o-tolyl-cyclohexanol
2-[4-(2-Methoxy-phenyl)-piperazin-1-ylmethyl]-1-vinyl-cyclohexanol
1-(4-tert-Butyl-phenyl)-2-[4-(2-methoxy-phenyl)-piperazin-1-ylmethyl]-cyclohexanol
1-Cyclopentyl-2-[4-(2-methoxy-phenyl)-piperazin-1-ylmethyl]-cyclohexanol
2-[4-(2-Methoxy-phenyl)-piperazin-1-ylmethyl]-1-m-tolyl-cyclohexanol
2-[4-(2-Methoxy-phenyl)-piperazin-1-ylmethyl]-bicyclohexyl-1-ol
1-(4-Fluoro-phenyl)-2-[4-(2-methoxy-phenyl)-piperazin-1-ylmethyl]-cyclohexanol
2-[4-(2-Methoxy-phenyl)-piperazin-1-ylmethyl]-1-phenethyl-cyclohexanol
2-[4-(2-Methoxy-phenyl)-piperazin-1-ylmethyl]-1-phenylethynyl-cyclohexanol
2-[4-(2-Methoxy-phenyl)-piperazin-1-ylmethyl]-1-thiophen-2-yl-cyclohexanol
1-(3-Methoxy-phenyl)-2-[4-(2-methoxy-phenyl)-piperazin-1-ylmethyl]-cyclohexanol
2-[4-(2-Methoxy-phenyl)-piperazin-1-ylmethyl]-1-(3-phenyl-propyl)-cyclohexanol
1-(2,3-Dichloro-phenyl)-2-[4-(2-methoxy-phenyl)-piperazin-1-ylmethyl]-cyclohexanol
2-[4-(2-Methoxy-phenyl)-piperazin-1-ylmethyl]-1-p-tolyl-cyclohexanol
1-(4-Methoxy-phenyl)-2-[4-(2-methoxy-phenyl)-piperazin-1-ylmethyll-cyclohexanol
1-Cyclohexylmethyl-2-[4-(2-methoxy-phenyl)-piperazin-1-ylmethyl]-cyclohexanol
1-(5-Fluoro-2-methoxy-phenyl)-2-[4-(2-methoxy-phenyl)-piperazin-1-ylmethyl]-cyclohexanol
1-(3-Fluoro-phenyl)-2-[4-(2-methoxy-phenyl)-piperazin-1-ylmethyl]-cyclohexanol
1-(3-Chloro-phenyl)-2-[4-(2-methoxy-phenyl)-piperazin-1-ylmethyl]-cyclohexanol
1-(3,5-Dichloro-phenyl)-2-[4-(2-methoxy-phenyl)-piperazin-1-ylmethyl]-cyclohexanol
1-(2-Chloro-benzyl)-2-[4-(2-methoxy-phenyl)-piperazin-1-ylmethyl]-cyclohexanol
1-(4-Fluoro-benzyl)-2-[4-(2-methoxy-phenyl)-piperazin-1-ylmethyl]-cyclohexanol
1-(3-Methoxy-benzyl)-2-[4-(2-methoxy-phenyl)-piperazin-1-ylmethyl]-cyclohexanol
1-(4-Chloro-3-trifluoromethyl-phenyl)-2-[4-(2-methoxyphenyl)-piperazin-1-ylmethyl]-cyclohexanol
1-(3-Fluoro-benzyl)-2-[4-(2-methoxy-phenyl)-piperazin-1-ylmethyl]-cyclohexanol
1-(2-Methoxy-phenyl)-2-[4-(2-methoxy-phenyl)-piperazin-1-ylmethyl]-cyclohexanol
2-[4-(2-Methoxy-phenyl)-piperazin-1-ylmethyl]-1-(2-methyl-benzyl)-cyclohexanol
1-(3-Chloro-4-fluoro-phenyl)-2-[4-(2-methoxy-phenyl)-piperazin-1-ylmethyl]-cyclohexanol
2-[4-(2-Methoxy-phenyl)-piperazin-1-ylmethyl]-1-(3-trifluoromethyl-phenyl)-cyclohexanol
2-[4-(2-Methoxy-phenyl)-piperazin-1-ylmethyl]-1-(3-methyl-benzyl)-cyclohexanol
1-(9-Chloro-benzyl)-2-[4-(2-methoxy-phenyl)-piperazin-1-ylmethyl]-cyclohexanol
1-(2-Chloro-6-fluoro-benzyl)-2-[4-(2-methoxy-phenyl)-piperazin-1-ylmethyl]-cyclohexanol
1-(2,5-Dimethyl-benzyl)-2-[4-(2-methoxy-phenyl)-piperazin-1-ylmethyl]-cyclohexanol
1-(3-Chloro-benzyl)-2-[4-(2-methoxy-phenyl)-piperazin-1-ylmethyl]-cyclohexanol
1-(2,4-Dichloro-benzyl)-2-[4-(2-methoxy-phenyl)-piperazin-1-ylmethyl]-cyclohexanol
1-Benzyl-2-[4-(2-methoxy-phenyl)-piperazin-1-ylmethyl]-cyclododecanol
2-[4-(2-Methoxy-phenyl)-piperazin-1-ylmethyl]-1-vinyl-cyclododecanol
1-Phenethyl-2-[phenyl-(4-phenyl-piperazin-1-yl)-methyl]-cyclohexanol
1-Benzyl-2-[(4-methyl-piperazin-1-yl)-phenyl-methyl]-cyclohexanol
J.-Benzyl-2-[phenyl-(4-phenyl-piperazin-1-yl)-methyl]-cyclohexanol
2-[(4-Methyl-piperazin-1-yl)-phenyl-methyl]-1-phenethyl-cyclohexanol
2-[4-(2-Methoxy-phenyl)-piperazin-1-ylmethyl]-1-phenethyl-cyclododecanol
in the form of the diastereomer, the enantiomer and a mixture thereof - including the racemate - and in the form of the corresponding base, the corresponding salt and the corresponding solvate.

15. A process for the production of N,N'-disubstituted piperazine compounds according to any one of claims 1-14, **characterised in that**
A₁) a ketone of the formula (1), in which R¹ and R² have the meaning stated in claim 1, 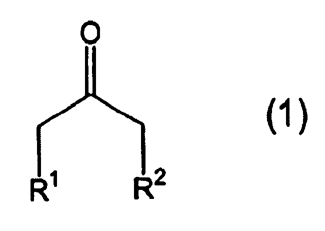 is reacted with paraformaldehyde and a piperazine of the formula (2), in which R⁵ has the meaning stated in claim 1, 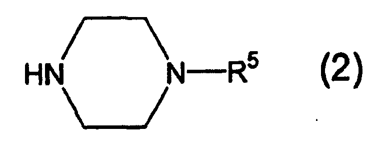 according to a Mannich reaction in a suitable solvent, preferably in ethanol with the addition of hydrochloric acid or in acetic acid, with heating, then the reaction mixture is worked up and the product of the formula (3) is isolated and optionally purified or 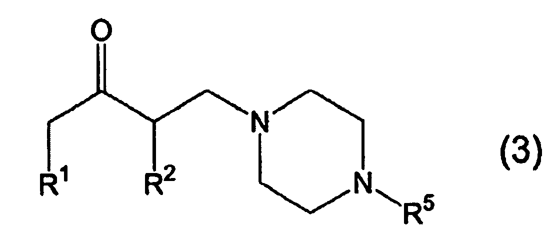
A₂) an enamine of the formula (1a), in which R¹ and R² have the meaning stated in claim 1 and R denotes an aliphatic C₁₋₆ residue, a morpholinyl, piperidyl or pyrrolidinyl residue, wherein the two residues R may be identical or different, 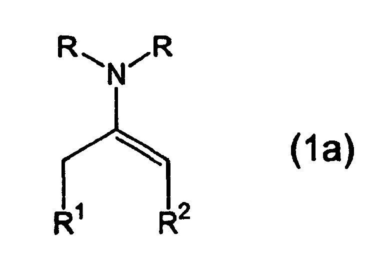 is reacted with an aldehyde of the formula (4), in which R⁴ has the meaning stated in claim 1 with the exception of hydrogen, 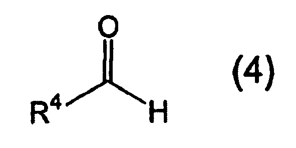 and a piperazine of the formula (2), in which R⁵ has the meaning stated in claim 1, optionally in the form of the hydrochloride thereof 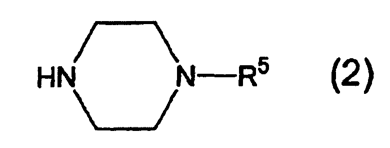 according to a Mannich reaction in the presence of triethylamine, chlorotrimethylsilane and sodium iodide in a suitable solvent, preferably in acetonitrile, then the reaction mixture is worked up and the product of the formula (3a) is isolated and optionally purified or 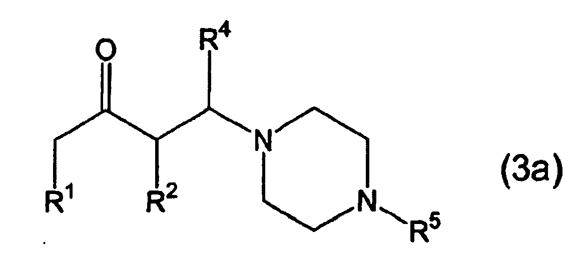
A₃) an enamine of the formula (1a), in which R¹ and R² have the meaning stated in claim 1 and R denotes an aliphatic C₁₋₆ residue, a morpholinyl, piperidyl or pyrrolidinyl residue, wherein the two residues R may be identical or different, 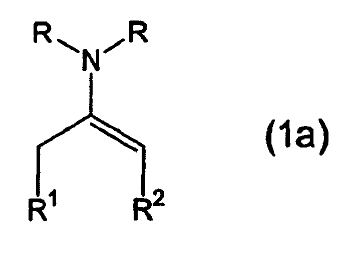 is reacted with an iminium salt of the formula (5), in which R⁴ has the meaning stated in claim 1 with the exception of hydrogen and R⁵ has the meaning stated in claim 1 and Y⁻ denotes a chloride, bromide, iodide or AlCl₄⁻ ion, 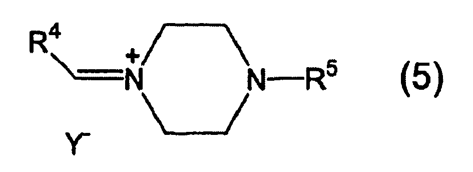 according to a Mannich reaction in a suitable solvent, preferably in acetonitrile, then the reaction mixture is worked up and the product of the formula (3a) is isolated and optionally purified and 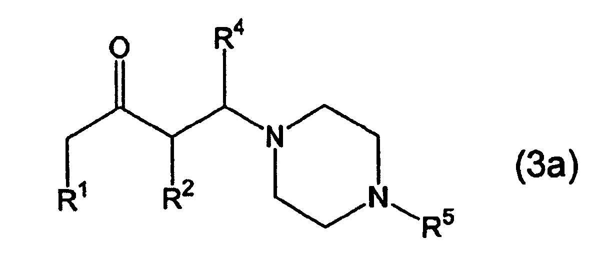
B) a compound of the formula (3) or (3a) is reacted with a Grignard compound or an organolithium compound of the formulae R³MgCl, R³MgBr, R³MgI, MgR³₂ or LiR³, in which R³ has the meaning stated in claim 1, in a suitable solvent, preferably diethyl ether or tetrahydrofuran, then the reaction mixture is worked up and the compound of the general formula I is isolated and optionally purified.

16. A pharmaceutical preparation containing at least one compound according to any one of claims 1-14 as active ingredient and optionally physiologically acceptable auxiliary substances.

17. A pharmaceutical preparation according to claim 16 for combatting pain.

18. A pharmaceutical preparation according to claim 16 for local anaesthesia.

19. A pharmaceutical preparation according to claim 16 as an antiarrhythmic.

20. A pharmaceutical preparation according to claim 16 as an antiemetic.

21. A pharmaceutical preparation according to claim 16 as a nootropic (neurotropic).

22. A pharmaceutical preparation according to claim 16 for the treatment of cardiovascular diseases.

23. A pharmaceutical preparation according to claim 16 for the treatment of urinary incontinence.

24. A pharmaceutical preparation according to claim 16 for the treatment of diarrhoea.

25. A pharmaceutical preparation according to claim 16 for the treatment of pruritus.

26. A pharmaceutical preparation according to claim 16 for the treatment of dependency on alcohol.

27. A pharmaceutical preparation according to claim 16 for the treatment of dependency on drugs.

28. A pharmaceutical preparation according to claim 16 for the treatment of dependency on medicines.

29. A pharmaceutical preparation according to claim 16 for the treatment of inflammation.

30. A pharmaceutical preparation according to claim 16 for the treatment of depression.

31. A pharmaceutical preparation according to claim 16 for increasing vigilance.

32. A pharmaceutical preparation according to claim 16 for increasing libido.

33. Use of at least one compound according to any one of claims 1-14 for the production of a pharmaceutical preparation for combatting pain.

34. Use of at least one compound according to any one of claims 1-14 for the production of a pharmaceutical preparation for the treatment of depression.

35. Use of at least one compound according to any one of claims 1-14 for the production of a pharmaceutical preparation for local anaesthesia and/or for the treatment of arrhythmias, emesis, cardiovascular diseases, urinary incontinence, diarrhoea, pruritus, inflammation, dependency on alcohol and/or drugs and/or medicines, to increase drive, vigilance and/or libido.

## Revendications

1. Composés N,N'-disubstitués de pipérazine de formule générale I[AP1], 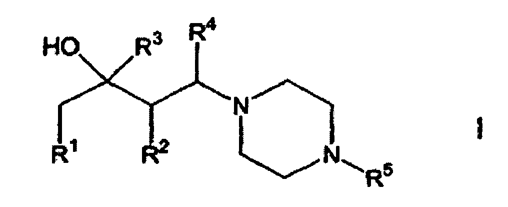
dans laquelle
R¹ et R², identiques ou différents, représentent chaque fois un radical aliphatique, saturé ou insaturé, linéaire ou ramifié ou constituent ensemble une chaîne (CH₂)ₙ, dans laquelle n représente un nombre entier,
R³ et R⁵, identiques ou différents, représentent chaque fois un radical aliphatique, saturé ou insaturé, linéaire ou ramifié, un radical cycloaliphatique saturé ou insaturé, un radical aryle ou hétéroaryle, dans lequel le système cyclique considéré peut éventuellement être substitué une fois ou plusieurs fois et/ou peut être lié via un pont aliphatique saturé ou insaturé, linéaire ou ramifié et/ou être la partie formant radical aryle ou hétéroaryle d'un système polycyclique,
R⁴ représente l'hydrogène ou un radical aryle ou hétéroaryle substitué une fois ou plusieurs fois, de manière identique ou différente, dans lequel le radical peut être la partie formant radical aryle ou hétéroaryle d'un système polycyclique,
sous la forme de leurs diastéréoisomères, de leurs énantiomères et de mélanges d'entre eux- y compris leurs racémiques- ainsi que sous la forme de bases, de sels et de sels solvatés correspondants.

2. Composés selon la revendication 1, **caractérisés en ce que**
R¹ et R², identiques ou différents, représentent chaque fois un radical aliphatique en C₁₋₆, saturé ou insaturé, linéaire ou ramifié ou constituent ensemble une chaîne (CH₂)ₙ, dans laquelle n vaut un nombre entier allant de 2 à 9,
R³ représente un radical aliphatique en C₁₋₆ saturé ou insaturé, linéaire ou ramifié, un radical cycloaliphatique en C₃₋₇ saturé ou insaturé, un radical phényle ou hétéroaryle à cinq ou six branches ou côtés, dans lequel le système cyclique considéré peut éventuellement être substitué une fois ou plusieurs fois et/ou peut être lié via un pont aliphatique en C₁₋₅ saturé ou insaturé, linéaire ou ramifié,
R⁴ représente l'hydrogène ou un radical phényle,
R₅ représente un radical aliphatique en C₁₋₆, saturé ou insaturé, linéaire ou ramifié ou un radical phényle éventuellement substitué.

3. Composés selon la revendication 1, **caractérisés en ce que**
R¹ et R², identiques ou différents, représentent chaque fois un radical aliphatique en C₁₋₃, saturé ou insaturé, linéaire ou ramifié ou constituent ensemble une chaîne (CH₂)ₙ, dans laquelle n vaut un nombre entier allant de 2 à 9,
R³ représente un radical aliphatique en C₁₋₃, saturé ou insaturé, linéaire ou ramifié, un radical cycloaliphatique en C₅₋₆ saturé ou insaturé, un radical phényle ou hétéroaryle à 5 ou 6 côtés, dans lequel le système cyclique considéré peut éventuellement être substitué une fois ou plusieurs fois avec un halogène, un groupe alkyle, un groupe alcoxy et/ou un groupe alkyle trihalogéné et/ou peut être lié via un pont aliphatique en C₁₋₃ saturé ou insaturé, linéaire ou ramifié,
R⁴ représente l'hydrogène ou un radical phényle,
R⁵ représente un radical aliphatique en C₁₋₃, saturé ou insaturé, linéaire ou ramifié ou un radical phényle substitué avec un halogène ou un groupe alcoxy.

4. Composés selon la revendication 1, **caractérisés en ce que**
R¹ et R² représentent chaque fois un groupe méthyle ou constituent ensemble une chaîne (CH₂)ₙ, dans laquelle n vaut 3, 4, 5 ou 9,
R³ représente un radical vinyle, un radical cyclopentyle, un radical cyclohexyle, un radical thiophényle ou un radical phényle, le radical cyclohexyle pouvant être éventuellement lié via un pont méthylène ou le radical phényle pouvant être éventuellement substitué une ou plusieurs fois avec le fluor, le chlore, un groupe méthyle, un groupe isopropyle, un groupe méthoxy et/ou un groupe trifluorométhyle et/ou être éventuellement lié via un pont aliphatique en C₁₋₃, saturé et linéaire ou un pont éthinyle,
R⁴ représente l'hydrogène ou un radical phényle,
R⁵ représente un groupe méthyle ou un radical phényle éventuellement substitué avec le chlore ou un groupe méthoxy.

5. Composés selon la revendication 1 correspondant à la formule générale II[AP2]

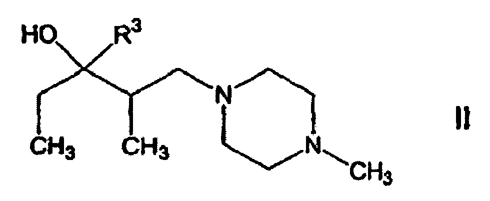

6. Composés selon la revendication 1 correspondant à la formule générale III[AP3] 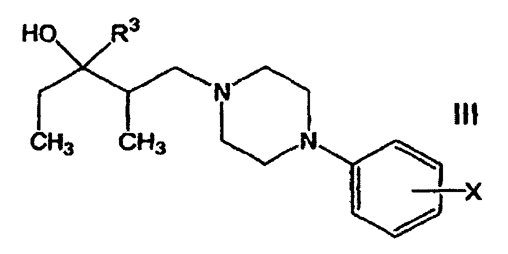
dans laquelle
X représente l'hydrogène, un halogène ou un groupe alcoxy, de préférence l'hydrogène, le chlore ou un groupe méthoxy.

7. Composés selon la revendication 1 correspondant à la formule générale IV[AP4], 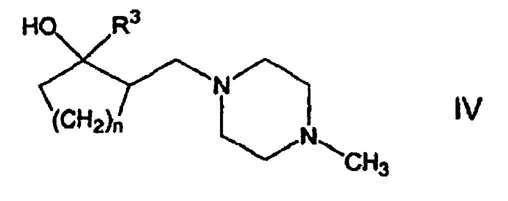
dans laquelle
n représente un nombre entier allant de 2 à 9.

8. Composés selon la revendication 1 correspondant à la formule générale V[AP5] 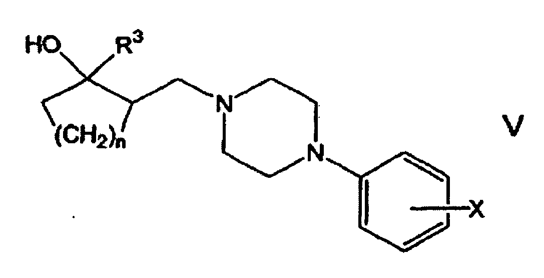
dans laquelle
n représente un nombre entier allant de 2 à 9 et
X représente l'hydrogène, un halogène ou un groupe alcoxy, de préférence l'hydrogène, le chlore ou un groupe méthoxy.

9. Composés selon la revendication 1 correspondant à la formule générale VI[AP6] 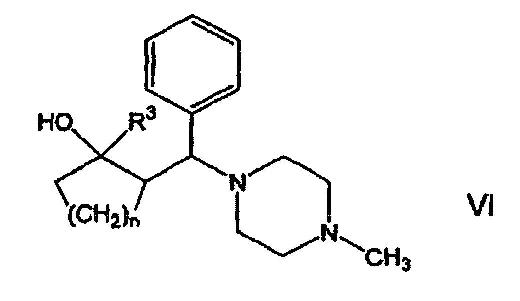
dans laquelle
n représente un nombre entier allant de 2 à 9.

10. Composés selon la revendication 1 correspondant à la formule générale VII[AP7], 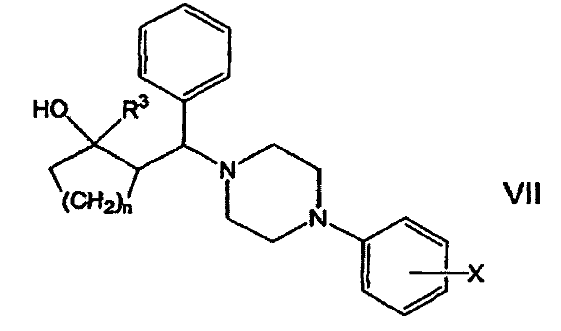
dans laquelle
n représente un nombre entier allant de 2 à 9 et
X représente l'hydrogène, un halogène ou un groupe alcoxy, de préférence l'hydrogène, le chlore ou un groupe méthoxy.

11. Composés selon l'une des revendications 5 - 10, **caractérisés en ce que**
R³ représente un radical aliphatique en C₁₋₆ saturé ou insaturé, linéaire ou ramifié, un radical cycloaliphatique en C₃₋₇ saturé ou insaturé, un radical phényle ou hétéroaryle à cinq ou six branches ou côtés, dans lequel le système cyclique considéré peut éventuellement être substitué une ou plusieurs fois et/ou peut être lié via un pont aliphatique en C₁₋₅ saturé ou insaturé, linéaire ou ramifié.

12. Composés selon l'une des revendications 5 - 10, **caractérisés en ce que**
R³ représente un radical aliphatique en C₁₋₃ saturé ou insaturé, linéaire ou ramifié, un radical cycloaliphatique en C₅₋₆ saturé ou insaturé, un radical phényle ou hétéroaryle à cinq ou six branches ou côtés, dans lequel le système cyclique considéré peut éventuellement être substitué une fois ou plusieurs fois avec un halogène, un groupe alkyle, un groupe alcoxy et/ou un groupe alkyle trihalogéné et/ou peut être lié via un pont aliphatique en C₁₋₃ saturé ou insaturé, linéaire ou ramifié.

13. Composés selon l'une des revendications 5 - 10, **caractérisé en ce que**
R3 représente un radical vinyle, un radical cyclopentyle, un radical cyclohexyle, un radical thiophényle ou un radical phényle, le radical cyclohexyle pouvant être éventuellement lié via un pont méthylène ou le radical phényle pouvant être éventuellement substitué une ou plusieurs fois avec le fluor, le chlore, un groupe méthyle, un groupe isopropyle, un groupe méthoxy et/ou un groupe trifluorométhyle et/ou être éventuellement lié via un pont aliphatique en C₁₋₃, saturé et linéaire ou un pont éthinyle.

14. Un composé N, N'- disubstitué de pipérazine selon la revendication 1, choisi dans le groupe comprenant :
2-méthyle-1-(4-méthyle-pipérazine-1-yle)-3-phényle-pentane-3-ol
3-(4-chloro-phényle)-2-méthyle-1-(4-méthyle-pipérazine-1-yle)-pentane-3-ol
3-benzyle-2-méthyle-1-(4-méthyle-pipérazine-1-yle)-pentane-3-ol
3-(4-fluoro-3-méthyle-phényle)-2-méthyle-1-(4-méthyle-pipérazine-1-yle)- pentane-3-ol
2-méthyle-1-(4-méthylepipérazine-1-yle)-3-o-toluyle-pentane-3-ol
3-éthyle-4-méthyle-5-(4-méthyle-pipérazine-1-yle)-pent-1-ène-3-ol
3-(4-tertiobutyle-phényle)-2-méthyle-1-(4-méthyle-pipérazine-1-yle)-pentane-3-ol
3-cyclopentyle-2-méthyle-1-(4-méthyle-pipérazine-1-yle)-pentane-3-ol
2-méthyle-1-(4-méthyle-pipérazine-1-yle)-3-m-toluyle-pentane-3-ol
3-cyclohexyle-2-méthyle-1-(4-méthyle-pipérazine-1-yle)- pentane-3-ol
3-(9-fluorophényle)-2-méthyle-1-(4-méthyle-pipérazine-1-yle)- pentane-3-ol
3-éthyle-2-méthyle-1-(4-méthyle-pipérazine-1-yle)-5-phényle-pentane-3-ole
3-éthyle-4-méthyle-5-(4-méthyle-pipérazine-1-yle)-5-phényle-pent-1-yne-3-ole
2-méthyle-1-(4-méthyle-pipérazine-1-yle)-3-thiophène-2-yle-pentane-3-ole
3-(3-méthoxy-phényle)-2-méthyle-1-(4-méthyle-pipérazine-1-yle)-5-phényle-pentane-3-ole
3-éthyle-2-méthyle-1-(4-méthyle-pipérazine-1-yle)-6-phényle-hexane-3-ole
2-méthyle-1-(4-méthyle-pipérazine-1-yle)-3-p-toluyle-pentane-3-ole
3-(4-méthoxy-phényle)-2-méthyle-1-(4-méthyle-pipérazine-1-yle)-pentane-3-ole
2-(4-méthyle-pipérazine-1-yleméthyle)-1-phényle-cyclohexanol
1-benzyle-2-(4-méthyle-pipérazine-1-yleméthyle)-cyclohexanol
1-(4-fluoro-3-méthyle-phényle)-2-(4-méthyle-pipérazine-1-yleméthyle)-cyclohexanol
2-(4-méthyle-pipérazine-1-yleméthyle)-1-o-toluyle-cyclohexanol
2-(4-méthyle-pipérazine-1-yleméthyle)-1-vinyle-cyclohexanol
1-cyclopentyle-2-( 4-méthyle-pipérazine-1-yleméthyle) -cyclohexanol
2-(4-méthyle-pipérazine-1-yleméthyle)-1-m-toluyle-cyclohexanol
2-(4-méthyle-pipérazine-1-yleméthyle) -bicyclohexyle-1-ol
1-(4-fluoro-phényle)-2-(4-méthyle-pipérazine-1-yleméthyle)-cyclohexanol
2-(4-méthyle-pipérazine-1-yleméthyle)-1-phénétyle-cyclohexanol
2-(4-méthyle-pipérazine-1-yleméthyle)-1-thiophéne-2-yle-cyclohexanol
1-(2,4-dichloro-phényle)-2-(4-méthyle-pipérazine-1-yleméthyle)- cyclohexanol
1-(3-méthoxy-phényle)-2-(4-méthyle-pipérazine-1-yleméthyle)- cyclohexanol
2-(4-méthyle-pipérazine-1-yleméthyle)-1-(3-phényle-propyle)-cyclohexanol
2-(4-méthyle-pipérazine-1-yleméthyle)-1-p-toluyle-cyclohexanol
1-(4-méthoxy-phényle)-2-(4-méthyle-pipérazine-1-yleméthyle)-cyclohexanol
1-(benzyle-2-(4-méthyle-pipérazine-1-yleméthyle)-cyclooctanol
2-(4-méthyle-pipérazine-1-yleméthyle)-1-vinyle-cyclooctanol
1-(4-tertiobutyle-phényle)-2-(4-méthyle-pipérazine-1-yleméthyle)-cyclooctanol
1-cyclopentyle-2-(4-méthyle-pipérazine-1-yleméthyle)-cyclooctanol
2-(4-méthyle-pipérazine-1-yleméthyle)-1-phénétyle-cyclooctanol
2-(4-méthyle-pipérazine-1-yleméthyle)-1-phényléthynyle-cyclooctanol
2-(4-méthyle-pipérazine-1-yleméthyle)-1-thiophène-2-yle-cyclooctanol
1-(3-méthoxy-phényle)-2-(4-méthyle-pipérazine-1-yleméthyle)-cyclooctanol
2-(4-méthyle-pipérazine-1-yleméthyle)-1-(3-phényle-propyle)-cyclooctanol
2-(4-méthyle-pipérazine-1-yleméthyle)-1-p-toluyle-cyclooctanol
1-(4-méthoxy-phényle)-2-(4-méthyle-pipérazine-1-yleméthyle)-cyclooctanol
2-(4-méthyle-pipérazine-1-yleméthyle)-1-phényle-cycloheptanol
1-(4-chloro-phényle)-2--(4-méthyle-pipérazine-1-yleméthyle)-cycloheptanol
1-(benzyle-2-(4-méthyle-pipérazine-1-yleméthyle)-cycloheptanol
1-(4-fluoro-3-méthyle-phényle)-2-(4-méthyle-pipérazine-1-yleméthyle)- cycloheptanol
2-(4-méthyle-pipérazine-1-yleméthyle)-1-o-toluyle-cycloheptanol
2-(4-méthyle-pipérazine-1-yleméthyle)-1-vinyle-cycloheptanol
1-(4-tertiobutyle-phényle)-2--(4-méthyle-pipérazine-1-yleméthyle) cycloheptanol
1-cyclopentyle-2-(4-méthyle-pipérazine-1-yleméthyle)-cycloheptanol
2-(4-méthyle-pipérazine-1-yleméthyle)-1-m-toluyle-cycloheptanol
1-[4-(3-chlorophényle)-pipérazine-1-yle]-2-méthyle-3-phényle-pentane-3-ol
3-(4-chlorophényle)-1-[4-(3-chlorophényle)-pipérazine-1-yle]-2-méthyle-pentane-3-ol
3-benzyle-1-[4-(3-chlorophényle)-pipérazine-1-yle]-2-méthyle-pentane-3-ol
1-[4-(3-chlorophényle)-pipérazine-1-yle]-3-(4-fluoro-3-méthyle-phényle)- 2-méthyle-pentane-3-ol
5-[4-(3-chlorophényle)-pipérazine-1-yle]-3-éthyle-4-méthyle-pent-1-ène-3-ol
3-(4-tertiobutyle-phényle)-1-[4-(3-chlorophényle)-pipérazine-1-yle]-2-méthyle-pentane-3-ol
1-[4-(3-chlorophényle)-pipérazine-1-yle]-2-méthyle-3-m-toluyle-pentane-3-ol
1-[4-(3-chlorophényle)-pipérazine-1-yle]-3-cyclohexyle-2-méthyle-pentane-3-ol
1-[4-(3-chlorophényle)-pipérazine-1-yle]-3-(4-fluorophényle)-2-méthyle-pentane-3-ol
1-[4-(3-chlorophényle)-pipérazine-1-yle]-3-éthyle-2-méthyle-5-phényle-pentane-3-ol
5-[4-(3-chlorophényle)-pipérazine-1-yle]-3-éthyle-4-méthyle-1-phényle-pent-1-yne-3-ol
1-[4-(3-chlorophényle)-pipérazine-1-yle]-2-méthyle-3-thiophène-2-yle-pentane-3-ol
1-[4-(3-chlorophényle)-pipérazine-1-yle]-3-(3-méthoxy-phényle)-2-méthyle-pentane-3-ol
1-[4-(3-chlorophényle)-pipérazine-1-yle]-3-éthyle-2-méthyle-6-phényle-hexane-3-ol
1-[4-(3-chlorophényle)-pipérazine-1-yle]-2-méthyle-3-p-toluyle-pentane-3-ol
1-[4-(3-chlorophényle)-pipérazine-1-yle]-3-(4-méthoxy-phényle)-2-méthyle-pentane-3-ol
1-[4-(3-chlorophényle)-pipérazine-1-yle]-3-(5-fluoro-2-méthoxy-phényle)-2-méthyle-pentane-3-ol
1-[4-(3-chlorophényle)-pipérazine-1-yle]-3-(3-fluorophényle)-2-méthyle-pentane-3-ol
3-(3-chlorophényle)-1-[4-(3-chlorophényle)-pipérazine-1-yle]-2-méthyle-pentane-3-ol
3-(2-chlorobenzyle)-1-[4-(3-chlorophényle)-pipérazine-1-yle]-2-méthyle-pentane-3-ol
1-[4-(3-chlorophényle)-pipérazine-1-yle]-3-(4-fluorobenzyle)-2-méthylé-pentane-3-ol
1-[4-(3-chlorophényle)-pipérazine-1-yle]-3-(3-méthoxy-benzyle)-2-méthyle-pentane-3-ol
1-[4-(3-chlorophényle)-pipérazine-1-yle]-3-(3-fluorobenzyle)-2-méthyle-pentane-3-ol
1-[4-(3-chlorophényle)-pipérazine-1-yle]-3-(2-méthoxy-phényle)-2-méthyle- pentane-3-ol
1-[4-(3-chlorophényle)-pipérazine-1-yle]-2-méthyle-3-(2-méthyle-benzyle)- pentane-3-ol
1-[4-(3-chlorophényle)-pipérazine-1-yle]-2méthyle-3-(trifluorométhyle-phényle)- pentane-3-ol
1-[4-(3-chlorophényle)-pipérazine-1-yle]-2-méthyle-3-(3-méthyle-benzyle)- pentane-3-ol
3-(4-chlorobenzyle)-1-[4-(3-chlorophényle)-pipérazine-1-yle]-2méthyle-pentane-3-ol
3-(2-chloro-6-fluorobenzyle)-1-[4-(3-chlorophényle)-pipérazine-1-yle]- 2méthyle- pentane-3-ol
1-[4-(3-chlorophényle)-pipérazine-1-yle]-3-(2,5-diméthylbenzyle)-2méthyle- pentane-3-ol
1-[4-(3-chlorophényle)-pipérazine-1-yle]-3-(2,4-dichlorobenzyle)-2-méthyle- pentane-3-ol
3-cyclohexylméthyle-2-méthyle-1-(4-méthyle-pipérazine-1-yle)-pentane-3-ole
3-(5-fluoro-2-méthoxy-phényle)-2-méthyle-1-(4-méthyle-pipérazine-1-yle) -pentane-3-ole
3-(3-fluoro-phényle)-2-méthyle-1-(4-méthyle-pipérazine-1-yle)-pentane-3-ole
3-(3-chloro-phényle)-2-méthyle-1-(4-méthyle-pipérazine-1-yle)-pentane-3-ole
3-(3,5-dichloro-phényle)-2-méthyle-1-(4-méthyle-pipérazine-1-yle)-pentane-3-ole
3-(2-chloro-benzyle)-2-méthyle-1-(4-méthyle-pipérazine-1-yle)-pentane-3-ole
3-(4-fluoro-benzyle)-2-méthyle-1-(4-méthyle-pipérazine-1-yle)-pentane-3-ole
3-(3-méthoxy-benzyle)-2-méthyle-1-(4-méthyle-pipérazine-1-yle)-pentane-3-ole
3-(4-chloro-3trifluorométhyle-phényle)-2-méthyle-1-(4-méthyle-pipérazine-1-yle) -pentane-3-ole
3-(3-fluoro-benzyle)-2-méthyle-1-(4-méthyle-pipérazine-1-yle)-pentane-3-ole
3-(2-méthoxy-phényle)-2-méthyle-1-(4-méthyle-pipérazine-1-yle)-pentane-3-ole
2-méthyle-3-(2-méthyle-benzyle)-1-(4-méthyle-pipérazine-1-yle)-pentane-3-ole
3-(3-chloro-4-fluoro-phényle)-2-méthyle-1-(4-méthyle-pipérazine-1-yle)-pentane-3-ole
2-méthyle-1-(4-méthyle-pipérazine-1-yle)-3-(3-trifluorométhyle-phényle)- -pentane-3-ole
2-méthyle-3(3-méthyle-benzyle)-1-(4-méthyle-pipérazine-1-yle)-pentane-3-ole
3-(4-chlorobenzyle)-2-méthyle-1-(4-méthyle-pipérazine-1-yle)-pentane-3-ole
3-(2-chloro-6-fluorobenzyle)-2-méthylel-(4-méthyle-pipérazine-1-yle)-pentane-3-ole
3-(2,5-diméthyle-benzyle)-2-méthylel-(4-méthyle-pipérazine-1-yle)-pentane-3-ole
3-(3-chloro-benzyle)-2-méthylel-(4-méthyle-pipérazine-1-yle)-pentane-3-ole
3-(2,4-dichloro-benzyle)-2-méthylel-(4-méthyle-pipérazine-1-yle)-pentane-3-ole
1-cyclohexylméthyle-2-(4-méthyle-pipérazine-1-yleméthyle)- cyclohexanol
1-(5-fluoro-2-méthoxy-phényle)-2-(4-méthyle-pipérazine-1-yleméthyle)-cyclohexanol
1-(3-fluoro-phényle)-2-(4-méthyle-pipérazine-1-yleméthyle)-cyclohexanol
1-(3-chlorophényle)-2-(4-méthyle-pipérazine-1-yleméthyle)- cyclohexanol
1-(3,5-dichloro-phényle)-2-4-méthyle-pipérazine-1-yleméthyle)-cyclohexanol
1-(2-chloro-benzyle)-2-(4-méthyle-pipérazine-1-yleméthyle)- cyclohexanol
1-(4-fluoro-benzyle)-2-(4-méthyle-pipérazine-1-yleméthyle)-cyclohexanol
1-(3-méthoxy-benzyle)-2-(4-méthyle-pipérazine-1-yleméthyle)- cyclohexanol
1-(4-chloro-3-trifluorométhyle-phényle)-2-(4-méthyle-pipérazine-1-yleméthyle)-cyclohexanol
1-(3-fluoro-benzyle)-2-(4-méthyle-pipérazine-1-yleméthyle)-cyclohexanol
1-(2-méthoxy-phényle)-2-(4-méthyle-pipérazine-1-yleméthyle)-cyclohexanol
1-2(méthyl-benzyle)-2(4-méthyle-pipérazine-1-yleméthyle)-cyclohexanol
1-(3-chloro-4-fluoro-phényle)-2-(4-méthyle-pipérazine-1-yleméthyle)-cyclohexanol
2-(4-méthyle-pipérazine-1-yleméthyle)-1-3-(3-trifluorométhyle-phényle)-cyclohexanol
1-(3-méthyl-benzyle)-2-(4-méthyle-pipérazine-1-yleméthyle)-cyclohexanol
1-(4-chloro-benzyle)-2-(4-méthyle-pipérazine-1-yleméthyle)-cyclohexanol
1-(2,5-diméthyl-benzyle)-2-(4-méthyle-pipérazine-1-yleméthyle)-cyclohexanol
1-(3-chloro-benzyle)-2-(4-méthyle-pipérazine-1-yleméthyle)-cyclohexanol
1-(2,4-dichloro-benzyle)-2-(4-méthyle-pipérazine-1-yleméthyle)-cyclohexanol
1-cyclohexylméthyl-2-(4-méthyle-pipérazine-1-yleméthyle)-cyclooctanol
1-(5-fluoro-2-méthoxy-phényle)-2-(4-méthyle-pipérazine-1-yleméthyle)- cyclooctanol
1-(2-chloro-benzyle)-2-(4-méthyle-pipérazine-1-yleméthyle)-cyclooctanol
1-(4-fluoro-benzyle)-2-(4-méthyle-pipérazine-1-yleméthyle)-cyclooctanol
1-(3-fluoro-benzyle)-2-(4-méthyle-pipérazine-1-yleméthyle)-cyclooctanol
1-(2-méthoxy-phényle)-2-(4-méthyle-pipérazine-1-yleméthyle)-cyclooctanol
1-(3-méthyl-benzyle)-2-(4-méthyle-pipérazine-1-yleméthyle)-cyclooctanol
1-(4-chloro-benzyle)-2-(4-méthyle-pipérazine-1-yleméthyle)- cyclooctanol
1-(2-chloro-6-fluoro-benzyle)-2-(4-méthyle-pipérazine-1-yleméthyle)- cyclooctanol
1-(2,5-diméthyl-benzyle)-2-(4-méthyle-pipérazine-1-yleméthyle)- cyclooctanol
1-(3-chloro-benzyle)-2-(4-méthyle-pipérazine-1-yleméthyle)- cyclooctanol
1-(2,4-dichloro-benzyle)-2-(4-méthyle-pipérazine-1-yleméthyle)- cyclooctanol
1-cyclohexylméthyl-2-(4-méthyle-pipérazine-1-yleméthyle)-cyclooctanol
1-(5-fluoro-2-méthoxy-phényle)-2-(4-méthyle-pipérazine-1-yleméthyle)- cyclooctanol
1-(3-fluoro-phényle)-2-(4-méthyle-pipérazine-1-yleméthyle)- cyclooctanol
1-(3-chloro-phényle)-2-(4-méthyle-pipérazine-1-yleméthyle)- cyclooctanol
1-(3,5-dichloro-phényle)-2-(4-méthyle-pipérazine-1-yleméthyle)- cyclooctanol
1-cyclohexylméthyl-2-(4-méthyle-pipérazine-1-yleméthyle)- cycloheptanol
1-(5-fluoro-2-méthoxy-phényle)-2-(4-méthyle-pipérazine-1-yleméthyle)- cycloheptanol
1-(3-fluoro-phényle)- 2-(4-méthyle-pipérazine-1-yleméthyle)-cycloheptanol
1-(3-chloro-phényle)-2-(4-méthyle-pipérazine-1-yleméthyle)- cycloheptanol
1-(3,5-dichlorophényle)-2-(4-méthyle-pipérazine-1-yleméthyle)-cycloheptanol
1-benzyle-2-(4-méthyle-pipérazine-1-yleméthyle)-cyclododécanol
2-(4-méthyle-pipérazine-1-yleméthyle)-1-phényle-cyclododécanol
1-cyclopentyle-2-(4-méthyle-pipérazine-1-yleméthyle)-cyclododécanol
2-(4-méthyle-pipérazine-1-yleméthyle)-1-phénétyle-cyclododécanol
2-(4-méthyle-pipérazine-1-yleméthyle)-1-phényléthynyle-cyclododécanol
2-(4-méthyle-pipérazine-1-yleméthyle)-1-thiophène-2-yle-cyclododécanol
2-(4-méthyle-pipérazine-1-yleméthyle)-1-(3-phénylpropyle)-cyclododécanol
1-(5-fluoro-2-méthoxy-phényle)-2-(4-méthyle-pipérazine-1-yleméthyle)-cyclododécanol
1-(2-chloro-benzyle)-2-(4-méthyle-pipérazine-1-yleméthyle)- cyclododécanol
1-(4-fluoro-benzyle)-2-(4-méthyle-pipérazine-1-yleméthyle)- cyclododécanol
1-(3-méthoxy-benzyle)-2-(4-méthyle-pipérazine-1-yleméthyle)- cyclododécanol
1-(3-fluorobenzyle)-2-(4-méthyle-pipérazine-1-yleméthyle)-cyclododécanol
1-(2-méthoxy-phényle)-2-(4-méthyle-pipérazine-1-yleméthyle)-cyclododécanol
1-(2-méthylbenzyle)-2-(4-méthyle-pipérazine-1-yleméthyle)- cyclododécanol
1-(3-méthylbenzyle)-2-(4-méthyle-pipérazine-1-yleméthyle)- cyclododécanol
1-(4-chloro-benzyle)-2-(4-méthyle-pipérazine-1-yleméthyle)- cyclododécanol
1-(2,5-diméthylbenzyle)-2-(4-méthyle-pipérazine-1-yleméthyle)- cyclododécanol
1-(3-chloro-benzyle)-2-(4-méthyle-pipérazine-1-yleméthyle)- cyclododécanol
1-(2,4-dichloro-benzyle)-2-(4-méthyle-pipérazine-1-yleméthyle)- cyclododécanol
2-[4-3-(chlorophényle)-pipérazine-1-yleméthyle)-1-cyclohexylméthyl-cyclohexanol
2-[4-3-(chlorophényle)-pipérazine-1-yleméthyle)-5-fluoro-2-méthoxy-cyclohexanol
2-[4-3-(chlorophényle)-pipérazine-1-yleméthyle)-3-fluoro-phényle- cyclohexanol
1-(3-(chlorophényle)-2-[4-(3-chloro-phényle)-pipérazine-1-yleméthyle] -cyclohexanol
2-[4-3-(chlorophényle)-pipérazine-1-yleméthyle)-1-(3,5-dichloro-phényle)-cyclohexanol
1-(2-chloro-benzyle)-2-[4-(3-chloro-phényle)-pipérazine-1-yleméthyle] -cyclohexanol
2-[4-3-(chlorophényle-pipérazine-1-yleméthyle)]-1-(4-fluorobenzyle)- cyclohexanol
2-[4-3-(chlorophényle-pipérazine-1-yleméthyle)]-1-(4-chloro-3-trifluorométhyle-phényle)-cyclohexanol
2-[4-3-(chlorophényle-pipérazine-1-yleméthyle)]-1-(3-fluorobenzyle)- cyclohexanol
2-[4-(3-chloro-phényle)-pipérazine-1-yleméthyle]-1-(2-méthoxy-phényle)-cyclohexanol
1-(3-chloro-4-fluoro-phényle)-2-[4-(3-chloro-phényle)-pipérazine-1-yleméthyle] -cyclohexanol
2-[4-3-(chlorophényle-pipérazine-1-yleméthyle)-1-(3-trifluorométhylphényle)- cyclohexanol
2-[4-3-(chlorophényle-pipérazine-1-yleméthyle)-1-(3-méthylbenzyle)- cyclohexanol
1-(4-chloro-benzyle)-2-[4-(3-chloro-phényle)-pipérazine-1-yleméthyle] -cyclohexanol
1-(2-chloro-6-fluoro-benzyle)-2-[4-(3-chloro-phényle)-pipérazine-1-yleméthyle] -cyclohexanol
2-[4-(3-chloro-phényle)-pipérazine-1-yleméthyle]-1-(2,5-diméthylbenzyle)-cyclohexanol
1-(3-(chlorobenzyle)-2-[4-(3-chloro-phényle)-pipérazine-1-yleméthyle] -cyclohexanol
2-[4-2-(chlorophényle-pipérazine-1-yleméthylel-1-phényle-cyclohexanol
1-(4-chloro-phényle)-2-[4-2-(chlorophényle-pipérazine-1-yleméthyle]- cyclohexanol
1-benzyle-2-[4-2-(chlorophényle-pipérazine-1-yleméthyle]- cyclohexanol
2-[4-2-(chlorophényle-pipérazine-1-yleméthyle]-1-(4-fluoro-3-méthyle-phényle)-cyclohexanol
2-[4-2-(chlorophényle-pipérazine-1-yleméthyle]-1-o-toluyle-cyclohexanol
2-[4-2-(chlorophényle-pipérazine-1-yleméthyle]-1-vinyle- cyclohexanol
1-(4-tertiobutyle-phényle)-2-[4-2-(chlorophényle-pipérazine-1-yleméthyle]- cyclohexanol
2-[4-2-(chlorophényle-pipérazine-1-yleméthyle]-1-cyclopentyle- cyclohexanol
2-[4-2-(chlorophényle-pipérazine-1-yleméthyle]-1-m-toluyle- cyclohexanol
2-[4-2-(chlorophényle-pipérazine-1-yleméthyle]-bicyclohexyle-1-ol
2-[4-2-(chlorophényle-pipérazine-1-yleméthyle]-1-(4-fluorophényle)- cyclohexanol
2-[4-2-(chlorophényle-pipérazine-1-yleméthyle]-1-phénéthyle- cyclohexanol
2-[4-2-(chlorophényle-pipérazine-1-yleméthyle]-1-phényléthynyle- cyclohexanol[P8]
2-[4-2-(chlorophényle-pipérazine-1-yleméthyle]-1-thiophène-2-yle- cyclohexanol
2-[4-2-(chlorophényle-pipérazine-1-yleméthyle]-1-(3-méthoxy-phényle)- cyclohexanol
2-[4-2-(chlorophényle-pipérazine-1-yleméthyle]-1-(3-phényle-propyle)- cyclohexanol
2-[4-2-(chlorophényle-pipérazine-1-yleméthyle]-1-p-toluyle- cyclohexanol
2-[4-2-(chlorophényle-pipérazine-1-yleméthyle]-1-(4-méthoxy-phényle)- cyclohexanol
2-[4-2-(chlorophényle-pipérazine-1-yleméthyle]-1-cyclohexyleméthyle- cyclohexanol
2-[4-2-(chlorophényle-pipérazine-1-yleméthyle]-1-(5fluoro-2-méthoxy-phényle)- cyclohexanol
1-(3-chloro-phényle-2-[4-2-(chlorophényle-pipérazine-1-yleméthyle]- cyclohexanol
2-[4-2-(chlorophényle-pipérazine-1-yleméthyle]-1-(3,5-dichloro-phényle)- cyclohexanol
1-(2-chlorobenzyle)-2-[4-2-(chlorophényle-pipérazine-1-yleméthyle]- cyclohexanol
2-[4-2-(chlorophényle-pipérazine-1-yleméthyle]-1-(4-fluoro-benzyle)- cyclohexanol
2-[4-2-(chlorophényle-pipérazine-1-yleméthyle]-1-(3-méthoxy-benzyle)- cyclohexanol
2-[4-2-(chlorophényle-pipérazine-1-yleméthyle]-1-(4-chloro-3-trifluorométhyl-phényle)- cyclohexanol
2-[4-2-(chlorophényle-pipérazine-1-yleméthyle]-1-(3-fluoro-benzyle)- cyclohexanol
2-[4-2-(chlorophényle-pipérazine-1-yleméthyle]-1-(2-méthoxy-phényle)- cyclohexanol
2-[4-2-(chlorophényle-pipérazine-1-yleméthyle]-1-(2-méthyle-benzyle)- cyclohexanol
1-(3-chloro-4-fluorophényle)-2-[4-2-(chlorophényle-pipérazine-1-yleméthyle]- cyclohexanol
2-[4-2-(chlorophényle-pipérazine-1-yleméthyle]-1-(3-trifluorométhyle-phényle)- cyclohexanol
2-[4-2-(chlorophényle-pipérazine-1-yleméthyle]-1-(3-méthyl-benzyle)- cyclohexanol
1-(4-chlorobenzyle)-2-[4-2-(chlorophényle-pipérazine-1-yleméthyle]- cyclohexanol
1-(2-chloro-6-fluorobenzyle)-2-[4-2-(chlorophényle-pipérazine-1-yleméthyle]- cyclohexanol
2-[4-2-(chlorophényle-pipérazine-1-yleméthyle]-1-(2,5-diméthyl-benzyle)- cyclohexanol
1-(3-chloro-benzyle)-2-[4-2-(chlorophényle-pipérazine-1-yleméthyle]- cyclohexanol
2-[4-2-(chlorophényle-pipérazine-1-yleméthyle]-1-(2,4-dichloro-benzyle)-cyclohexanol
2-[4-2-(méthoxyphényle-pipérazine-1-yleméthyle]-1-phényle- cyclohexanol
1-(4-chlorophényle)-2-[4-2-(méthoxyphényle-pipérazine-1-yleméthylel- cyclohexanol
1-benzyle-2-[4-2-(méthoxyphényle-pipérazine-1-yleméthyle]- cyclohexanol
1-(4-fluoro-3-méthylphényle)-2-[4-2-(méthoxyphényle-pipérazine-1-yleméthyle]- cyclohexanol
2-[4-2-(méthoxyphényle-pipérazine-1-yleméthyle]-1-o-toluyle- cyclohexanol
2-[4-2-(méthoxyphényle-pipérazine-1-yleméthyle]-1-vinyle- cyclohexanol[P9]
1-(4-tertiobutyle-phényle)-2-[4-2-(méthoxyphényle-pipérazine-1-yleméthyle]- cyclohexanol
1-cyclopentyle-2-[4-2-(méthoxyphényle-pipérazine-1-yleméthyle]- cyclohexanol
2-[4-2-(méthoxyphényle-pipérazine-1-yleméthyle]-1-m-toluylecyclohexanol
2-[4-2-(méthoxyphényle-pipérazine-1-yleméthyle]-bicyclohexyle-cyclohexanol
1-(4-fluoro-phényle)-2-[4-2-(méthoxyphényle-pipérazine-1-yleméthyle]- cyclohexanol
2-[4-2-(méthoxyphényle-pipérazine-1-yleméthyle]-1-phénétyle-cyclohexanol
2-[4-2-(méthoxyphényle-pipérazine-1-yleméthyle]-1-phényléthynyle-cyclohexanol
2-[4-2-(méthoxyphényle-pipérazine-1-yleméthyle]-1-thiophène-2-yle-cyclohexanol
1-(3-méthoxy-phényle)-2-[4-2-(méthoxyphényle-pipérazine-1-yleméthyle]- cyclohexanol
2-[4-2-(méthoxyphényle-pipérazine-1-yleméthyle]-1-(3-phényle-propyle)-cyclohexanol
1-(2,3-dichloro-phényle)-2-[4-2-(méthoxyphényle-pipérazine-1-yleméthyle]- cyclohexanol
1-(4-méthoxy-phényle)-2-[4-2-(méthoxyphényle-pipérazine-1-yleméthyle]- cyclohexanol
1-cyclohexylméthyle)-2-[4-2-(méthoxyphényle-pipérazine-1-yleméthyle]- cyclohexanol
1-(5-fluoro-2-méthoxy-phényle)-2-[4-2-(méthoxyphényle-pipérazine-1-yleméthyle]- cyclohexanol
1-(3-fluoro-phényle)-2-[4-2-(méthoxyphényle-pipérazine-1-yleméthyle]- cyclohexanol
1-(3-chloro-phényle)-2-[4-2-(méthoxyphényle-pipérazine-1-yleméthyle]- cyclohexanol
1-(3,5-dichloro-phényle)-2-[4-2-(méthoxyphényle-pipérazine-1-yleméthyle]- cyclohexanol
1-(2-chlorobenzyle)-2-[4-2-(méthoxyphényle-pipérazine-1-yleméthyle]- cyclohexanol
1-(4-fluoro-benzyle)-2-[4-2-(méthoxyphényle-pipérazine-1-yleméthyle]- cyclohexanol
1-(3-méthoxy-benzyle)-2-[4-2-(méthoxyphényle-pipérazine-1-yleméthyle]- cyclohexanol
1-(4-chloro-3-trifluorométhyle-phényle)-2-[4-2-(méthoxyphényle-pipérazine-1-yleméthyle]- cyclohexanol
1-(3-fluoro-phényle)-2-[4-2-(méthoxyphényle-pipérazine-1-yleméthyle]- cyclohexanol
1-(2-méthoxy-phényle)-2-[4-2-(méthoxyphényle-pipérazine-1-yleméthyle]- cyclohexanol
2-[4-2-(méthoxyphényle-pipérazine-1-yleméthyle]-1-(2-méthylbenzyle)-cyclohexanol
1-(3-chloro-4-fluoro-phényle)-2-[4-2-(méthoxyphényle-pipérazine-1-yleméthyle]- cyclohexanol
2-[4-2-(méthoxyphényle-pipérazine-1-yleméthyle]-1-(3-trifluorométhyl-phényle)-cyclohexanol
2-[4-2-(méthoxyphényle-pipérazine-1-yleméthyle]-1-(3-méthylbenzyle)-cyclohexanol
1-(4-chlorobenzyle)-2-[4-2-(méthoxyphényle-pipérazine-1-yleméthyle]-cyclohexanol
1-(2-chloro-6-fluorobenzyle)-2-[4-2-(méthoxyphényle-pipérazine-1-yleméthyle]- cyclohexanol
1-(2,5-diméthylbenzyle)-2-[4-2-(méthoxyphényle-pipérazine-1-yleméthyle]- cyclohexanol
1-(3-chlorobenzyle)-2-[4-2-(méthoxyphényle-pipérazine-1-yleméthyle]-cyclohexanol
1-(2,4-dichloro-benzyle)-2-[4-2-(méthoxyphényle-pipérazine-1-yleméthyle]- cyclohexanol
1-(benzyle)-2-[4-2-(méthoxyphényle-pipérazine-1-yleméthyle]-cyclododécanol
2-[4-2-(méthoxyphényle-pipérazine-1-yleméthyle]-1-vinyle-cyclododécanol
1-phénéthyle-2-[4-2-(méthoxyphényle-pipérazine-1-yleméthyle]-cyclodohexanol
1-benzyle-2-[4-2-(méthoxyphényle-pipérazine-1-yleméthyle]-cyclohexanol
1-benzyle-2-[phényle-(4-phényle-pipérazine-1-yleméthyle]- cyclohexanol
2-[(4-(méthyle-pipérazine-1-yle)-phényle-méthyle]-1-phénétyle- cyclohexanol
2-[4-2-(méthoxyphényle-pipérazine-1-yleméthyle]-1-phénéthyle-cyclododécanol
sous la forme du diastéréoisomère, de l'énantiomère et d'un mélange d'entre eux- y compris le racémique-ainsi que sous la forme de la base correspondante, du sel correspondant et du sel solvaté correspondant.

15. Procédé pour la préparation de composés N,N'-disubstitués de pipérazine selon l'une des revendications 1 - 14, **caractérisé en ce que** l'on
A₁) fait réagir en chauffant une cétone de formule (1), dans laquelle R¹ et R² ont la signification indiquée dans la revendication 1[AP10], 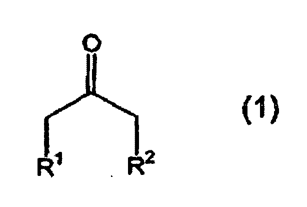 avec du paraformaldéhyde et une pipérazine de formule (2[AP11]), dans laquelle R⁵ a la signification indiquée dans la revendication 1 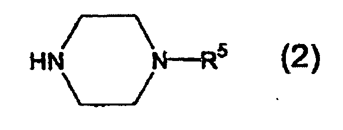 selon une réaction de Mannich dans un solvant approprié, de préférence dans l'éthanol avec addition d'acide chlorhydrique ou dans l'acide acétique, puis **en ce que** l'on traite le mélange réactionnel et l'on isole et purifie éventuellement le produit de formule (3[AP12]) 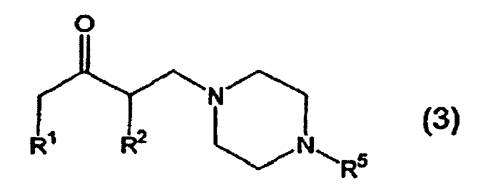
A₂) fait réagir une énamine de formule (1a), dans laquelle R¹ et R² ont la signification indiquée dans la revendication 1 et R représente un radical aliphatique en C₁₋₆, un radical morpholino, pipéridyle ou pyrrolidinyle, les deux radicaux R pouvant être identiques ou différents[AP13][AP14], 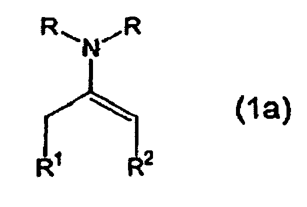 avec un aldéhyde de formule (4), dans laquelle R⁴ a la signification indiquée dans la revendication 1 à l'exception de l'hydrogène[AP15], 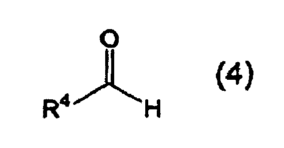 et une pipérazine de formule (2), dans laquelle R⁵ a la signification indiquée dans la revendication 1, éventuellement sous la forme de son hydrochlorure 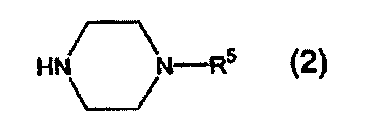 selon une réaction de Mannich en présence de triéthylamine, de chlorotriméthylsilane et d'iodure de sodium dans un solvant approprié, de préférence dans l'acétonitrile, puis **en ce que** l'on traite le mélange réactionnel et l'on isole et purifie éventuellement le produit de formule (3[AP16]a) 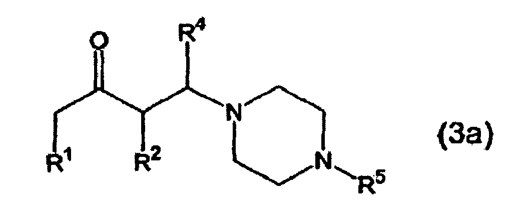
A₃) fait réagir une énamine de formule (1a), dans laquelle R¹ et R² ont la signification indiquée dans la revendication 1 et R représente un radical aliphatique en C₁₋₆, un radical morpholino, pipéridyle ou pyrrolidinyle, les deux radicaux R pouvant être identiques ou différents[AP17], 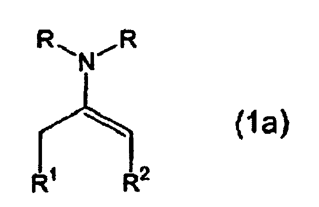 avec un sel d'iminium de formule (5), dans laquelle R⁴ a la signification indiquée dans la revendication 1 à l'exception de l'hydrogène, R⁵ a la signification indiquée dans la revendication 1 et Y⁻ représente un ion chlorure, bromure, iodure ou AlCl₄[AP18]⁻ 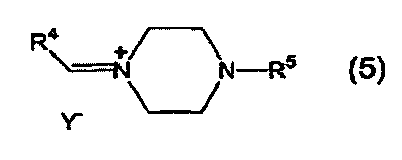 selon une réaction de Mannich dans un solvant approprié, de préférence dans l'acétonitrile, puis en ce que l'on traite le mélange réactionnel et on isole et purifie éventuellement le produit de formule (3[AP19]a) 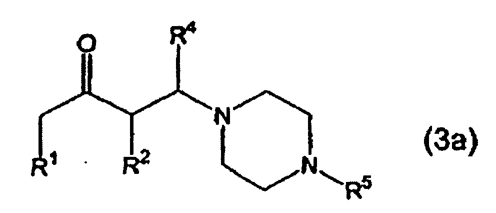 et
B) un composé de formule (3) ou (3a) avec un composé de Grignard ou un composé organo-lithien répondant aux formules R³MgCl, R³MgBr, R³MgI, MgR³₂ ou LiR³, dans lesquelles R³ a la signification indiquée dans la revendication 1, dans un solvant approprié, de préférence le diéthyléther ou le tétrahydrofuranne, puis **en ce que** l'on traite le mélange réactionnel et l'on isole et purifie éventuellement le composé de formule générale (I).

16. Médicament contenant au moins un composé selon l'une des revendications 1 - 14 en tant que principe actif, ainsi qu'éventuellement des adjuvants physiologiquement compatibles.

17. Médicament selon la revendication 16 destiné à combattre la douleur.

18. Médicament selon la revendication 16 destiné à l'anesthésie locale.

19. Médicament selon la revendication 16 en tant qu'antiarythmique.

20. Médicament selon la revendication 16 en tant qu'antiémétique.

21. Médicament selon la revendication 16 en tant que nootropique (neurotropique).

22. Médicament selon la revendication 16 destiné au traitement de maladies cardiovasculaires.

23. Médicament selon la revendication 16 destiné au traitement de l'incontinence rénale.

24. Médicament selon la revendication 16 destiné au traitement de la diarrhée.

25. Médicament selon la revendication 16 destiné au traitement du prurit.

26. Médicament selon la revendication 16 destiné au traitement de la dépendance à l'alcool.

27. Médicament selon la revendication 16 destiné au traitement de la dépendance à la drogue.

28. Médicament selon la revendication 16 destiné au traitement de la dépendance aux médicaments.

29. Médicament selon la revendication 16 destiné au traitement d'inflammations.

30. Médicament selon la revendication 16 destiné au traitement de dépressions.

31. Médicament selon la revendication 16 destiné à accroître la vigilance.

32. Médicament selon la revendication 16 destiné à l'augmentation de la libido.

33. Utilisation d'au moins un composé selon l'une des revendications 1 - 14, destinée à la préparation d'un remède destiné à combattre la douleur.

34. Utilisation d'au moins un composé selon l'une des revendications 1 - 14 destinée à la préparation d'un remède destiné au traitement de dépressions.

35. Utilisation d'au moins un composé selon l'une des revendication 1 - 14 destinée à la préparation d'un remède destiné à l'anesthésie locale et/ou au traitement de l'arythmie, de l'émésis, d'affections cardiovasculaires, de l'incontinence rénale, de la diarrhée, du prurit, d'inflammations, de la dépendance à l'alcool et/ou à la drogue et/ou aux médicaments, destiné à l'augmentation de l'entrain, de la vigilance et/ou de la libido.
